# FASCICULE DE BREVET EUROPEEN

(11) **EP 2 771 334 B1**
(45) Date de publication et mention de la délivrance du brevet: **19.08.2015**
(21) Numéro de dépôt: 12791826.6
(22) Date de dépôt: 26.10.2012
(51) Int. Cl.: C07D 405/06, C07D 405/14, C07D 407/06, C07D 407/14, C07D 409/14, C07D 413/14, A61K 31/341, A61P 11/00, A61P 11/06, A61K 31/4025, A61P 17/00, A61P 17/06, A61P 17/10, A61P 19/02, A61P 29/00, A61P 35/00

(54) **COMPOSÉS DI-SUBSTITUÉS DE LA DIAMINO-3,4-CYCLOBUTÈNE-3-DIONE-1,2 UTILES DANS LE TRAITEMENT DE PATHOLOGIES MÉDIÉES PAR DES CHIMIOKINES**
DISUBSTITUIERTE 3,4-DIAMINO-3-CYCLOBUTEN-1,2-DION-VERBINDUNGEN ZUR VERWENDUNG BEI DER BEHANDLUNG CHEMOKINBEDINGTER ERKRANKUNGEN
DISUBSTITUTED 3,4-DIAMINO-3-CYCLOBUTENE-1,2-DIONE COMPOUNDS FOR USE IN THE TREATMENT OF CHEMOKINE-MEDIATED PATHOLOGIES

(30) Priorité: 28.10.2011 US 201161552829 P; 28.10.2011 FR 1159829
(43) Date de publication de la demande: 03.09.2014
(73) Titulaire: Galderma Research & Development, 06410 Biot (FR)
(72) Inventeur: MUSICKI, Branislav, F-06000 Nice (FR); AUBERT, Jérôme, 06130 Grasse (FR); BOITEAU, Jean-Guy, 06560 Valbonne (FR); CLARY, Laurence, 06480 La Colle-Sur-Loup (FR); ROSSIO, Patricia, 06130 Grasse (FR); SCHUPPLI-NOLLET, Marlène, 06620 Le Bar Sur Loup (FR)
(74) Mandataire: Casalonga
(86) Numéro de dépôt international: PCT/FR2012/052479
(87) Numéro de publication internationale: WO 2013/061005

(56) Documents cités:
- WO-A1-02/083624
- WO-A1-2008/005570
- WO-A1-2010/063802
- AKI C ET AL: "Diaminocyclobutenediones as potent and orally bioavailable CXCR2 receptor antagonists: SAR in the phenolic amide region", BIOORGANIC & MEDICINAL CHEMISTRY LETTERS, PERGAMON, ELSEVIER SCIENCE, GB, vol. 19, no. 15, 1 août 2009 (2009-08-01), pages 4446-4449, XP026301721, ISSN: 0960-894X, DOI: 10.1016/J.BMCL.2009.05.049 [extrait le 2009-05-18]
- LAI G ET AL: "Synthesis and structure-activity relationships of new disubstituted phenyl-containing 3,4-diamino-3-cyclobutene-1,2-diones as CXCR2 receptor antagonists", BIOORGANIC & MEDICINAL CHEMISTRY LETTERS, PERGAMON, ELSEVIER SCIENCE, GB, vol. 18, no. 6, 15 mars 2008 (2008-03-15), pages 1864-1868, XP025694983, ISSN: 0960-894X, DOI: 10.1016/J.BMCL.2008.02.010 [extrait le 2008-02-10]
- CHAO ET AL: "C(4)-alkyl substituted furanyl cyclobutenediones as potent, orally bioavailable CXCR2 and CXCR1 receptor antagonists", BIOORGANIC & MEDICINAL CHEMISTRY LETTERS, PERGAMON, ELSEVIER SCIENCE, GB, vol. 17, no. 13, 1 juillet 2007 (2007-07-01), pages 3778-3783, XP022114581, ISSN: 0960-894X, DOI: 10.1016/J.BMCL.2007.04.016
- YU ET AL: "Synthesis and structure-activity relationships of heteroaryl substituted-3,4-diamino-3-cyclobut-3-ene-1 ,2-dione CXCR2/CXCR1 receptor antagonists", BIOORGANIC & MEDICINAL CHEMISTRY LETTERS, PERGAMON, ELSEVIER SCIENCE, GB, vol. 18, no. 4, 11 janvier 2008 (2008-01-11), pages 1318-1322, XP022479324, ISSN: 0960-894X, DOI: 10.1016/J.BMCL.2008.01.024

## Description

### Domaine de l'invention :

La présente invention porte sur de nouveaux composés di-substitués de la diamino-3,4-cyclobutène-3-dione-1,2, sur les compositions pharmaceutiques contenant ces composés ainsi que sur l'utilisation de ces composés et de ces compositions pour le traitement de pathologies médiées par des chimokines.

### Etat de la technique antérieur à l'invention :

Les chimiokines ou cytokines sont de petites protéines solubles. Leur rôle le plus connu est l'attraction et le contrôle de l'état d'activation des cellules du système immunitaire. Toutes les chimiokines exercent leurs fonctions en se fixant sur des récepteurs couplés aux protéines G. Certaines chimiokines sont considérées comme pro-inflammatoires. La sécrétion de ces chimiokines peut être induite lors de la réponse immune afin de favoriser l'arrivée de cellules du système immunitaire au niveau d'un site infectieux.

Il existe deux types de chimiokines : les chimiokines pro-inflammatoires et les chimiokines constitutives.

Les chimiokines pro-inflammatoires (ou « inductibles ») sont produites au niveau de sites d'inflammation par des cellules de tissus ou des leucocytes infiltrés, après contact avec un agent pathogène.

Les chimiokines constitutives (ou « homéostatiques ») sont produites dans les organes lymphoïdes et dans certains organes non-lymphoïdes tels que la peau et les muqueuses. Elles régulent le trafic lymphocytaire et la localisation des lymphocytes au sein de ces organes pendant la lymphopoïèse mais également pour maintenir l'immunosurveillance.

La nomenclature de ces récepteurs à chimiokines est basée sur le groupe de chimiokines auquel son ligand appartient. Ainsi, les récepteurs correspondant aux chimiokines du groupe CXC sont, par exemple, appelés CXCR1, CXCR2, CXCR3, CXCR4, etc, et les récepteurs correspondant aux chimiokines du groupe CC sont, par exemple, appelés CCR1, CCR2, CCR3, etc. Ces récepteurs présentent tous une structure tertiaire semblable, et ils sont couplés à une protéine G : ils font donc partie de la superfamille des GPCR (G Protein Coupled Receptor).

L'interleukine-8 ou IL-8 (également nommé CXCL-8) est un membre de la famille des chimiokines CXC, qui joue un rôle primordial dans le recrutement des neutrophiles vers le site d'inflammation. Deux récepteurs, CXCR1 & CXCR2 sont connus pour être spécifiquement activés par IL-8. Alors que CXCR2 se lie avec une forte affinité à IL-8 et aux chimiokines apparentées comme CXCL6, CXCL5, CXCL3, CXCL2 et CXCL1, CXCR1 se lie uniquement à IL-8. Des niveaux élevés d'IL-8 et de chimiokines apparentées (CXCL5, CXCL2 & CXCL1) ont été décrits dans les lésions d'acné inflammatoire (J Invest Dermatol. 2006;126:1071-9; Am J Pathol. 2005;166(6):1691-9; Diagn Pathol. 2007 Jan 30;2:4).

Des premiers indices démontrent l'expression de CXCR2 dans l'acné inflammatoire (Trivedi et al. J Invest Dermatol. 2006 126(5) :1071-9). Ainsi, des antagonistes doubles de CXCR1 et CXCR2 pourraient permettre de diminuer rapidement les effets délétères de la réponse inflammatoire par IL-8.

La demande de brevet WO 02/083624 (SCHERING/PHARMACOPEIA) divulgue plus particulièrement des composés substitués de la cyclobutenedione-1,2 capables de moduler l'activité des récepteurs aux chimiokines de type CXC, et plus particulièrement l'activité des récepteurs CXCR1 et CXCR2. Parmi ces composés, le composé SCH-527123 (correspondant à l'exemple 360.71 en page 281), appelé également Navarixin, est en cours de développement (Phase II) pour le traitement de la maladie pulmonaire obstructive chronique (ou COPD pour Chronic Obstructive Pulmonary Disease). Ce composé a également fait l'objet d'études de phase II dans l'asthme et dans le psoriasis, mais ces développements ont été arrêtés.

Il est aujourd'hui connu que de nombreuses pathologies d'ordre inflammatoire sont médiées par des chimiokines. Il existe cependant un besoin non satisfait à ce jour de traiter la composante inflammatoire des pathologies d'intérêt dans le domaine de la dermatologie comme par exemple l'acné, la rosacée ou encore les dermatoses neutrophiliques, notamment le psoriasis.

De la même manière, la promesse d'obtenir de nouvelles thérapies efficaces pour traiter des maladies médiées par des chimiokines à l'aide d'antagonistes des récepteurs aux chimiokines n'a pas été tenue. En effet, plusieurs études cliniques ont échoué en phase II. Une des raisons peuvant expliquer ces échecs est l' « overlap » des effets biologiques des différentes chimiokines induites en situation pathologique. A ce jour, le traditionnel « Drug discovery process » a pour objectif d'identifier des molécules ciblant un récepteur spécifique sans effet « off target ». Cette approche n'est sans doute pas la plus adaptée pour traiter des maladies inflammatoires complexes. De plus en plus d'approches semblent privilégier la recherche de molécules antagonistes à large spectre d'action (« promiscuous compounds »), lesdites approches pouvant ainsi s'avérer plus efficaces pour traiter des maladies complexes et multifactorielles (Frantz S. Drug discovery: playing dirty. Nature. 2005 Oct 13;437(7061):942-3 ; Roth BL, Sheffler DJ, Kroeze WK. Magic shotguns versus magic bullets: selectively non-selective drugs for mood disorders and schizophrenia. Nat Rev Drug Discov. 2004 Apr;3(4):353-9.)

Or, la demanderesse a découvert de nouveaux composés présentant une activité antagoniste non seulement vis-à-vis des récepteurs de type CXCR1 et CXCR2, mais également une forte activité antagoniste vis-à-vis des récepteurs aux chimiokines, notamment les récepteurs CCR6 et CXCR3. Ces nouveaux composés présentent de manière surprenante une polypharmacologie, ce qui leur confère un intérêt supplémentaire par rapport aux composés déjà connus dans le traitement de pathologies médiées par des chimiokines, et plus particulièrement des pathologies d'ordre dermatologique.

De plus, ces nouveaux composés présentent une stabilité hépatique bien inférieure à celle des composés déjà décrits capables de bloquer l'activation des récepteurs CXCR1 et CXCR2 comme par exemple le composé SCH-527123. Cette propriété particulière présente l'avantage de disposer de nouveaux composés ayant, de manière surprenante, un profil plus adapté pour le traitement par voie topique de pathologies d'ordre dermatologique. En effet, leur instabilité hépatique entraine une exposition systémique faible, voire nulle, et donc des effets secondaires limités.

Une autre particularité des composés décrits dans la présente invention est leur constante de dissociation vis-à-vis des récepteurs de type CXCR1 et CXCR2, constante qui est bien inférieure à celle des composés décrits dans la demande de brevet WO 02/083624, comme par exemple le SCH-527123. En effet, la molécule SCH-527123 a été décrite pour avoir un temps de dissociation de l'ordre de 22h (Dissociation pseudo-irréversible) (Pharmacological Characterization of SCH-527123, a Potent Allosteric CXCR1/CXCR2 Antagonist. JPET 322:477-485, 2007), alors que les temps de dissociation des composés de la présente invention sont bien inférieurs.

Des exemples de la littérature montrent qu'une dissociation rapide des antagonistes favorise une diminution de leur toxicité. Ceci a été décrit pour les antagonistes des récepteurs dopamine D2 (Am J Psychiatry (2001) 158(3):360-369), des récepteurs *N*-methyl-D-aspartate (NMDA) (Nat Rev Drug Disc (2006) 5(2):160-170.) ainsi que pour les anti-inflammatoires non stéroidiens (Lett Drug Des Discov (2006) 3(8):569-574*. et* Pharm Med (2008) 22(1):23-34). En effet, un temps de dissociation long aurait tendance à induire des effets indésirables. Avec des temps de dissociation rapide, les composés selon l'invention présentent par conséquent des effets secondaires réduits.

### Résumé de l'invention:

Un premier objet selon l'invention concerne de nouveaux composés di-substitués de la diamino-3,4- cyclobutène-3-dione-1,2 répondant à la formule générale (I) suivante: 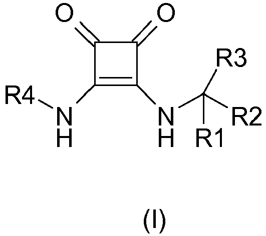 ainsi que leurs sels, solvates ou hydrates pharmaceutiquement acceptable pour lesquels les substituants R1, R2, R3 et R4 sont tels que définis ci-après dans la description détaillée de l'invention.

Un second objet selon l'invention concerne une composition pharmaceutique comprenant une quantité efficace d'un composé répondant à la formule générale (I), d'un de ses sels ou un de ses solvates ou hydrates pharmaceutiquement acceptable en association avec un solvant ou un support pharmaceutiquement acceptable.

Un troisième objet selon l'invention concerne un composé ou une composition pharmaceutique tel que décrit ci-dessus pour son utilisation en tant que médicament.

Un quatrième objet selon l'invention concerne un composé ou une composition pharmaceutique tel que décrit ci-dessus pour son utilisation dans le traitement de maladies médiées par les chimiokines.

Un cinquième objet selon l'invention concerne un composé ou composition pharmaceutique tel que décrit ci-dessus pour son utilisation dans le traitement de maladies du groupe comprenant les dermatoses neutrophiliques, et notamment le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn, le rejet de greffe, la mucoviscidose et les cancers cutanés.

### Description détaillée de l'invention :

A moins qu'il en soit indiqué autrement, les définitions suivantes s'appliquent à l'ensemble de la description et des revendications.

Ces définitions s'appliquent indépendamment de savoir si un terme est utilisé seul ou en combinaison avec d'autres termes. Ainsi, par exemple, la définition du terme « aryle » s'applique aussi bien à « aryle » en tant que tel qu'à la partie « aryle » du terme « aryloxy ».

"Alkyle" désigne une chaîne hydrocarbonée saturée linéaire ou ramifiée dont le nombre d'atomes de carbone est précisé.

Lorsque le nombre d'atomes de carbone n'est pas précisé, cela signifie que la chaine alkyle contient de 1 à 20 atomes de carbone.

Les radicaux alkyles préférés contiennent de 1 à 12 atomes de carbone, et ceux encore plus préférés contiennent de 1 à 6 atomes de carbone dans la chaîne.

«Alkoxy" désigne un oxygène substitué par un radical alkyle tel que défini précédemment.

Des exemples de radicaux alkoxy comprennent les radicaux méthoxy, éthoxy, n-propoxy, isopropoxy et n-butoxy.

«Aryle» désigne un système cyclique aromatique monocyclique ou polycyclique (2 à 3 cycles) comprenant de 6 à 14 atomes de carbone, et de préférence de 6 à 10 atomes de carbone.

A titre d'exemples de radical aryle, on peut citer les radicaux phényle, naphtyle, indényle, tétrahydronaphtyle, indanyle, anthracényle et fluorényle.

« Hétéroaryle » désigne un système aromatique monocyclique ou polycyclique (2 à 3 cycles) comprenant de 5 à 14 atomes cycliques, de préférence de 5 à 10 atomes cycliques, dans lequel un ou plusieurs des atomes cycliques représente(nt) un ou plusieurs (de 1 à 5) hétéroatome(s) choisi(s) dans le groupe comprenant l'azote, l'oxygène et le soufre.

Les hétéroaryle préférés contiennent 5 ou 6 atomes cycliques et 1 à 3 hétéroatomes.

Le préfixe aza, oxa ou thia avant le nom de la racine hétéroaryle signifie qu'au moins un azote, un oxygène ou un soufre est respectivement présent dans le cycle.

Un atome d'azote d'un hétéroaryle peut être éventuellement oxydé en N-oxyde. A titre d'exemples d'heteroaryles appropriés, on peut citer les hétéroaryles suivants :
pyridyle, pyrazinyle, furanyle, thiényle, pyrimidinyle, isoxazolyle, isothiazolyle, oxazolyle, thiazolyle, pyrazolyle, furazanyle, pyrrolyle, pyrazolyle, triazolyle, le 1,2,4-thiadiazolyle, pyrazinyle, pyridazinyle, quinoxalinyle, phtalazinyle, imidazo [1,2-a] pyridinyle, imidazo [2,1-b] thiazolyle, benzofurazanyl, indolyle, azaindolyl, benzimidazolyle, benzothiényle, quinolinyle, imidazolyle, thienopyridyle, quinazolinyle, thienopyrimidyle, pyrrobpyridyle, imidazopyridyle, isoquinolinyle, benzoazaindolyle, 1,2,4 triazinyle et benzothiazolyle.

«Arylalkyle» désigne un radical dont les parties aryle et alkyle sont telles que définies ci-dessus.

A titre d'exemples d'arylalkyle, on peut citer les radicaux benzyle, phénéthyle et naphthalénylméthyle.

La liaison à la structure à laquelle il est rattaché se fait par le radical alkyle.

«Hétéroarylalkyle» désigne un radical dont les parties heteroaryle et alkyle sont telles que définies ci-dessus.

A titre d'exemples d'hétéroarylalkyle, on peut citer les radicaux pyridylméthyle, pyridyléthyle, imidazolylméthyle, imidazolyléthyle, pyrazolylméthyle et pyrazolyléthyle.

La liaison à la structure à laquelle il est rattaché se fait par le radical alkyle.

«Cycloalkyle» désigne un système cyclique hydrocarboné non aromatique, ayant de 3 à 10 atomes de carbone, de préférence de 5 à 10 atomes de carbone, et de un à trois cycles.

Les radicaux cycloalkyle préférés contiennent de 5 à 7 atomes cycliques.

A titre d'exemples de radicaux cycloalkyle, on peut citer les radicaux cyclopropyle, cyclopentyle, cyclohexyle, cycloheptyle, norbornyle et adamantyle.

«Cycloalkylalkyle" désigne un radical dont les parties cycloalkyle et alkyles sont telles que définies ci-dessus.

A titre d'exemples de cycloalkylalkyle, on peut citer les radicaux cyclopropylméthyle, cyclopropyléthyle, cyclobutylméthyle, cyclobutylèthyle, cyclopentylméthyle, cyclopentyléthyle, cyclohexylméthyle, cyclohexyléthyle, norbornylméthyle et adamantylméthyle.

La liaison à la structure à laquelle il est rattaché se fait par le radical alkyle.

«Hétérocycloalkyle » désigne un système cyclique hydrocarboné non aromatique, ayant de 4 à 10 atomes de carbone, de préférence de 5 à 10 atomes de carbone, et de un à trois cycles, et comprenant de un à trois hétéroatomes choisi dans le groupe constitué par l'azote, l'oxygène et le soufre.

Les radicaux hétérocycloalkyles préférés contiennent de 5 à 7 atomes cycliques.

A titre d'exemples de radicaux hétérocycloalkyle, on peut citer les groupes tétrahydrofuranyle, tétrahydrothiophényle, tétrahydropyrannyle et 7-oxa-bicyclo-[2.2.1]-heptanyle.

« Alkyle fluoré » désigne un radical alkyle tel que défini précédemment substitué par un ou plusieurs atomes de fluor.

A titre d'exemples de radicaux alkyle fluoré, on peut citer les radicaux fluorométhyle, difluorométhyle, 2-fluoroéthyle, 2,2-difluoroéthyle et 2,2,2-trifluoroéthyle.

« Alkyle perfluoré » désigne un radical alkyle tel que défini précédemment dans lequel chaque atome d'hydrogène a été substitué par un atome de fluor.

A titre d'exemples de radicaux perfluoré, on peut citer les radicaux trifluorométhyle, et pentafluoroéthyle.

Ainsi, un premier objet selon l'invention concerne de nouveaux composés di-substitués de la diamino-3,4- cyclobutène-3-dione-1,2 répondant à la formule générale (I) suivante ou un de ses sels ou solvates pharmaceutiquement acceptable: 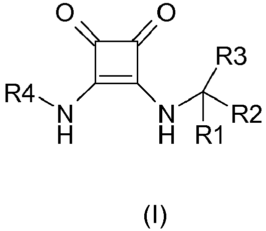 dans laquelle,
R1 représente un atome d'hydrogène ou un méthyle,
R2 représente un cycle à quatre atomes choisi parmi les structures (1) et (2) suivantes: 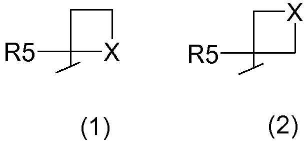 dans lesquelles R5 et X ont la signification donnée ci-après,

R3 représente un cycle aromatique ou heteroaromatique sélectionné dans le groupe constitué par les cycles répondant aux formules (a) à (o) suivantes : 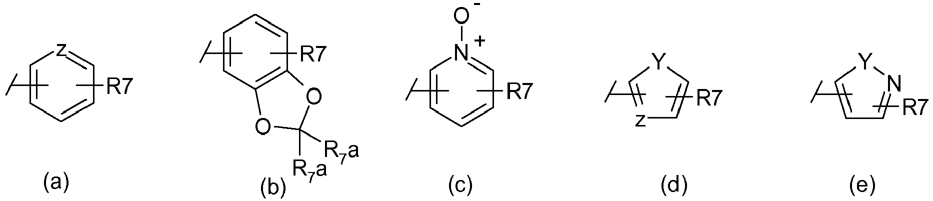 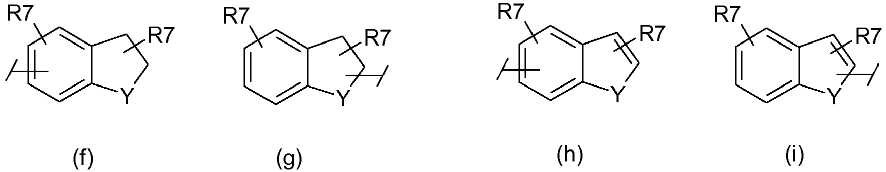 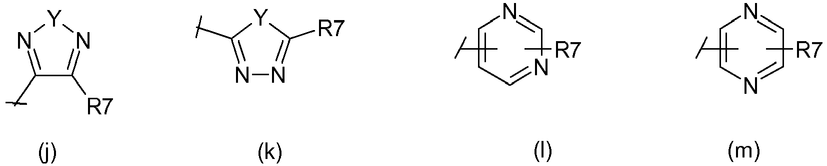 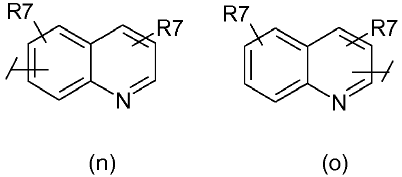 dans lesquelles R7, R7a, Y et Z ont la signification donnée ci-après, étant précisé que les cycles (a) à (o) peuvent éventuellement porter plusieurs groupes R7, identiques ou différents, le nombre total de tels groupes R7 étant au maximum égal au nombre d'atomes substituables du cycle ;

R4 représente un cycle aromatique ou hétéroaromatique sélectionné dans le groupe constitué par les cycles répondant aux formules (p) à (z) et (aa) à (ak) suivantes : 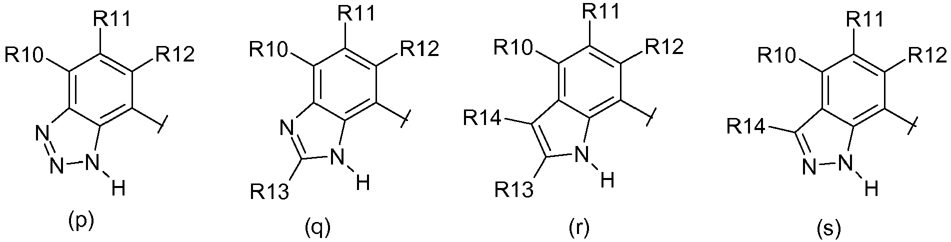 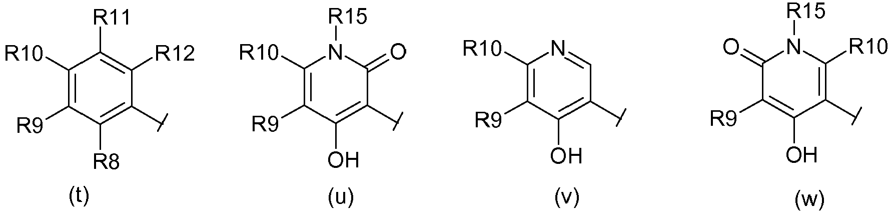 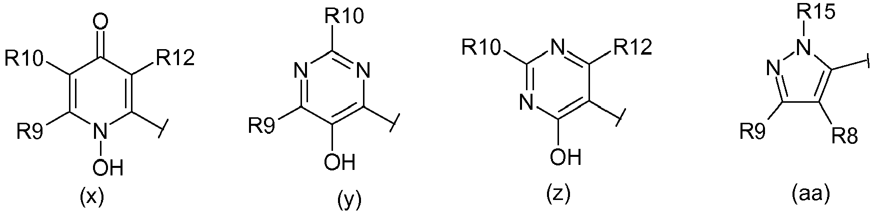 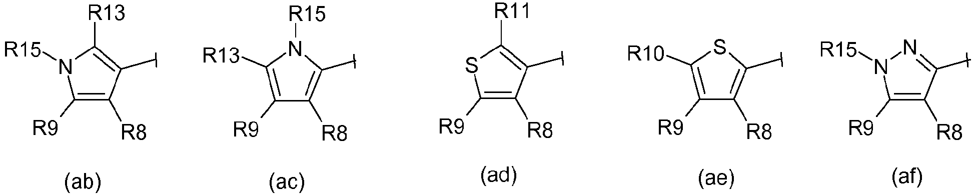 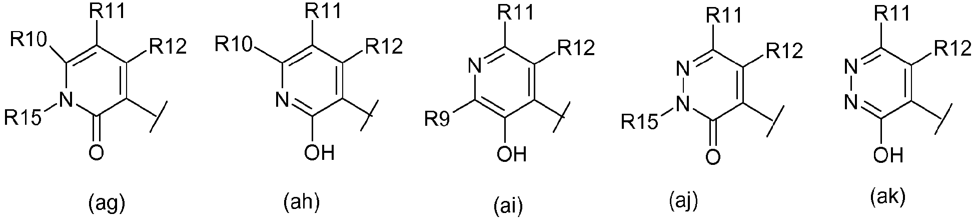 dans lesquelles R7, R8, R9, R10, R11, R12, R13, R14 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un radical R16, un halogène, -CF3, -COR16, -OR16, -NR16R17, -NO2, -CN,-SO2R16, -SO2NR16R17, -NR16COR17, -CONR16R17, -NR16CO2R17 ou -CO2R16,
R7a représente un atome d'hydrogène ou bien un radical alkyle ayant de 1 à 5 atomes de carbones,
R8 représente un atome d'hydrogène, un atome d'halogène, un radical -OH, -SH, - CONHOR16, -CONR16OH, -NR16R17, -SO3H, -OCOR16, -NHSO2R16, -SO2NR16R17,-NHCOR16, -CONR16R17, -NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un hydrogène, un atome d'halogène, un radical alkyle, alkoxy, -CF3, - OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p) à (z) et (aa) à (ak) ci-dessus, alors ils peuvent former ensemble, avec la liaison qui les réunit, un cycle aryle, hétéroaryle, cycloalkyle ou hétérocycloalkyle,
R13 et R14 sont identiques ou différents et sont indépendamment choisis dans le groupe constitué par un atome d'hydrogène, un atome d'halogène, un alkyle, -CF3, -OCF3, -OH, -SH, -CN, -SO2R16, -SO2NR16R17, -NHSO2NR16R17, -NR16R17, -NR16CONR16R17,-NR16COR17, -NR16CO2R17, -CONR16R17, -COR16 ou -CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, hétéroaryle, arylalkyle, hétéroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

Dans un mode de réalisation préféré selon l'invention, les composés ainsi que leurs sels, solvates ou hydrates pharmaceutiquement acceptables, répondent à la formule (I) précitée dans laquelle:
R1 représente un atome d'hydrogène,
R2 représente un cycle à quatre chainons répondant à la structure (2) suivante: 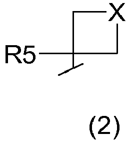 dans laquelle R5 et X ont la signification donnée ci-après,
   R3 représente un cycle aromatique ou hétéroaromatique sélectionné dans le groupe constitué par les cycles répondant aux formules (a), (b) et (d) suivantes : 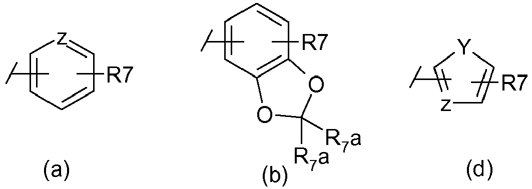 dans lesquelles R7, R7a, Y et Z ont la signification donnée ci-après, étant précisé que les cycles (a), (b) et (d) peuvent éventuellement porter plusieurs groupes R7, identiques ou différents, le nombre total de tels groupe R7 étant au maximum égal au nombre d'atomes substituables du cycle ;
   R4 représente un cycle aromatique ou hétéroaromatique sélectionné dans le groupe constitué par les cycles répondant aux formules (p), (q), (t), (z), (ad), (ag) et (ah) suivantes : 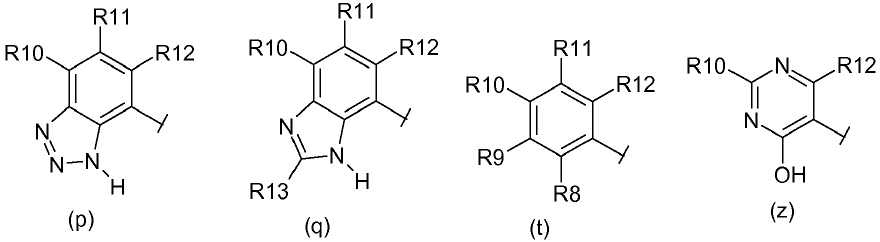 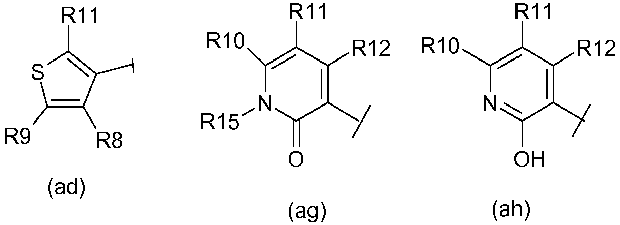 dans lesquelles R8, R9, R10, R11, R12, R13 et R15 ont la signification donnée ci-après,
   R5 représente un atome d'hydrogène, un atome de fluor, un radical alkyle ayant de 1 à 5 atomes de carbones ou un radical alkyle fluoré ou perfluoré comportant de 1 à 5 atomes de carbones,
   R6 représente un atome d'hydrogène, un radical -COOtBu ou un radical -COOBn,
   R7 représente un halogène, un radical R16, -CF3, -COR16, -OR16, -NR16R17, -NO2, -CN,-SO2R16, -SO2NR16R17, -NR16COR17, -CONR16R17, -NR16CO2R17 ou -CO2R16,
   R7a représente un atome d'hydrogène ou bien un radical alkyle ayant de 1 à 5 atomes de carbones,
   R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17, - NR16CO2R17, -NHSO2NR16R17, -CO2R16, pyrrolyle, imidazolyle, triazolyle ou tétrazolyle,
   R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17,-CONR16R17, -COR16 ou -CO2R16,
   ou bien encore, lorsque deux des radicaux R9, R10, R11 et R12 se trouvent en position ortho sur un cycle aromatique ou hétéroaromatique sélectionné dans le groupe constitué par les cycles répondant aux formules (p), (q), (t), (z), (ad), (ag) et (ah) ci-dessus, alors ils peuvent former ensemble, avec la liaison qui les réunit, un cycle aryle, hétéroaryle, cycloalkyle ou hétérocycloalkyle,
   R13 est choisi dans le groupe constitué par un atome d'hydrogène, un atome d'halogène, un radical alkyle, -CF3, -OCF3, -OH, -SH, -CN, -SO2R16, -SO2NR16R17, -NHSO2NR16R17,-NR16R17, -NR16CONR16R17, -NR16COR17, -NR16CO2R17, -CONR16R17, -COR16 ou-CO2R16,
   R15 représente un atome d'hydrogène, un radical -OH, -SO2R16, -COR16, -CO2R16, aryle, hétéroaryle, arylalkyle, hétéroarylalkyle, alkyle, cycloalkyle ou ycloalkylalkyle,
   R16 et R17 sont identiques ou différents et sont indépendamment choisis dans le groupe constitué par un atome d'hydrogène, un radical aryle, hétéroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
   ou bien encore lorsque R16 et R17 sont portés par un même atome d'azote, ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
   X représente un atome d'oxygène, un atome de soufre, ou un atome d'azote substitué par un radical R6,
   Y représente un atome d'oxygène, un atome de soufre, ou un atome d'azote substitué par un radical R15, et
   Z représente un atome de carbone ou d'azote.

Dans un mode de réalisation plus particulièrement préféré selon l'invention, les composés ainsi que leurs sels, solvates ou hydrates pharmaceutiquement acceptable, répondent à la formule (I) précitée dans laquelle:
R1 représente un atome d'hydrogène,
R2 représente un cycle à quatre atomes répondant à la structure (2) suivante: 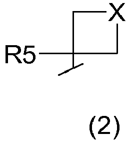 dans laquelle R5 et X ont la signification donnée ci-après,
R3 représente un cycle hétéroaromatique répondant à la formule (d) suivante : 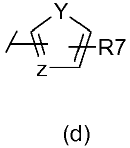 dans laquelle R7, Y et Z ont la signification donnée ci-après, étant précisé que le cycle (d) peut éventuellement porter plusieurs groupes R7, identiques ou différents, le nombre total de tels groupe R7 étant au maximum égal au nombre d'atomes substituables du cycle ;
R4 représente un cycle aromatique ou heteroaromatique sélectionné dans le groupe constitué par les cycles répondant aux formules (p), (q), (t), (z), (ad), (ag) et (ah) suivantes : 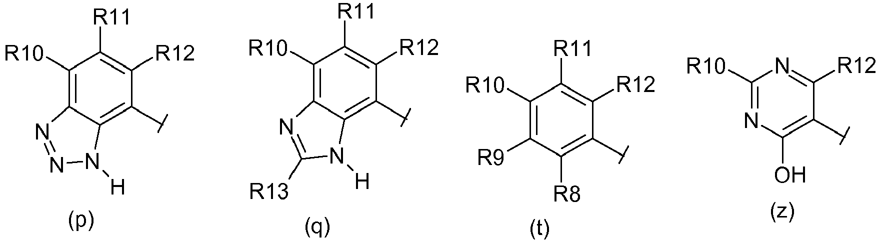 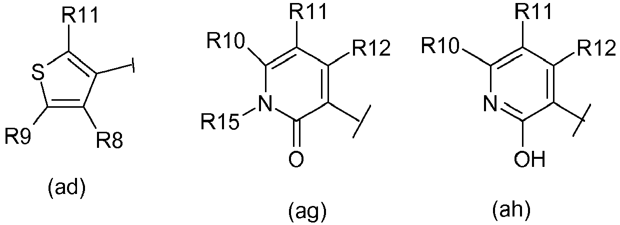 dans lesquelles R8, R9, R10, R11, R12, R13 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un atome d'halogène, un radical R16, -CF3, -COR16, -OR16, -NR16R17, - NO2, -CN, -SO2R16, -SO2NR16R17, -NR16COR17, -CONR16R17, -NR16CO2R17 ou - CO2R16,
R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17, - NR16CO2R17, -NHSO2NR16R17, -CO2R16, pyrrolyle, imidazolyle, triazolyle ou tétrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p), (q), (t), (z), (ad), (ag) et (ah) ci-dessus, ils peuvent former ensemble, avec la liaison qui les réunit, un cycle aryle, hétéroaryle, cycloalkyle ou hétérocycloalkyle,
R13 est choisi dans le groupe constitué par un atome d'hydrogène, un atome d'halogène, un radical alkyle, -CF3, -OCF3, -OH, -SH, -CN, -SO2R16, -SO2NR16R17, -NHSO2NR16R17,-NR16R17, -NR16CONR16R17, -NR16COR17, -NR16CO2R17, -CONR16R17, -COR16 ou-CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

Dans un mode de réalisation encore plus particulièrement préféré selon l'invention, les composés ainsi que leurs sels, solvates ou hydrates pharmaceutiquement acceptable, répondent à la formule (I) précitée dans laquelle:
R1 représente un atome d'hydrogène,
R2 représente un cycle à quatre atomes répondant à la structure (2) suivante: 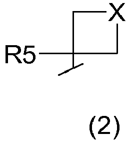 dans laquelle R5 et X ont la signification donnée ci-après,
R3 représente un cycle hétéroaromatique répondant à la formule (d) suivante : 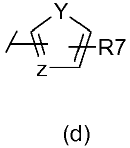 dans laquelle R7, Y et Z ont la signification donnée ci-après, étant précisé que le cycle (d) peut éventuellement porter plusieurs groupes R7, identiques ou différents, le nombre total de tels groupe R7 étant au maximum égal au nombre d'atomes substituables du cycle ;
R4 représente un cycle aromatique répondant à la formule (t) suivante : 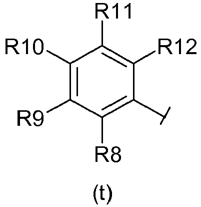 dans lesquelles R8, R9, R10, R11 et R12 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un radical R16, un halogène, -CF3, -COR16, -OR16, -NR16R17, -NO2, -CN,-SO2R16, -SO2NR16R17, -NR16COR17, -CONR16R17, -NR16CO2R17 ou -CO2R16,
R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17,-NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur le cycle aromatique (t), ils peuvent former ensemble, avec la liaison qui les réunit, un cycle aryle, hétéroaryle, cycloalkyle ou hétérocycloalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

Parmi les composés plus particulièrement préférés, on peut citer par exemple ceux choisis dans la liste comprenant:
1/ 2-hydroxy-N,N-diméthyl-3-(2-{[((R)-5-méthyl-furan-2-yl)-(3-méthyl-oxetan-3-yl)-méthyl]-amino}-3,4-dioxo-cyclobut-1-enylamino)-benzamide
2/ 3-(2-{[(3-Fluorométhyl-oxetan-3-yl)-(5-méthyl-furan-2-yl)-méthyl]-amino}-3,4-dioxo-cyclobut-1-enylamino)-2-hydroxy-N,N-diméthyl-benzamide
3/ 3-(2-{[(3-Ethyl-oxetan-3-yl)-(5-méthyl-furan-2-yl)-méthyl]-amino}-3,4-dioxo-cyclobut-1-enyl-amino)-2-hydroxy-N,N-diméthyl-benzamide
4/ acide 2-hydroxy-3-(2-{[(5-méthyl-furan-2-yl)-(3-méthyl-oxetan-3-yl)-méthyl]-amino}-3,4-dioxo-cyclobut-1 -enylamino)-benzoïque
5/ 3-[2-hydroxy-3-((R)-3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène-1,2-dione
6/ 3-[2-hydroxy-3-((S)-3-hydroxy-pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione
7/ (R)-1-[2-hydroxy-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylic acid tert-butyl ester
8/ (R)-1-[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
9/ (S)-1-[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
10/ acide (R)-1-[2-hydroxy-3-(2-{[(5-méthyl-furan-2-yl)-(3-méthyl-oxetan-3-yl)-méthyl]-amino}-3,4-dioxocyclobut-1-enylamino)-benzoyl]-pyrrolidine-2-carboxylique
11/ 3-[2-hydroxy-3-(1-hydroxy-ethyl)-phenylamino]-4-{[(5-méthyl-furan-2-yl)-(3-méthyl-oxetan-3-yl)-méthyl]-amino}-cyclobut-3-ène-1,2-dione
12/ 3-(2-hydroxy-3-isobutyryl-phenylamino)-4-{[(5-méthyl-furan-2-yl)-(3-méthyl-oxetan-3-yl)-méthyl]-amino}-cyclobut-3-ène-1,2-dione
13/ 3-(4-hydroxy-2-méthyl-3-oxo-2,3-dihydro-1H-isoindol-5-ylamino)-4-{[(5-méthyl-furan-2-yl)-(3-méthyl-oxetan-3-yl)-méthyl]-amino}-cyclobut-3-ène-1,2-dione
14/ 3-[2-hydroxy-3-(pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione
15/ 3-[2-hydroxy-3-(morpholine-4-carbonyl)-phenylamino]-4-{[(5-méthyl-furan-2-yl)-(3-méthyl-oxetan-3-yl)-méthyl]-amino}-cyclobut-3-ène-1,2-dione
21/ 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzènesulfonamide 22/ 2-Hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzènesulfonamide
24/ 3-{[(5-Méthyl-furan-2-yl)-(3-méthyl-oxetan-3-yl)-méthyl]-amino}-4-(2-oxo-2,3-dihydro-benzooxazol-7-ylamino)-cyclobut-3-ène-1,2-dione
26/ 3-[[2-(3-Diméthylcarbamoyl-2-hydroxy-phenylamino)-3,4-dioxo-cyclobut-1-enylamino]-(5-méthyl-furan-2-yl)-méthyl]-3-méthyl-azetidine-1-carboxylate de tert-butyle
27/ 3-(2-{[(4,5-diméthyl-furan-2-yl)-(3-méthyl-oxetan-3-yl)-méthyl]-amino}-3,4-dioxo-cyclobut-1-enylamino)-2-hydroxy-N,N-diméthyl-benzamide
31/ 3-[3-(3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène-1,2-dione
32/ 1-[3-(2-{[(5-méthyl-furan-2-yl)-(3-méthyl-oxetan-3-yl)-méthyl]-amino}-3,4-dioxo-cyclobut-1-enylamino)-benzoyl]-piperidine-2-carboxylate de méthyle
33/ 1-[3-(2-{[(5-Méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
34/ 2-hydroxy-N,N-diméthyl-3-(2-{[(3-méthyl-oxetan-3-yl)-thiophen-2-yl-méthyl]-amino}-3,4-dioxo-cyclobut-1-enylamino)-benzamide
35/ 2-hydroxy-N,N-diméthyl-3-(2-{[(3-méthyl-oxetan-3-yl)-(5-méthyl-thiophen-2-yl)-méthyl]-amino}-3,4-dioxo-cyclobut-1-enylamino)-benzamide
36/ 3-(2-{[furan-2-yl-(3-méthyl-oxetan-3-yl)-méthyl]-amino}-3,4-dioxo-cyclobut-1 enylamino)-2-hydroxy-N,N-diméthyl-benzamide
37a/ 2-hydroxy-N,N-diméthyl-3-(2-{[(4-méthyl-furan-2-yl)-(3-méthyl-oxetan-3-yl)-méthyl]-amino}-3,4-dioxo-cyclobut-1-enylamino)-benzamide
37b/ 2-Hydroxy-N,N-diméthyl-3-(2-{[(3-méthyl-furan-2-yl)-(3-méthyl-oxetan-3-yl)-méthyl]-amino}-3,4-dioxo-cyclobut-1-enylamino)-benzamide
38/ 2-hydroxy-N,N-diméthyl-3-(2-{[(4-isopropyl-furan-2-yl)-(3-méthyl-oxetan-3-yl)-méthyl]-amino}-3,4-dioxo-cyclobut-1-enylamino)-benzamide
42/ 2-hydroxy-3-(2-{[(5-méthyl-furan-2-yl)-(3-méthyl-oxetan-3-yl)-méthyl]-amino}-3,4-dioxo-cyclobut-1-enylamino)-benzonitrile
43/ (R)-1-[3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle
44/ (S)-1-[4-chloro-3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle
49/ 2-hydroxy-N,N-diméthyl-3-(2-{[((R)-5-méthyl-furan-2-yl)-(3-méthyl-oxetan-3-yl)-méthyl]-amino}-3,4-dioxo-cyclobut-1-enylamino)-benzamide
51/ {[2-hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-méthyl-amino}-acétate de méthyle
52/ 6-chloro-2-hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide
53/ 3-[4-chloro-2-hydroxy-3-(4-méthyl-pipérazine-1-sulfonyl)-phénylamino]-4-{[(R)-(5-méthyl-furan-2-yl)-(3-méthyl-oxetan-3-yl)-méthyl]-amino}-cyclobut-3-ene-1,2-dione

Un second objet selon l'invention concerne une composition pharmaceutique comprenant une quantité efficace d'un composé répondant à la formule générale (I) ou d'un sel pharmaceutiquement acceptable duduit composé tel que décrit ci-dessus en association avec un solvant ou un support pharmaceutiquement acceptable.

Un troisième objet selon l'invention concerne les composés répondant à la formule générale (I), ainsi que leurs sels, solvates ou hydrates pharmaceutiquement acceptable ou encore une composition pharmaceutique comprenant une quantité efficace d'un composé répondant à la formule générale (I), d'un de ses sels ou un de ses solvates ou hydrates pharmaceutiquement acceptable pour leur utilisation en tant que médicament.

Un quatrième objet selon l'invention concerne les composés répondant à la formule générale (I), ainsi que leurs sels, solvates ou hydrates pharmaceutiquement acceptable ou encore une composition pharmaceutique comprenant une quantité efficace d'un composé répondant à la formule générale (I), d'un de ses sels ou un de ses solvates ou hydrates pharmaceutiquement acceptable pour leur utilisation dans le traitement de maladies médiées par les α-chimiokines.

A titre d'exemples de maladies médiées par les α- chimiokines, on peut citer les dermatoses neutrophiliques, notamment le psoriasis, les dermatites atopiques, l'acné, la rosacée, l'asthme, les maladies pulmonaires obstructives chroniques, les maladies respiratoires des adultes, l'arthrite, les maladies intestinales inflammatoires, la maladie de Crohn, le rejet de greffe, la mucoviscidose et les cancers cutanés.

Par dermatoses neutrophiliques, on entend dans son sens le plus large, le syndrome de Sweet, « eccrines hydranite », le syndrome SAPHO, le syndrome de Sneddon Wilkinson, le pyoderma gangrenosum, l'érythème elevatum duitinum, le psoriasis, le psoriasis vulgaire, le psoriasis pustuleux, la pustulose palmo-plantaire, la pustulose exanthématique (PEAG), la pustulose vascularite, l'acro-pustulose de l'enfant, la maladie de Behcet, ainsi que certains maladies bulleuses comme l'herpès dérivés sous forme de dermatite, la dermatose neutrophilique à IgA, IgA intra-épiderme pustilosis, la pemphigoïde bulleuse, le pemphigus à IgA, la vascularite, le syndrome de Leroy Reiter Fiellinger, la pustulose du cuir chevelu, l'acrodermatite continue d' Hallopeau et les dermatoses liées à l'angio-immunoblastique lymphodenopathie, avec dysmieiopoësis induite par le cyclophosphamide, avec des p-ANCA anticorps.

Dans un mode de réalisation préféré selon l'invention, le composé ou la composition pharmaceutique précitée est utilisé dans le traitement de maladies dermatologiques telles que les dermatoses neutrophiliques, notamment le psoriasis, les dermatites atopiques, l'acné et la rosacée.

Un autre aspect de l'invention concerne l'utilisation d'un composé répondant à la formule générale (I), ainsi que leurs sels, solvates ou hydrates pharmaceutiquement acceptable ou encore l'utilisation d'une composition pharmaceutique comprenant une quantité efficace d'un composé répondant à la formule générale (I), d'un de ses sels ou un de ses solvates ou hydrates pharmaceutiquement acceptable pour la préparation d'un médicament pour le traitement des maladies du groupe comprenant les dermatoses neutrophiliques, notamment le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn et les cancers cutanés.

Les composés de formule générale (I) de la présente invention sont préparés suivant une ou plusieurs des voies de synthèse telles que décrites ci-dessous ou telles qu'elles ressortent des différents exemples de préparation donnés ci-après de façon non limitative.

La voie de synthèse générale pour la préparation des composés de formule (III) est illustrée sur le **schéma 1.** Le traitement séquentiel des intermédiaires alkyles squarates (A) avec les amines R'2-NH₂ et R'3-NH₂ donne les composés de formule (III). Dans la formule (A), R'1 est un alkyle C1-C6, de préférence méthyle ou éthyle. La réaction est effectuée dans un solvant inerte et polaire (ou dans un mélange de solvants) comme l'éthanol, le méthanol, le diméthylsulfoxide, le diméthylformamide ou l'acétonitrile. Les amines R'2-NH₂ et R'3-NH₂ peuvent être utilisées comme bases libres ou sous forme de sels. Les réactions peuvent être conduites en présence d'une base convenable comme la triéthylamine, le diisopropyléthylamine, le carbonate de sodium ou le carbonate de potassium et à 25 °C ou de préférence à des températures élevées de 50-80 °C. Le temps de réaction est généralement compris entre 1 heure et72 heures pour avoir une conversion complète.

Les amines R'3-NH₂ de formule (IX) sont préparées selon le **schéma 2** à partir de réactifs commerciaux en utilisant des méthodes bien connues de l'homme du métier décrites dans les manuels de synthèse organique comme par exemple « Comprehensive Organic Functional Group Transformation» Vol. 1-7 A.R.Katritzky, O. Meth-Cohn, C.W.Rees, Pergamon Press, 1998.

Les alcools primaires (IV) [dans lesquels X et R ont la même signification que X et R5 respectivement définis ci-dessus pour les composés de formule générale (I)] sont oxydés en aldéhydes de formule (V) dans les conditions de Swern (Mancuso, A. J.; Huang, S.-L.; Swern, D. (1978). "Oxidation of long-chain and related alcohols to carbonyls by dimethyl sulfoxide "activated" by oxalyl chloride" J. Org. Chem. 43 (12), 2480-2482) ou avec du chlorochromate de pyridinium.

L'aldéhyde de formule (V) est successivement traité avec un réactif de Grignard aryle ou hétéroaryle ou avec un dérivé lithié pour donner un alcool secondaire de formule (VI). Les azotures correspondants (VII) sont préparés à partir des alcools (VI) soit en les transformant en mésylates (VIII) qui sont ensuite traités avec les azotures métalliques (par exemple azoture de sodium), soit en les transformant directement en azoture après traitement avec l'azoture de diphénylphosphoryle (DPPA). L'azoture (VII) est finalement réduit en amine (IX) correspondante par de l'hydrogène en présence de différents catalyseurs (par exemple, palladium sur charbon actif) ou par traitement avec la triphénylphosphine suivi par hydrolyse des intermédiaires imidophosphoranes (Gololobov, Y. G. (1981), "Sixty years of staudinger reaction", Tetrahedron 37 (3), 437).

Alternativement, les amines primaires R'3-NH2 de formule (IX) peuvent être préparées selon le **schéma 3** à partir d'acides commerciaux (X) [dans lesquels X et R ont la même signification que X et R5 respectivement définis ci-dessus pour les composés de formule générale (I)], en les convertissant en amides de Weinreb (XI) (Nahm, S.; Weinreb, S. M. (1981), "N-methoxy-n-methylamides as effective acylating agents", Tetrahedron Letters 22, 3815), qui après réaction avec les réactifs de Grignard aryle ou hétéroaryle ou avec des dérivés lithiés aryle ou hétéroaryle donnent les cétones (XII) qui peuvent être réduites en alcools secondaires (VI).

En suivant les étapes décrites dans le schéma 2, l'alcool (VI) est éventuellement transformé en amine R'3-NH2 de formule (IX).

L'amine primaire chirale R'3-NH2 de structure (XV) peut également être préparée selon le **schéma 4** par condensation du 2-méthyl-2-propanesulfinamide énantiomériquement pur (*tert-*butanesulfinamide, Elman's sulfinamide: Liu, G. et al. J. Am. Soc. Chem. 1997, 119, 9913) avec l'aldéhyde (IV) dans des conditions douces. Cette réaction fournit les *tert*-butanesulfinyl imines (XIII). Le groupement *tert*-butanesulfinyle active les imines pour l'addition des réactifs de Grignard et sert de groupement directeur chiral important pour donner les produits (XIV) avec une diastéréosélectivité élevée. La déprotection du groupement *tert-*butanesulfinyle dans des conditions acides douces donne l'amine chirale (XV).

Les dérivés amide de l'acide 3-aminosalicylique de formule (XVIII) sont préparés selon le **schéma 5a/** à partir de l'acide 3-nitrosalicylique (XVI) en utilisant des conditions de couplage peptidique standard (Recent development of peptide coupling reagents in organic synthesis Tetrahedron, Volume 60(11), 2447-2467, Han, S.-Y.;Kim, Y.-A. ), suivi d'une réduction du groupement nitro en groupement amino par de l'hydrogène en présence d'un catalyseur approprié (par exemple palladium sur charbon actif). Le dérivé (XVIII) réagit ensuite avec le diméthoxysquarate ou diéthoxysquarate commercial pour donner l'intermédiaire (XIX), lequel est transformé en composé (XX) après réaction avec l'amine primaire R'3-NH2.

Alternativement, le couplage de l'acide 3-aminosalicylique (XXI) avec le diméthoxysqyarate ou diéthoxysquarate commercial donne, selon le **schéma 5b/,** le dérivé acide intermédiaire (XXII) qui, après réaction avec l'amine primaire R'3-NH2, peut donner le composé (XXIII). Ce dernier peut enfin être engagé dans une réaction de couplage peptidique avec une amine de formule RaRbNH pour conduire au composé de formule (XX).

A titre d'illustration, les composés suivants répondant à la formule générale (I) de la présente invention ont été préparés en suivant l'un des schémas présentés ci-dessus.

### EXEMPLE 1

### Préparation du 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzamide

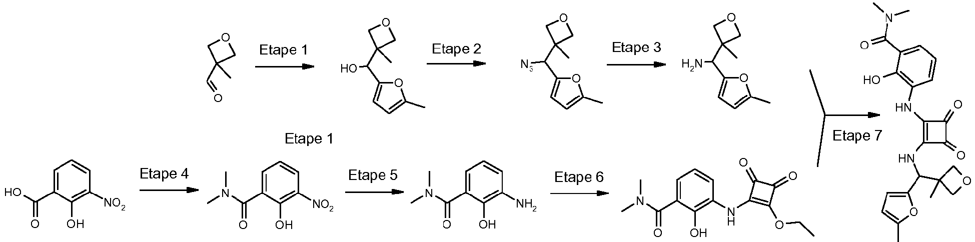

### Etape 1:

### (5-Méthyl-furan-2-yl)-(3-méthyl-oxetan-3-yl)-méthanol

12.0 mL (30 mmol, 1.7 éq) d'une solution de n-butyllithium 2.5 M dans l'hexane ont été ajoutés goutte à goutte sur une solution de 2.46 g (30 mmol, 1.7 éq) de 2-méthylfurane dans 50 mL de tétrahydrofuranne refroidie à -70°C. Le milieu réactionnel a été agité et laissé remonter à température ambiante pendant 3 heures. Le milieu réactionnel a été refroidi à - 70°C puis 2.17 g (18 mmol, 1 éq) de 3-méthyl-oxétane-3-carbaldéhyde à 83% ont été ajoutés. Le milieu réactionnel a été agité à température ambiante pendant 3 heures. Le milieu réactionnel a été traité avec une solution saturée de chlorure d'ammonium et extrait à l'acétate d'éthyle. Les phases organiques ont été rassemblées, lavées avec une solution saturée de chlorure de sodium et évaporées.

3.02 g de (5-méthyl-furan-2-yl)-(3-méthyl-oxetan-3-yl)-methanol ont été obtenus sous forme d'une huile orange. Rendement = 92%.

### Etape 2:

### 2-[Azido-(3-méthyl-oxetan-3-yl)-méthyl]-5-méthyl-furane

5.30 g (19 mmol, 1.1 éq) de diphenylphosphoryl azide ont été ajoutés goutte à goutte sur une solution de 3.02 g (17 mmol, 1 éq) de (5-méthyl-furan-2-yl)-(3-méthyl-oxetan-3-yl)-méthanol dans 50 mL de toluène. Le milieu réactionnel a été refroidi à 0°C puis 2.9 mL (19 mmol, 1.1 éq) de 1,8-diazabicyclo[5.4.0]undec-7-ene ont été ajoutés goutte à goutte. Le milieu réactionnel a été agité à température ambiante pendant 23 heures. Le milieu réactionnel a été décanté et la phase organique a été lavée à l'eau puis à l'acide chlorhydrique IN, séchée sur sulfate de magnésium, filtrée et évaporée. Le résidu a été chromatographié sur gel de silice (colonne Analogix SF40-150g, Spot II) élué à l'heptane/acétate d'éthyle (95/5). 1.68 g de 2-[azido-(3-méthyl-oxetan-3-yl)-méthyl]-5-méthyl-furane ont été obtenus sous forme d'une huile orange. Rendement = 48%.

### Etape 3:

### C-(5-Méthyl-furan-2-yl)-C-(3-méthyl-oxetan-3-yl)-méthylamine

Une solution de 1.68 g (8 mmol, 1 éq) de 2-[azido-(3-méthyl-oxetan-3-yl)-méthyl]-5-méthyl-furane dans 30 mL d'éthanol en présence de 252 mg (15% en masse) de palladium sur charbon à 10% a été agitée sous pression atmosphérique d'hydrogène pendant 3 heures. Le milieu réactionnel a été filtré et le filtrat a été évaporé. 1.42 g de C-(5-méthyl-furan-2-yl)-C-(3-méthyl-oxetan-3-yl)-méthylamine ont été obtenus sous forme d'une huile verdâtre. Rendement = 98%.

### Etape 4:

### 2-Hydroxy-N,N-diméthyl-3-nitro-benzamide

42.9 mL (0.50 mol, 3 éq) de chlorure d'oxalyle ont été ajoutés goutte à goutte sur une suspension de 30 g (0.16 mol, 1 éq) d'acide 3-nitrosalicylique dans 1200 mL de dichlorométhane. 30 gouttes de N,N-diméthylformamide ont été ajoutées (fort dégagement de gaz, adaptation d'un système de piégeage de vapeurs de monoxide de carbone toxique). Le milieu réactionnel a été agité à température ambiante pendant 24 heures. Le milieu réactionnel a été refroidi à 0-5°C puis 246 mL (0.49 mol, 3 éq) d'une solution de diméthylamine dans tétrahydrofurane 2N ont été ajoutés. Le milieu réactionnel a été agité à température ambiante pendant 2 Jours. Le milieu réactionnel a été concentré à sec et le résidu a été mis en solution dans 300 mL de soude IN. La solution aqueuse (rouge) a été extraite 3 fois avec 300 mL de dichlorométhane. La phase aqueuse a été refroidie dans un bain d'eau-glace, et le pH a été ajusté à 2 avec environ 50 mL d'acide hydrochlorique 6N. Le mélange (devenu jaune) a été extrait 3 fois avec 300 mL de dichlorométhane. Les phases organiques ont été rassemblées, lavées 2 fois avec 250 mL d'eau puis une fois avec 250 mL d'une solution saturée de chlorure de sodium, séchées sur sulfate de magnésium anhydre et évaporées. 33.5 g de 2-hydroxy-N,N-diméthyl-3-nitro-benzamide ont été obtenus sous forme d'un solide coton jaune. Rendement = 97%.

### Etape 5:

### 3-Amino-2-hydroxy-N,N-diméthyl-benzamide

Une solution de 33.5 g de 2-hydroxy-N,N-diméthyl-3-nitro-benzamide dans 600 mL d'éthanol ont été ajoutés sur une suspension de 3.35 g de Pd/C 10% dans 70 mL d'éthanol. Le milieu réactionnel a été agité sous 2 bars d'hydrogène pendant la nuit. Contrôle CCM et HPLC (t=0.66 M+181). Le milieu réactionnel a été filtré sur célite et le filtrat a été évaporé. 29 g de 3-amino-2-hydroxy-N,N-diméthyl-benzamide ont été obtenus sous forme d'un solide marron huileux. Rendement = 100%.

### Etape 6:

### 3-(2-Éthoxy-3,4-dioxo-cyclobut-1-enylamino)-2-hydroxy-N,N-diméthyl-benzamide

Sous azote et à température ambiante, 39.7 g de diéthoxysquarate ont été ajoutés (en 15 minutes) sur une solution de 28 g de 3-amino-2-hydroxy-N,N-diméthyl-benzamide dans 840 mL d'éthanol refroidie à 0°C. Le milieu réactionnel a été agité pendant 2 heures à 0°C et 48 heures à température ambiante. 700 mL d'éthanol ont été rajoutés (ce qui augmente la précipitation du produit attendu). Le solide a été filtré, lavé à l'éthanol ambiant et séché. 36.9 g de 3-(2-éthoxy-3,4-dioxo-cyclobut-1-enylamino)-2-hydroxy-N,N-diméthyl-benzamide ont été obtenus sous forme d'un solide vert kaki clair. Rendement = 78%.

### Etape 7:

### 2-Hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzamide

1.42 g (7.8 mmol, 2 éq) de C-(5-méthyl-furan-2-yl)-C-(3-méthyl-oxetan-3-yl)-méthylamine ont été ajoutés sur 1.19 g (3.9 mmol, 1 éq) de 3-(2-éthoxy-3,4-dioxo-cyclobut-1-enylamino)-2-hydroxy-N,N-diméthyl-benzamide solubilisés à chaud dans 100 mL de méthanol. Le milieu réactionnel a été agité à température ambiante pendant 22 heures (75% de produit formé au bout de 6 heures). Le méthanol a été évaporé et le résidu (huile verte) a été chromatographié sur gel de silice (colonne Analogix SF40-150g, Spot II) élué au dichlorométhane/méthanol 98/2. Le solide amorphe a été repris avec de l'éther diéthylique, filtré et séché sous vide à 45°C.

1.51 g de 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzamide ont été obtenus sous forme d'un solide jaune. Rendement = 88%. (Tf= 196-198°C); LC/MS: 98.69% [439].

RMN ¹H (DMSO-d6, 400 MHz)_: 1.32 (s, 3H) ; 2.26 (s, 3H) ; 2.94 (s, 6H) ; 4.29 (dd, *J*=6.2 Hz, 2H) ; 4.6 (dd, *J*=28.8 Hz, 2H) ; 5.6 (d, *J*=9.7 Hz, 1H) ; 6.06 (d, *J*=2.1 Hz, 1H), 6.25 (d, J=3.1 Hz, 1H) ; 6.88 (dd, *J*=11.2 Hz, 2H) ; 7.76 (q, *J*=9.3 Hz, 1H) ; 8.83 (d, *J*=9.7 Hz, 1H) ; 9.45 (s, 1H).

### EXEMPLE 2

### 3-(2-{[(3-Fluorométhyl-oxetan-3-yl)-(5-méthyl-furan-2-yl)-méthyl]-amino}-3,4-dioxo-cyclobut-1-enylamino)-2-hydroxy-N,N-diméthyl-benzamide

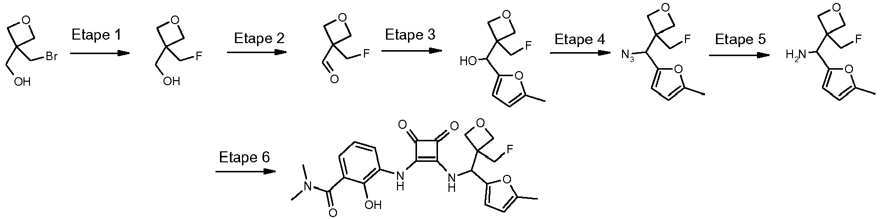

### Etape 1:

### (3-Fluorométhyl-oxetan-3-yl)-methanol

Un mélange de 21.5 g (0.119 mol, 1 éq) de 3-bromométhyl-3-hydroxyméthyloxetane et de 27.7 g (0.476 mol, 4 éq) de fluorure de potassium dans 60 mL de diéthylène glycol a été chauffé à 150°C pendant 3 heures. Le milieu réactionnel a été dilué avec 120 mL d'eau et extrait à l'éther diéthylique (18 x 30 mL) puis à l'acétate d'éthyle (8 x 50 mL). Les phases organiques ont été rassemblées, séchées sur sulfate de magnésium et évaporées. Le résidu a été chromatographié sur gel de silice (colonne *Super*Flash SF40-240g) élué à l'heptane/acétate d'éthyle 30/70 (élimination du diéthylène glycol). 10.55 g de (3-fluorométhyl-oxetan-3-yl)-methanol ont été récupérés sous forme d'une huile incolore. Rendement = 74%. CCM SiO₂/Heptane:AcOEt (20/80), révélation au KMnO₄.

### Etape 2:

### 3-Fluorométhyl-oxetane-3-carbaldéhyde

Une solution de 5.3 g (44 mmol, 1 éq) de (3-fluorométhyl-oxetan-3-yl)-méthanol dans 50 mL de dichlorométhane a été ajoutée goutte à goutte sur un mélange de 15.2 g (70 mmol, 1.6 éq) de pyridinium chlorochromate dans 220 mL de dichlorométhane. 5.3 g de celite ont été ajoutés et le milieu réactionnel a été agité à température ambiante pendant 7 heures. Le milieu réactionnel a été filtré sur 75 g de silice et élué au dichlorométhane. 2.16 g de 3-fluorométhyl-oxetane-3-carbaldéhyde ont été obtenus sous forme d'un liquide Jaunâtre. Rendement = 42% (présence de ∼9% de dichlorométhane). CCM SiO₂/CH₂Cl₂:MeOH (96/4), révélation au KMnO₄

### Etape 3:

### (3-Fluorométhyl-oxetan-3-yl)-(5-méthyl-furan-2-yl)-méthanol

11.0 mL (27 mmol, 1.5 éq) d'une solution de n-butyllithium 2.5 M dans l'hexane ont été ajoutés goutte à goutte sur une solution de 2.25 g (27 mmol, 1.5 éq) de 2-méthylfurane dans 40 mL de tétrahydrofuranne refroidie à -70°C. Le milieu réactionnel a été agité et laissé remonter à température ambiante pendant 3 heures. Le milieu réactionnel a été refroidi à - 70°C puis 2.16 g (18 mmol, 1 éq) de 3-fluorométhyl-oxetane-3-carbaldehyde ont été ajoutés. Le milieu réactionnel a été agité à température ambiante pendant 2 heures et demie. Le milieu réactionnel a été traité avec une solution saturée de chlorure d'ammonium et extrait à l'acétate d'éthyle. Les phases organiques ont été rassemblées, lavées avec une solution saturée de chlorure de sodium et évaporées. 3.83 g de (3-fluorométhyl-oxetan-3-yl)-(5-méthyl-furan-2-yl)-methanol ont été obtenus sous forme d'une huile orange foncé. Rendement = 71%. CCM SiO₂/CH₂Cl₂:MeOH (96/4), révélation au KMnO₄

### Etape 4:

### 2-[Azido-(3-fluorométhyl-oxetan-3-yl)-méthyl]-5-méthyl-furane

5.80 g (21 mmol, 1.1 éq) de diphénylphosphoryl azide ont été ajoutés goutte à goutte sur une solution de 3.83 g (19 mmol, 1 éq) de (3-fluorométhyl-oxetan-3-yl)-(5-méthyl-furan-2-yl)-méthanol dans 50 mL de toluène. Le milieu réactionnel a été refroidi à 0°C puis 3.15 mL (21 mmol, 1.1 éq) de 1,8-diazabicyclo[5.4.0]undec-7-ene ont été ajoutés goutte à goutte. Le milieu réactionnel a été agité à température ambiante pendant 15 heures. Le milieu réactionnel (hétérogène) a été traité à l'eau et à l'acétate d'éthyle puis décanté. La phase organique a été lavée à l'acide chlorhydrique IN, séchée sur sulfate de magnésium, filtrée et évaporée. Le résidu a été chromatographié sur gel de silice (colonne puriFlash IR-50SI/200G, Spot II) élué à l'heptane / acétate d'éthyle (91/9). 2.25 g de 2-[azido-(3-fluorométhyl-oxetan-3-yl)-méthyl]-5-méthyl-furan ont été obtenus sous forme d'une huile jaune. Rendement = 53%. CCM SiO₂/Heptane:AcOEt (80/20), révélation au KMnO₄

### Etape 5:

### C-(5-Méthyl-furan-2-yl)-C-(3-fluorométhyl-oxetan-3-yl)-méthylamine

Une solution de 2.25 g (10 mmol, 1 éq) de 2-[azido-(3-fluorométhyl-oxetan-3-yl)-méthyl]-5-méthyl-furane dans 40 mL d'éthanol en présence de 378 mg (17% en masse) de palladium sur charbon à 10% a été agitée sous pression atmosphérique d'hydrogène pendant 2 heures et demie. Le milieu réactionnel a été filtré et le filtrat a été évaporé. 2.0 g de C-(5-méthyl-furan-2-yl)-C-(3-fluorométhyl-oxetan-3-yl)-méthylamine ont été obtenus sous forme d'une huile grisâtre. Rendement = 100%. CCM SiO₂/Heptane:AcOEt (70/30), révélation au KMnO₄

### Etape 6:

### 3-(2-{[(3-Fluorométhyl-oxetan-3-yl)-(5-méthyl-furan-2-yl)-méthyl]-amino}-3,4-dioxo-cyclobut-1-enylamino)-2-hydroxy-N,N-diméthyl-benzamide

1.50 g (7.5 mmol, 1.5 éq) de C-(5-méthyl-furan-2-yl)-C-(3-fluorométhyl-oxetan-3-yl)-méthylamine ont été ajoutés sur 1.52 g (5.0 mmol, 1 éq) de 3-(2-éthoxy-3,4-dioxo-cyclobut-1-enylamino)-2-hydroxy-N,N-diméthyl-benzamide solubilisés à chaud dans 100 mL de méthanol. Le milieu réactionnel a été chauffé à 50°C pendant 16 heures puis à 60°C pendant 6 heures. Le méthanol a été évaporé et le résidu (huile verte) a été chromatographié sur gel de silice (colonne puriFlash IR50SI-120G, Spot II) élué au dichlorométhane / méthanol 98/2. Le solide amorphe a été repris avec de l'éther diéthylique, filtré et séché sous vide à 45°C.

1.85 g de 3-(2-{[(3-fluorométhyl-oxetan-3-yl)-(5-méthyl-furan-2-yl)-méthyl]-amino}-3,4-dioxo-cyclobut-1-enylamino)-2-hydroxy-N,N-diméthyl-benzamide ont été obtenus sous forme d'un solide beige. (Tf= 186-188°C). Rendement = 81%. LC/MS: 100% [457]

RMN ¹H (DMSO-d6, 400 MHz)_: 2.26 (s, 3H) ; 2.94 (s, 6H) ; 4.47 (t, 2H) ; 4.59-4.81 (m, 4H) ; 5.7 (d, *J*=9.7 Hz, 1H) ; 6.08 (d, *J*=2.1 Hz, 1H), 6.3 (d, *J*=3.0 Hz, 1H) ; 6.88 (m, 2H) ; 7.75 (q, *J*=9.5 Hz, 1H) ; 8.95 (d, *J*=12.6 Hz, 1H) ; 9.48 (s, 1H).

### EXEMPLE 3

### Préparation du 3-(2-{[(3-Ethyl-oxetan-3-yl)-(5-méthyl-furan-2-yl)-méthyl]-amino}-3,4-dioxo-cyclobut-1-enyl-amino)-2-hydroxy-N,N-diméthyl-benzamide

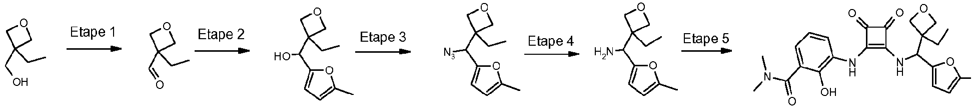

### Etape 1:

### 3-Ethyl-oxetane-3-carbaldéhyde

Une solution de (3-éthyloxetan-3-yl)méthanol (5.0 g, 43.0 mmol) dans 60 mL de dichlorométhane a été ajoutée goutte à goutte sur un mélange de chlorochromate de pyridinium (14.85 g, 68.9 mmol) et de celite (4.30 g) dans 200 mL de dichlorométhane. Le milieu réactionnel a été agité à température ambiante pendant 5 heures. Le milieu réactionnel a été filtré sur 70 g de silice et élué au dichlorométhane. Les phases organiques ont été rassemblées et concentrées. 4.0 g du produit attendu ont été obtenus sous forme d'une huile vert pâle. Rendement = 82.3 %.

### Etape 2:

De manière analogue à l'EXEMPLE 1 (étape 1), (3-ethyl-oxetan-3-yl)-(5-méthyl-furan-2-yl)-methanol a été préparé. Rendement = 82%.

### Etape 3:

De manière analogue à l'EXEMPLE 1 (étape 2), 2-[azido-(3-éthyl-oxetan-3-yl)-méthyl]-5-méthyl-furane a été préparé. Rendement = 53%.

### Etape 4:

De manière analogue à l'EXEMPLE 1 (étape 3), C-(3-éthyl-oxetan-3-yl)-C-(5-méthyl-furan-2-yl)-méthylamine a été préparé. Rendement = 94%.

### Etape 5:

De manière analogue à l'EXEMPLE 1 (étape 7), 3-(2-{[(3-éthyl-oxetan-3-yl)-(5-méthyl-furan-2-yl)-méthyl]-amino}-3,4-dioxo-cyclobut-1-enyl-amino)-2-hydroxy-N,N-diméthyl-benzamide a été préparé. Rendement = 66%, LC / MS: 97.0% ES+ [454].

RMN ¹H (DMSO-d6, 400 MHz): 0.95 (t, J=7.4Hz, 3H) ; 1.43 (m, 1H) ; 1.79 (m, 1H) ; 2.26 (s, 3H) ; 2.94 (s, 6H) ; 4.34-4.40 (m, 2H) ; 4.55 (d, 1H) ; 4.74 (d, 1H) ; 5.55 (d, 1H) ; 6.06 (m, 1H) ; 6.27 (d, 1H) ; 6.87-6.92 (m, 2H) ; 7.75-7.78 (dm, 1H) ; 8.95 (d, 1H) ; 9.46 (s, 1H) ; 10.00 (s, 1H).

### EXEMPLE 4

### Préparation de l'acide 2-hydroxy-3-(2-{[(5-méthyl-furan-2-yl)-(3-méthyl-oxetan-3-yl)-méthyl]-amino}-3,4-dioxo-cyclobut-1-enylamino)-benzoïque

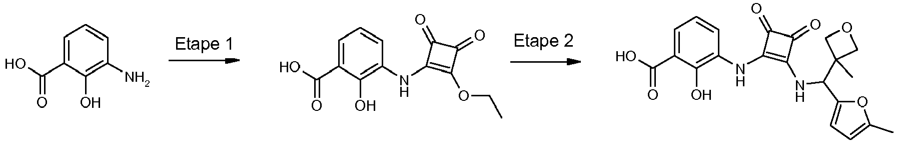

### Etape 1:

### Acide 3-(2-éthoxy-3,4-dioxo-cyclobut-1-enylamino)-2-hydroxybenzoïque

Un mélange de 1.68 g (11 mmol, 1.1 éq) d'acide 3-aminosalicylique et de 1.70 g (10 mmol, 1 éq) de 3,4-diéthoxy-3-cyclobutene-1,2-dione dans 15 mL d'éthanol a été chauffé à 50°C pendant 3 heures et demie puis à 60°C pendant 20 heures (66% produit formé) et à 70°C pendant 4 heures (55% produit formé). Le milieu réactionnel a été filtré et le filtrat a été chromatographié sur gel de silice (colonne puriFlash IR-50SI/120G, Spot II) élué au dichlorométhane/méthanol (gradient). Le solide a été repris avec un peu d'acétate d'éthyle, filtré et séché sous vide à 55°C. 1.04 g de 3-(2-éthoxy-3,4-dioxo-cyclobut-1-enylamino)-2-hydroxy-benzoic acid ont été obtenus sous forme d'un solide jaune-beige. Rendement = 38%.

### Etape 2:

### Acide 2-hydroxy-3-(2-{[(5-méthyl-furan-2-yl)-(3-méthyl-oxetan-3-yl)-méthyl]-amino}-3,4-dioxo-cyclobut-1-enylamino)-benzoïque

453 mg (2.5 mmol, 1.5 éq) de C-(5-méthyl-furan-2-yl)-C-(3-méthyl-oxetan-3-yl)-méthylamine ont été ajoutés sur 471 mg (1.7 mmol, 1 éq) de d'acide 3-(2-éthoxy-3,4-dioxo-cyclobut-1-enylamino)-2-hydroxybenzoïque solubilisés à chaud dans 50 mL de méthanol. Le milieu réactionnel a été chauffé à 50°C pendant 18 heures puis à 60°C pendant 7 Jours. Le méthanol a été évaporé et le résidu a été chromatographié sur gel de silice (colonne puriFlash IR50SI-80G, Spot II) élué au dichlorométhane / méthanol (gradient). Le solide a été repris avec un peu d'acétate d'éthyle, filtré et séché sous vide à 55°C. 310 mg d'acide 2-hydroxy-3-(2-{[(5-méthyl-furan-2-yl)-(3-méthyl-oxetan-3-yl)-méthyl]-amino}-3,4-dioxo-cyclobut-1-enylamino)-benzoïque ont été obtenus sous forme d'un solide marron. (Tf = 180-185°C).
Rendement = 44%. LC/MS: 97.08% [412]
RMN ¹H (DMSO-d6, 400 MHz)_: 1.32 (s, 3H) ; 2.26 (s, 3H) ; 4.29 (dd, J=6.2 Hz, 2H) ; 4.6 (dd, J=28.8 Hz, 2H) ; 5.66 (d, J=9.8 Hz, 1H) ; 6.06 (d, J=3.1 Hz, 1H), 6.24 (d, J=3.1 Hz, 1H) ; 6.46 (t, J=7.8 Hz, 1H) ; 7.33 (d, J=7.7 Hz, 1H) ; 7.79 (d, J=7.8 Hz, 1H) ; 8.90 (d, J=9.8 Hz, 1H) ; 9.46 (s, 1H).

### EXEMPLE 5

### Préparation du 3-[2-hydroxy-3-((R)-3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène-1,2-dione

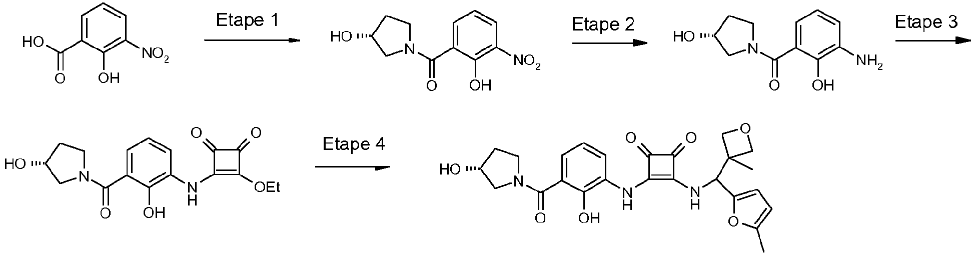

### Etape 1:

### (2-Hydroxy-3-nitro-phenyl)-((R)-3-hydroxy-pyrrolidin-1-yl)-méthanone

Un mélange de 4.86 g (26 mmol, 1 éq) d'acide 3-nitrosalicylique et de 12.16 g (26 mmol, 1 éq) de bromotripyrrolidinophosphonium hexafluorophosphate dans 65 mL de dichlorométhane et en présence de 13.7 mL (78 mmol, 3 éq) de N,N-düsopropyléthylamine a été agité à température ambiante pendant 30 minutes. 4.52 g (4.10 mmol, 2 éq) de (R)-(+)-3-pyrrolidinol en solution dans 10 mL de dichlorométhane ont été ajoutés goutte à goutte et le milieu réactionnel a été agité à température ambiante pendant la nuit. Le milieu réactionnel a été extrait avec une solution de soude IN et décanté. La phase aqueuse a été acidifiée avec une solution d'acide chlorhydrique 1 N et extraite à l'acétate d'éthyle (5x150 mL). Les phases organiques ont été rassemblées, séchées sur sulfate de magnésium, filtrées et évaporées. 7.83 g de (2-hydroxy-3-nitro-phenyl)-((R)-3-hydroxy-pyrrolidin-1-yl)-methanone ont été obtenus sous forme d'un solide amorphe Jaune d'or. Rendement brut > 100%. HPLC : 93% [252].

### Etape 2:

### (3-Amino-2-hydroxy-phenyl)-((R)-3-hydroxy-pyrrolidin-1-yl)-méthanone

Une solution de 7.83 g (26 mmol, 1 éq) de (2-hydroxy-3-nitro-phényl)-((R)-3-hydroxy-pyrrolidin-1-yl)-méthanone à 84% dans 100 mL de méthanol a été agité sous pression atmosphérique d'hydrogène en présence de 728 mg (10% en masse) de palladium sur charbon à 10% pendant 16 heures. Le milieu réactionnel a été filtré et le filtrat a été évaporé. Le résidu a été chromatographié sur gel de silice (colonne puriFlash IR-50SI/300G, spot II) élué au dichlorométhane / méthanol (96/4). 3.63 g de (3-amino-2-hydroxy-phenyl)-((R)-3-hydroxy-pyrrolidin-1-yl)-methanone ont été obtenus sous forme d'un solide amorphe Jaune. Rendement = 63%. SiO₂ / CH₂Cl₂ 90 : MeOH 10, révélation au KMnO₄.

### Etape 3:

### 3-Éthoxy-4-[2-hydroxy-3-((R)-3-hydroxy-pyrrolidine-1-carbonyl)-phénylamino]-cyclobut-3-ène-1,2-dione

Un mélange de 3.63 g (16 mmol, 1 éq) de (3-amino-2-hydroxy-phényl)-((R)-3-hydroxy-pyrrolidin-1-yl)-méthanone et de 3.40 g (20 mmol, 1.2 éq) de 3,4-diéthoxy-3-cyclobutène-1,2-dione dans 70 mL d'éthanol a été chauffé à 60°C pendant 16 heures. Le milieu réactionnel a été filtré et le filtrat a été chromatographié sur gel de silice (colonne puriFlash IR-50SI/300G, Spot II avec dépôt solide) élué au dichlorométhane/méthanol (gradient). 3.67 g de 3-éthoxy-4-[2-hydroxy-3-((R)-3-hydroxy-pyrrolidine-1-carbonyl)-phenylamino]-cyclobut-3-ène-1,2-dione ont été obtenus sous forme d'un solide beige. Rendement = 66%.

### Etape 4:

### 3-[2-Hydroxy-3-((R)-3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène-1,2-dione

544 mg (3 mmol, 1.5 éq) de C-(5-Méthyl-furan-2-yl)-C-(3-méthyl-oxetan-3-yl)-méthylamine ont été ajoutés sur 693 mg (2 mmol, 1 éq) de 3-éthoxy-4-[2-hydroxy-3-((R)-3-hydroxy-pyrrolidine-1-carbonyl)-phénylamino]-cyclobut-3-ène-1,2-dione solubilisés à chaud dans 30 mL de méthanol. Le milieu réactionnel a été chauffé à 50°C pendant 3 Jours puis à 60°C pendant 5 heures. Le méthanol a été évaporé et le résidu a été chromatographié sur gel de silice (colonne puriFlash IR-50SI/80G, Spot II) puis ensuite sur gel de silice (colonne puriFlash PF-15SIHP/25Gx2, Spot II) élué au dichlorométhane/méthanol (gradient). 694 mg de 3-[2-hydroxy-3-((R)-3-hydroxy-pyrrolidine-1-carbonyl)-phenylamino]-4- [(5-méthyl-furan-2-yl)-(3-méthyl-oxetan-3-yl)-méthyl]-amino}-cyclobut-3-ène-1,2-dione ont été obtenus sous forme d'un solide amorphe orange. Rendement = 72%. LC/MS: 99.6% [481]

RMN ¹H (DMSO-d6, 400 MHz)_: 1.32 (s, 3H) ; 1.83-1.94 (m, 2H) ; 2.26 (s, 3H) ; 3.38 (m, 2H) ; 3.57 (m, 1H) ; 3.68 (m, 2H) ; 4.29 (dd, J=6.2 Hz, 2H) ; 4.6 (dd, J=29.2 Hz, 2H) ; 5.02 (s, 1H) ; 5.64 (d, J=9.7 Hz, 1H) ; 6.06 (dd, J=3.0 Hz, 1H), 6.26 (d, J=3.1 Hz, 1H) ; 6.90 (t, J=8.0 Hz, 1H) ; 7.16 (d, J=6.9 Hz, 1H) ; 7.85 (d, J=7.8 Hz, 1H) ; 8.85 (dd, J=9.7 Hz, 1H) ; 9.50 (s, 1H).

### EXEMPLE 6

### Préparation du 3-[2-hydroxy-3-((S)-3-hydroxy-pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione

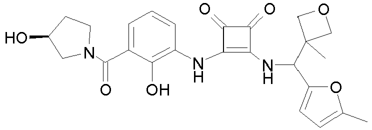

De manière analogue à l'EXEMPLE 5 (étapes 1 à 4), 3-[2-hydroxy-3-((S)-3-hydroxy-pyrrolidine-1-carbonyl)-phenylamino]-4-[(5-méthyl-furan-2-yl)-(3-méthyl-oxetan-3-yl)-méthyl]-amino}-cyclobut-3-ène-1,2-dione a été préparé. HPLC = 98.87 % (mélange de deux diastéréoisomères 53.84 % + 45.03 %).

RMN ¹H (méthanol-d4, 400 MHz)_: = 1.40 (s, 3H); 1.90-2.15 (m, 2H); 2.28 (s, 3H); 3.47-3.65 (m, 1H); 3.66-3.90 (m, 3H); 4.35-4.55 (m, 3H); 4.79 (d, J=6.3 Hz, 1H); 4.82-5.00 (m, 1H); 5.67 (s, 1H); 6.00 (dd, J=2.8 Hz, J=0.7 Hz, 1H); 6.23 (d, J=3.0 Hz, 1H); 6.92 (t, J=8.0 Hz, 1H); 7.15-7.25 (m, 1H); 8.05 (dl, J=7.9 Hz, 1H).

### EXEMPLE 7

### Préparation du (R)-1-[2-hydroxy-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylic acid tert-butyl ester

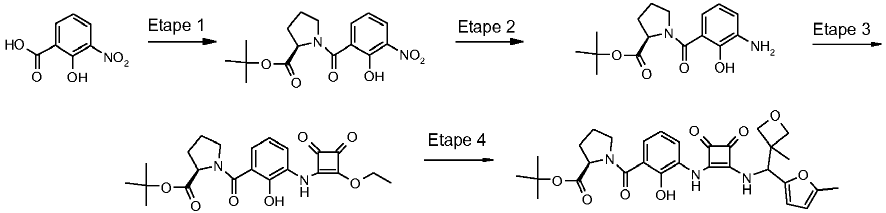

### Etape 1:

De manière analogue à l'EXEMPLE 5 (étape 1), (R)-1-(2-hydroxy-3-nitro-benzoyl)-pyrrolidine-2-carboxylate de tert-butyle a été préparé. Rendement = 68%.

### Etape 2:

De manière analogue à l'EXEMPLE 5 (étape 2), (R)-1-(3-amino-2-hydroxy-benzoyl)-pyrrolidine-2-carboxylate de tert-butyle a été préparé. Rendement = 87%.

### Etape 3:

De manière analogue à l'EXEMPLE 5 (étape 3), (R)-1-[3-(2-éthoxy-3,4-dioxo-cyclobut-1-enylamino)-2-hydroxy-benzoyl]-pyrrolidine-2-carboxylate de tert-butyle a été préparé. Rendement = 66%.

### Etape 4:

### (R)-1-[2-Hydroxy-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de tert-butyle

Un mélange de 253 mg (1.44 mmol, 1.2 éq) de C-(5-méthyl-furan-2-yl)-C-(3-méthyl-oxetan-3-yl)-méthylamine et de 516 g (1.2 mmol, 1 éq) de (R)-1-[3-(2-éthoxy-3,4-dioxo-cyclobut-1-enylamino)-2-hydroxy-benzoyl]-pyrrolidine-2-carboxylic acid tert-butyl ester dans 10 mL de méthanol a été chauffé à 50°C pendant 4 Jours. Le milieu réactionnel a été évaporé et le résidu a été chromatographié sur gel de silice HP (colonne Redi*Sep R*f Gold 40g, Spot II) élué dichlorométhane/méthanol (gradient). Le solide amorphe a été repris avec un peu d'éther diéthylique, filtré et séché sous vide à 50°C.

476 mg de (R)-1-[2-hydroxy-3-(2-[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de tert-butyle ont été obtenus. (Tf = 162-164°C). Rendement = 70%. LC/MS: 98.7% [565] : présence de 2 diastéréoisomères.

RMN ¹H (DMSO-d6, 400 MHz)-: 1.20 (s, 3H) ; 1.32 (s, 3H) ; 1.42 (s, 6H) ; 1.89 (m, 3H) ; 2.24 (m, 1H) ; 2.26 (s, 3H) ; 3.61 (m, 2H) ; 4.29 (dd, J=6.0 Hz, 2H) ; 4.40 (s, 1H) ; 4.6 (dd, J=28.9 Hz, 2H) ; 5.64 (d, J=9.8 Hz, 1H) ; 6.06 (dd, J=3.0 Hz, 1H), 6.26 (d, J=3.0 Hz, 1H) ; 6.91-7.15 (m, 2H) ; 7.75-7.87 (m, 1H) ; 8.83 (d, J=9.8 Hz, 1H) ; 9.48 (s, 1H).

### EXEMPLE 8

### Préparation du (R)-1-[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle

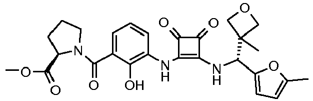

De manière analogue à l'EXEMPLE 7 (étape 1 à 4), (R)-1-[2-hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle a été préparé.

Présence de deux conformères, description du conformère majoritaire à environ 80 % : RMN ¹H (DMSO-d6, 400 MHz) 1.32 (s, 3H), 1.80-2.00 (m, 3H), 2.15-2.35 (m, 4H), 3.55-3.75 (m, 5H), 4.27-4.30 (m, 2H), 4.50-4.60 (m, 2H), 4.65 (d, J = 6.2 Hz, 1H), 5.64 (d, J = 9.8 Hz, 1H), 6.06 (dd, J = 3.1 Hz, J = 1.0 Hz, 1H), 6.26 (d, J = 3.1 Hz, 1H), 6.93 (t, J = 7.9 Hz, 1H), 7.14 (dl, J = 7.52 Hz, 1H), 7.86 (dl, J = 7.8 Hz, 1H), 8.84 (dl, J = 9.72 Hz, 1H), 9.49 (sl, 1H).

### EXEMPLE 9

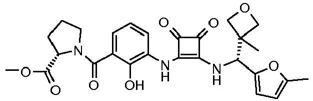

### Préparation du (S)-1-[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle

De manière analogue à l'EXEMPLE 7 (étape 1 à 4), (S)-1-[2-hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle a été préparé.

RMN ¹H /DMSO-d6, 400 MHz 1.33 (s, 3H) ; 1.88-1.92 (m, 3H) ; 2.26-2.33 (m,4H) ; 3.36-3.41 (m, 1H) ; 3.62-3.68 (m, 4H) ; 4.28-4.36 (m, 2H) ; 4.55-4.60 (m, 2H) ; 4.65 (d, j=6.2Hz, 1H) ; 5.65 (d, j=9.7Hz, 1H) ; 6.06 (m, 1H) ; 6.25 (d, j=3.0Hz, 1H) ; 6.94 (d, j=6.7Hz, 1H) ; 7.15 (d, j=7.5Hz, 1H) ; 7.86 (d, j=7.4Hz, 1H) ; 8.83 (d, j=9.7Hz, 1H) ; 9.49 (s, 1H) ; 10.00-11.20 (m, 1H)

### EXEMPLE 10

### Préparation de l'acide (R)-1-[2-hydroxy-3-(2-{[(5-méthyl-furan-2-yl)-(3-méthyl-oxetan-3-yl)-méthyl]-amino}-3,4-dioxocyclobut-1-enylamino)-benzoyl]-pyrrolidine-2-carboxylique

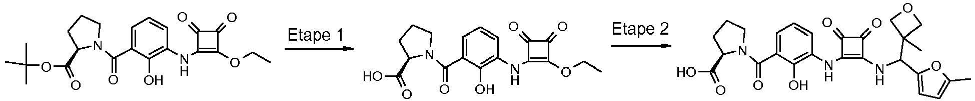

### Etape 1:

### Acide (R)-1-[3-(2-éthoxy-3,4-dioxo-cyclobut-1-enylamino)-2-hydroxybenzoyl]-pyrrolidine-2-carboxylique

1.00 g (2.32 mmol) de (R)-1-[3-(2-éthoxy-3,4-dioxo-cyclobut-1-enylamino)-2-hydroxy-benzoyl]-pyrrolidine-2-carboxylate de tert-butyle ont été mis en solution dans 5 mL d'acide trifluoroacétique. 25 minutes plus tard, le milieu réactionnel a été concentré à sec et a été repris dans du toluène avant d'être à nouveau concentré. Une meringue rose a été obtenue. Cette meringue a été reprise avec de l'éther diéthylique sous agitation puis filtrée. 0.75 g a été obtenu sous forme d'un solide beige. Rendement = 86%.

### Etape 2:

### Acide (R)-1-[2-hydroxy-3-(2-{[(5-méthyl-furan-2-yl)-(3-méthyl-oxetan-3-yl)-méthyl]-amino}-3,4-dioxocyclobut-1-enylamino)-benzoyl]pyrrolidine-2-carboxylique

A une solution de 500 mg (1.34 mmol) de (R)-1-[3-(2-éthoxy-3,4-dioxo-cyclobut-1-enylamino)-2-hydroxy-benzoyl]-pyrrolidine-2-carboxylic acid dans 47 mL de méthanol à 50 °C ont été rajoutés 494 mg (2.73 mmol) de C-(5-méthyl-furan-2-yl)-C-(3-méthyl-oxetan-3-yl)-méthylamine dans 3 mL de méthanol. Au bout de 3 Jours, 248 mg (1.37 mmol) de C-(5-méthyl-furan-2-yl)-C-(3-méthyl-oxetan-3-yl)-méthylamine ont été rajoutés. Au bout de 4 Jours, le milieu réactionnel a été concentré à sec. Le milieu réactionnel a été repris dans de l'acétate d'éthyle et a été lavé avec une solution aqueuse saturée de chlorure d'ammonium puis avec une solution aqueuse à 1M de dihydrogénophosphate de sodium. La phase organique a été séchée sur sulfate de magnésium, filtrée et concentrée. Le solide obtenu a été repris sous agitation dans de l'éther diéthylique et un peu d'acétate d'éthyle et a été filtrée. 400 mg d'un solide beige ont été obtenus. Ce solide a été chromatographié sur gel de silice (acétate d'éthyle/acétone/eau : 50/50/2 puis 50/50/5). 240 mg ont été obtenus sous forme d'un solide beige. Rendement = 35%, HPLC = 52.43% + 45.15% (mélange des deux diastéréoisomères), Tf = 198-205 °C.

RMN ¹H (DMSO-d6, 400 MHz): = 1.32 (s, 3H); 1.75-1.95 (m, 3H); 2.18-2.26 (m, 4H); 3.40-3.51 (m, 2H); 4.27-4.44 (m, 1H); 4.58 (d, J=6.2 Hz, 1H); 4.65 (d, J=6.1 Hz, 1H); 5.66 (d, J=9.7 Hz, 1H); 6.05-6.06 (m, 1H); 6.25 (d, J=3.1 Hz, 1H); 6.80-6.88 (m, 1H); 7.01 (d, J=7.4 Hz, 1H); 7.80-7.85 (m, 1H); 8.92 (dl, J=9.4 Hz, 1H); 9.57 (s, 1H).

### EXEMPLE 11

### Préparation du 3-[2-hydroxy-3-(1-hydroxy-ethyl)-phenylamino]-4-{[(5-méthyl-furan-2-yl)-(3-méthyl-oxetan-3-yl)-méthyl]-amino}-cyclobut-3-ène-1,2-dione

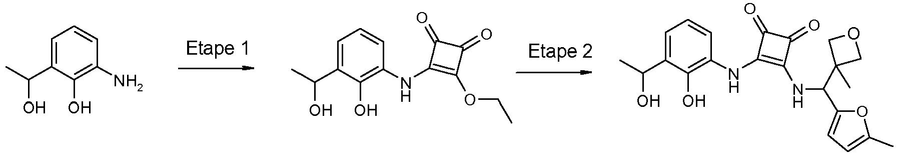

### Etape 1:

### 3-Ethoxy-4-[2-hydroxy-3-(1-hydroxy-ethyl)-phenylamino]-cyclobut-3-ène-1,2-dione

Un mélange de 1.16 g (7.6 mmol, 1 éq) de 2-amino-6-(1-hydroxy-éthyl)-phénol et de 5.15 g (30.3 mmol, 4 éq) de 3,4-diéthoxy-3-cyclobutène-1,2-dione dans 50 mL d'éthanol a été chauffé à 60°C pendant 18 heures. Le milieu réactionnel a été évaporé et le résidu a été chromatographié sur gel de silice (colonne puriFlash IR-50SI/300G, Spot II) élué à l'heptane/acétate d'éthyle (gradient). 800 mg de 3-éthoxy-4-[2-hydroxy-3-(1-hydroxy-ethyl)-phenylamino]-cyclobut-3-ène-1,2-dione ont été obtenus sous forme d'un solide orange. Rendement = 38%.

### Etape2:

### 3-[2-Hydroxy-3-(1-hydroxy-ethyl)-phénylamino]-4-{[(5-méthyl-furan-2-yl)-(3-méthyl-oxetan-3-yl)-méthyl]-amino}-cyclobut-3-ène-1,2-dione

Un mélange de 314 mg (1.73 mmol, 1.2 éq) de C-(5-méthyl-furan-2-yl)-C-(3-méthyl-oxetan-3-yl)-méthylamine et de 400 g (1.44 mmol, 1 éq) de 3-éthoxy-4-[2-hydroxy-3-(1-hydroxy-ethyl)-phenylamino]-cyclobut-3-ène-1,2-dione dans 15 mL de méthanol a été chauffé à 50°C pendant 2 Jours. Le milieu réactionnel a été évaporé et le résidu a été chromatographié sur gel de silice HP (colonne Redi*Sep R*f Gold 40g, Spot II) élué dichlorométhane/méthanol (gradient). Le solide amorphe a été repris avec un peu d'éther diéthylique, filtré et séché sous vide à 50°C. 151 mg de 3-[2-hydroxy-3-(1-hydroxy-ethyl)-phenylamino]-4-{[(5-méthyl-furan-2-yl)-(3-méthyl-oxetan-3-yl)-méthyl]-amino}-cyclobut-3-ène-1,2-dione ont été obtenus sous forme d'un solide orange. (Tf= 129-131°C). Rendement = 25%. LC/MS: 96.1% [412] : 2 diastéréoisomères.

RMN ¹H (DMSO-d6, 400 MHz) : 1.32 (s, 3H) ; 1.35 (d, *J*=6.4 Hz, 3H) ; 2.26 (s, 3H) ; 4.29 (dd, *J*=6.1 Hz, 2H) ; 4.6 (dd, *J*=29.9 Hz, 2H) ; 5.06 (q, *J*=6.3 Hz, 1H) ; 5.64 (d, *J*=9.7 Hz, 1H) ; 6.06 (d, *J*=2.0 Hz, 1H) ; 6.25 (d, *J*=3.0 Hz, 1H) ; 6.82 (t, *J*=7.9 Hz, 1H) ; 6.95 (d, *J*=7.6 Hz, 1H) ; 7.58 (d, *J*=7.8 Hz, 1H) ; 8.76 (d, *J*=9.8 Hz, 1H) ; 9.39 (s, 2H).

### EXEMPLE 12

### Préparation du 3-(2-hydroxy-3-isobutyryl-phenylamino)-4-{[(5-méthyl-furan-2-yl)-(3-méthyl-oxetan-3-yl)-méthyl]-amino}-cyclobut-3-ène-1,2-dione

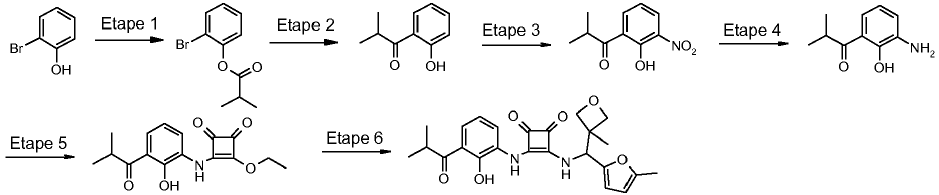

### Etape 1:

### Isobutyrate de 2-bromo-phényle

A une suspension de 6.40 g (160 mmol) d'hydrure de sodium (60 % dans l'huile) dans 150 mL de tétrahydrofuranne à 0°C sous azote ont été rajoutés goutte à goutte 25 g (145 mmol) de 2-bromophénol dans 100 mL de tétrahydrofuranne. 15 minutes plus tard, 26.5 mL (160 mmol) ont été rajoutés goutte à goutte, le milieu réactionnel prend en masse, 100 mL de tétrahydrofuranne ont été alors rajoutés. Une heure plus tard, le milieu réactionnel a été hydrolysé avec de l'eau et une solution aqueuse saturée d'hydrogénocarbonate de sodium a été rajouté ainsi que de l'acétate d'éthyle (présence d'un précipité dans la phase aqueuse, sûrement des sels). La phase organique a été ensuite lavée avec de l'eau, séchée sur sulfate de magnésium anhydre, filtrée et concentrée. 34.75 g d'une huile incolore ont été obtenus et filtrés sur silice (heptane 100 % puis environ 90/10 heptane/acétate d'éthyle). 31.59 g ont été obtenus sous forme d'une huile incolore. Rendement = 90% (présence d'anhydride d'isobutyrique en RMN ¹H).

### Etape 2:

### 1-(2-Hydroxy-phenyl)-2-méthyl-propan-1-one

A une solution de 31.59 g (130 mmol) d'isobutyrate de 2-bromo-phényle dans 300 mL de tétrahydrofuranne à -85 °C sous azote ont été rajoutés goutte à goutte 100 mL de *sec-*butyllithium à 1.4 M dans le cyclohexane. Le milieu réactionnel a été maintenu 30 minutes à environ -75°C et a été ensuite laissé revenir en douceur à température ambiante. Après 2 heures, un peu d'acétate d'éthyle a été rajouté suivi d'environ 400 mL d'une solution aqueuse de dihydrogénocarbonate de sodium 1M. La phase organique a été recueillie, lavée avec de l'eau, séchée sur sulfate de magnésium anhydre, filtrée et concentrée. 23.97 g d'une huile orange ont été obtenus et purifiés sur gâteau de silice. Le résidu obtenu a été chromatographié sur colonne de silice prépackée (éluant heptane/acétate d'éthyle). 4.3 g de produit ont été obtenus sous forme d'une huile jaune. Rendement = 20%.

### Etape 3:

### 1-(2-Hydroxy-3-nitro-phenyl)-2-méthyl-propan-1-one

A une solution de 4.1 g de 1-(2-hydroxy-phenyl)-2-méthyl-propan-1-one dans 25 mL d'acide acétique refroidie à 5°C ont été ajoutés 1.6 mLd'acide nitrique fumant en 20 minutes. Le milieu réactionnel a été agité à température ambiante pendant 5 heures (suivi par CCM heptane/acetate d'éthyle 10/1). La réaction a été arrêtée avant que la totalité de 1-(2-hydroxy-phenyl)-2-méthyl-propan-1-one soit consommée. Le milieu réactionnel a été dilué avec 70 mL d'eau puis extrait à l'acétate d'éthyle (4:1, 3x50 mL). Les phases organiques ont été rassemblées, lavées à l'eau (20 mL) puis avec une solution saturée de chlorure de sodium (25 mL) et séchées sur sulfate de magnésium. Le solvent a été évaporé et le résidu a été chromatographié sur silice. 1.40 g de 1-(2-hydroxy-5-nitro-phenyl)-2-méthyl-propan-1-one and 1.2 g of 1-(2-hydroxy-3-nitro-phenyl)-2-méthyl-propan-1-one ont été obtenus.

### Etape 4:

### 1-(3-Amino-2-hydroxy-phenyl)-2-méthyl-propan-1-one

Une solution de 1.04 g de 1-(2-hydroxy-5-nitro-phenyl)-2-méthyl-propan-1-one dans 20 mL d'éthanol et en présence de 200 mg (20% en masse) de palladium sur charbon activé à 10% a été agité sous pression atmosphérique d'hydrogène pendant la nuit. A la fin de la réaction, le dérivé alcool IV a également été formé. Le solvant a été évaporé et le résidu a été purifié par chromatographie sur gel de silice élué à l'heptane/acétate d'éthyle (10/1). 390 mg de 1-(3-amino-2-hydroxy-phényl)-2-méthyl-propan-1-one ont été obtenus sous forme d'une huile jaune (Rendement = 44%). 220 mg de 2-amino-6-(1-hydroxy-2-méthyl-propyl)-phénol ont été obtenus sous forme d'un solide beige (Rendement = 25%).

### Etape 5:

### 3-Ethoxy-4-(2-hydroxy-3-isobutyryl-phénylamino)-cyclobut-3-ène-1,2-dione

Une solution de 0.39 g (2.18 mmol, 1 éq) de 1-(3-amino-2-hydroxy-phenyl)-2-méthyl-propan-1-one et de 1.51 g (8.87 mmol, 4 éq) de 3,4-diéthoxy-cyclobut-3-ène-1,2-dione dans 4 mL d'éthanol a été chauffée à 60°C pendant 24 heures. Le milieu réactionnel a été concentré. Le résidu (1.80 g) a été chromatographiée sur gel de silice (colonne prépackée de 120 g, éluant heptane/acétate d'éthyle de 10% à 50 % en acétate d'éthyle). 0.46 g de produit ont été obtenus sous forme d'une huile jaune. Rendement = 70%.

### Etape 6:

### 3-Ethoxy-4-(2-hydroxy-3-isobutyryl-phenylamino)-cyclobut-3-ène-1,2-dione

A une solution de 0.46 g (1.52 mmol, 1 éq) de 3-éthoxy-4-(2-hydroxy-3-isobutyryl-phenylamino)-cyclobut-3-ène-1,2-dione dans 10 mL de méthanol à 50°C ont été rajoutés 551 mg (3.04 mmol, 2 éq) de C-(5-méthyl-furan-2-yl)-C-(3-méthyl-oxetan-3-yl)-méthylamine en solution dans 3 mL de méthanol. Au bout de 15 heures, un précipité a été formé et a été filtré. 0.49 g de produit a été obtenu sous forme d'un solide jaune. Rendement = 73%, pureté HPLC = 96.75%, Tf = 216-217°C.

RMN ¹H (DMSO-d6, 400 MHz) : = 1.17 (d, J=6.8 Hz, 6H); 2.26 (s, 3H); 3.77 (sext, J=6.8 Hz, 1H); 4.28-4.31 (m, 2H); 4.57 (d, J=6.2 Hz, 1H); 4.65 (d, J=6.2 Hz, 1H); 5.64 (d, J=9.8 Hz, 1H); 6.06-6.07 (m, 1H); 6.26 (d, J=3.1 Hz, 1H); 7.00 (t, J=8.0 Hz, 1H); 7.72 (dd, J =8.3 Hz, J=1.0 Hz, 1H); 8.06 (dd, J=7.9 Hz, J=0.9 Hz, 1H); 8.86 (d, J=9.8 Hz, 1H); 9.57 (s, 1H); 13.00-13.20 (m, 1H).

### EXEMPLE 13

### Préparation du 3-(4-hydroxy-2-méthyl-3-oxo-2,3-dihydro-1H-isoindol-5-ylamino)-4-{[(5-méthyl-furan-2-yl)-(3-méthyl-oxetan-3-yl)-méthyl]-amino}-cyclobut-3-ène-1,2-dione

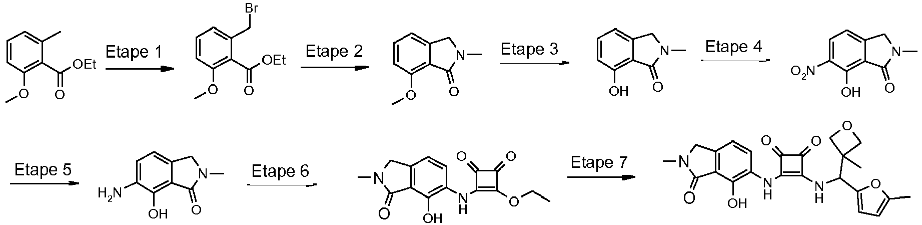

### Etape 1:

### 2-Bromométhyl-6-méthoxy-benzoate d'éthyle

A une solution de 19.40 g de 2-méthoxy-6-méthyl-benzoate d'éthyle dans 150 mL de tétrachlorure de carbone ont été additionnés 19.56 g de N-bromosuccinimide et 4.84 g de peroxide de benzoyle. Le milieu réactionnel a été chauffé à reflux pendant 6 heures (plus d'évolution). Le milieu réactionnel a été refroidi à 5°C et le succinimide a été filtré. La phase organique a été lavée avec une solution saturée d'hydrogénocarbonate de sodium (2x150 mL), séchée sur sulphate de magnésium et évaporée. Le résidu a été purifié par chromatographie sur gel de silice (colonne puriFlash, IR-50SI/800G, Spot II) élué à l'heptane/acétate d'éthyle (gradient). 15.5 g de 2-bromométhyl-6-méthoxy-benzoate d'éthyle ont été obtenus sous forme d'une huile jaune qui cristallise plus tard. Rendement = 56%

### Etape 2:

### 7-Méthoxy-2-méthyl-2,3-dihydro-isoindol-1-one

A une solution de 14.82 g de 2-bromométhyl-6-méthoxy-benzoate d'éthyle dans le méthanol a été ajoutée goutte à goutte une solution de méthylamine (2M dans méthanol) à 0°C et le milieu réactionnel a été agité 5 heures à 0°C laissé agité à température ambiante pendant 2 Jours. Le solvent a été évaporé et 30 mL d'une solution saturée d'hydrogénocarbonate de sodium ont été ajoutés sur le résidu. Le produit a été extrait à l'acétate d'éthyle (3x50 mL). Les phases organiques ont été rassemblées, séchées sur sulfate de magnesium et évaporées. Le résidu a été purifié par chromatographie sur gel de silice. 4.52 g de 7-méthoxy-2-méthyl-2,3-dihydro-isoindol-1-one ont été obtenus. Rendement = 47%.

### Etape 3:

### 7-hydroxy-2-méthyl-2,3-dihydro-isoindol-1-one

A une solution de 3.7 g de 7-méthoxy-2-méthyl-2,3-dihydro-isoindol-1-one dans 50 mL de dichlorométhane ont été ajoutés goutte à goutte 100 mL d'une solution de tribromure de bore 1M dans dichlorométhane à -78°C. Le milieu réactionnel a été agité à -78°C pendant 1 heure puis laissé remonter à 0°C pendant 1 heure. Le milieu réactionnel a été refroidi à -78°C puis hydrolyse lentement par addition goutte à goutte de 20 mL de méthanol (température inférieure à -60°C). Le milieu réactionnel a été lavé avec 30 mL d'une solution de dihydrogénophosphate de sodium 1M et la phase aqueuse a été extraite avec l'acétate d'éthyle (2x50 mL). Les phases organiques ont été rassemblées, séchées sur sulfate de magnésium et évaporées. Le résidu a été trituré avec de l'éther diéthylique. 2.6 g de 7-hydroxy-2-méthyl-2,3-dihydro-isoindol-1-one ont été obtenus.

### Etape 4:

### 7-Hydroxy-2-méthyl-6-nitro-2,3-dihydro-isoindol-1-one

2.49 g (91.5 mmol) de 7-hydroxy-2-méthyl-2,3-dihydro-isoindol-1-one ont été mis en solution à 0°C dans 6 mL d'acide sulfurique concentré. Le milieu réactionnel a été refroidi à - 20°C et 1.8 mL d'un mélange 4/5 d'acide sulfurique concentré / acide nitrique à 70% ont été rajoutés. Au bout de 10 minutes, le milieu réactionnel a été hydrolysé à l'eau. Du chlorure de sodium a été rajouté et le milieu a été extrait deux fois à l'acétate d'éthyle. Les phases organiques ont été rassemblées, séchées sur sulfate de sodium anhydre et concentrées. Le résidu a été chromatographiés sur colonne C18 en 2 fois (colonne prépackée de 40 g, gradient eau/acétonitrile). 350 mg de produit ont été obtenus sous forme d'un solide jaune. Rendement = 11%.

### Etape 5:

### 6-Amino-7-hydroxy-2-méthyl-2,3-diliydro-isoindol-1-one

A une solution de 0.35 g (1.68 mmol) de 7-hydroxy-2-méthyl-6-nitro-2,3-dihydro-isoindol-1-one dans 20 mL de méthanol sous azote, ont été rajoutés 38 mg de palladium sur charbon à 10%. Le milieu réactionnel a été agité sous atmosphère d'hydrogène pendant 18 heures. Le milieu réactionnel a été filtré et concentré. Le résidu a été chromatographiée sur colonne C18 prépackée (colonne de 12 g, 30 mL/min, eau/acétonitrile 95/5). 0.19 g de produit ont été obtenus sous forme d'une huile marron. Rendement = 64%.

### Etape 6:

### 3-Ethoxy-4-(4-hydroxy-2-méthyl-3-oxo-2,3-dihydro-1H-isoindol-5-ylamino)-cyclobut-3-ène-1,2-dione

Un mélange de 0.19 g (1.07 mmol) de 6-amino-7-hydroxy-2-méthyl-2,3-dihydro-isoindol-1-one et de 0.80 g (4.70 mmol) de 3,4-diéthoxy-cyclobut-3-ène-1,2-dione dans 10 mL d'éthanol a été chauffé à 60 °C pendant 24 heures. Le milieu réactionnel a été concentré et a été repris avec de l'éther diéthylique sous agitation (formation d'un précipité). Le solide obtenu a été filtré, lavé deux fois à l'éther diéthylique et séché. 235 mg de produit ont été obtenus. Rendement = 73%.

### Etape 7:

### 3-(4-hydroxy-2-méthyl-3-oxo-2,3-dihydro-1H-isoindol-5-ylamino)-4-{[(5-méthyl-furan-2-yl)-(3-méthyl-oxetan-3-yl)-méthyl]-amino}-cyclobut-3-ène-1,2-dione

A une suspension de 235 mg (0.78 mmol) de 3-(2-éthoxy-3,4-dioxo-cyclobut-1-enylamino)-2-hydroxy-N,N-diméthyl-benzamide dans 20 mL de méthanol à 55°C ont été rajoutés 287 mg (1.58 mmol) de C-(5-méthyl-furan-2-yl)-C-(3-méthyl-oxetan-3-yl)-méthylamine dans 3 mL de méthanol. Le milieu réactionnel a été chauffé à 55°C pendant 24 heures puis concentré à sec. Le résidu a été chromatographié sur gel de silice (colonne prépackée de 40 g, 30 mL/min, 100% dichlorométhane puis 4% de méthanol). Un résidu de 130 mg a été obtenu et a été purifié par CCM préparative (éluant : acétate d'éthyle/acétone/eau 50/50/1). 52 mg ont été obtenus puis précipités dans le diméthylsulfoxide et filtrés. 15 mg de produit ont été obtenus sous forme d'un solide blanc. Rendement = 4%, HPLC = 98.56%

RMN ¹H (DMSO-d6, 400 MHz) : 1.32 (s, 3H); 2.26 (s, 3H); 3.04 (s, 3H); 4.28-4.31 (m, 2H); 4.40 (s, 2H); 4.58 (d, J=6.2 Hz, 1H); 4.64 (d, J=6.2 Hz, 1H); 5.64 (d, J=9.8 Hz, 1H); 6.06-6.07 (m, 1H); 6.26 (d, J=3.0 Hz, 1H); 7.01 (d, J=8.1 Hz, 1H); 7.88 (d, J=8.1 Hz, 1H); 8.84 (d, J=9.8 Hz, 1H); 9.50 (s, 1H); 9.76 (s, 1H).

### EXEMPLE 14

### Préparation du 3-[2-hydroxy-3-(pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione

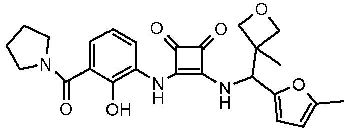

### Etape 1:

De manière analogue à l'EXEMPLE 1 (étape 4), (2-hydroxy-3-nitro-phenyl)-pyrrolidin-1-yl-méthanone a été préparée.

### Etape 2:

De manière analogue à l'EXEMPLE 1 (étape 5), (3-amino-2-hydroxy-phenyl)-pyrrolidin-1-yl-méthanone a été préparée.

### Etape 3:

Une solution de 0.99 g (4.8 mmol) de (3-amino-2-hydroxy-phenyl)-pyrrolidin-1-yl-méthanone et de 2.73 g (19.2 mmol) de 3,4-diméthoxy-cyclobut-3-ène-1,2-dione dans 100 mL de méthanol a été chauffée à 50°C pendant 5 heures et demie. Le milieu réactionnel a été concentré. Le résidu a été repris avec de l'acétate d'éthyle et a été lavé deux fois avec une solution aqueuse de dihydrogénophosphate de sodium à 1M. La phase organique a été séchée sur sulfate de sodium anhydre, filtrée et concentrée. Le résidu a été chromatographié sur gel de silice (colonne prépackée de 300 g, éluant heptane/acétate d'éthyle de 60% à 100% d'acétate d'éthyle). 0.85 g de produit ont été obtenus sous forme d'un solide jaune pâle. Rendement = 56%.

### Etape 4:

### 3-[2-Hydroxy-3-(pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione

A une solution de 443 mg (1.40 mmol) de 3-éthoxy-4-[2-hydroxy-3-(pyrrolidine-1-carbonyl)-phenylamino]-cyclobut-3-ène-1,2-dione dans 42 mL de méthanol ont été rajoutés 385 mg (2.12 mmol) de C-(5-méthyl-furan-2-yl)-C-(3-méthyl-oxetan-3-yl)-méthylamine en solution dans 3 mL de méthanol. Le milieu réactionnel a été chauffé à 50°C pendant 13 heures. Le milieu réactionnel a été concentré. Le résidu a été repris avec de l'acétate d'éthyle et a été lavée deux fois avec une solution aqueuse de dihydrogénophosphate de sodium à 1M. La phase organique a été séchée sur sulfate de sodium anhydre, filtrée et concentrée. 0.62 g de produit ont été obtenus sous forme d'un solide beige. Rendement = 95%, HPLC = 94.06%, Tf = 210°C.

RMN ¹H (DMSO-d6, 400 MHz) : 1.33 (s, 3H); 1.86 (m, 4H); 2.26 (s, 3H); 3.53 (m, 4H); 4.28-4.31 (m, 2H); 4.58 (d, J=6.2 Hz, 1H); 4.66 (d, J=6.2 Hz, 1H); 5.66 (d, J=9.8 Hz, 1H); 6.06-6.07 (m, 1H); 6.26 (d, J=3.1 Hz, 1H); 6.89 (t, J=8.0 Hz, 1H); 7.16 (dd, J=7.9 Hz, J=1.4 Hz, 1H); 7.86 (dd, J=8.0 Hz, J=1.2 Hz, 1H); 8.86 (d, J=9.8 Hz, 1H); 9.50 (s, 1H); 11.56 (s, 1H).

### EXEMPLE 15

### Préparation du 3-[2-hydroxy-3-(morpholine-4-carbonyl)-phenylamino]-4-{[(5-méthyl-furan-2-yl)-(3-méthyl-oxetan-3-yl)-méthyl]-amino}-cyclobut-3-ène-1,2-dione

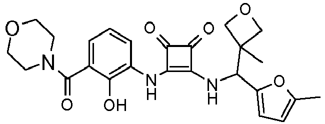

### Etape 1:

De manière analogue à l'EXEMPLE 5 (étape 1), (2-hydroxy-3-nitro-phenyl)-morpholin-4-yl-méthanone a été préparée.

### Etape 2:

De manière analogue à l'EXEMPLE 5 (étape 2), (3-amino-2-hydroxy-phenyl)-morpholin-4-yl-méthanone a été préparée.

### Etape 3:

### 3-[2-Hydroxy-3-(morpholine-4-carbonyl)-phenylamino]-4-méthoxy-cyclobut-3-ène-1,2-dione

Une solution de 1.00 g (4.5 mmol) de (3-amino-2-hydroxy-phenyl)-morpholin-4-yl-méthanone et de 2.56 g (18.0 mmol) de 3,4-diméthoxy-cyclobut-3-ène-1,2-dione dans 100 mL de méthanol a été chauffée à 50°C pendant 5 heures et demie. Le milieu réactionnel a été concentré. Le résidu a été repris dans de l'acétate d'éthyle et a été lavé deux fois avec une solution aqueuse de dihydrogénophosphate de sodium à 1M. La phase organique a été séchée sur sulfate de sodium anhydre, filtrée et concentrée. Le solvant a été évaporé et le résidu a été chromatographié sur gel de silice (colonne prépackée de 200 g, éluant dichlorométhane/méthanol de 0 à 5% en méthanol). 0.81 g d'un solide jaune a été obtenu (fraction sale). Ce solide a été repris dans du méthanol sous agitation, filtré et rincé au méthanol. 0.50 g de produit ont été obtenus sous forme d'un solide jaune pâle. Rendement = 33%.

### Etape 4:

### 3-[2-hydroxy-3-(morpholine-4-carbonyl)-phenylamino]-4-{[(5-méthyl-furan-2-yl)-(3-méthyl-oxetan-3-yl)-méthyl]-amino}-cyclobut-3-ène-1,2-dione

A une suspension de 249 mg (0.75 mmol) de 3-[2-hydroxy-3-(morpholine-4-carbonyl)-phenylamino]-4-méthoxy-cyclobut-3-ène-1,2-dione dans 22 mL de méthanol ont été rajoutés 205 mg (1.13 mmol) de C-(5-méthyl-furan-2-yl)-C-(3-méthyl-oxetan-3-yl)-méthylamine en solution dans 3 mL de méthanol. Le milieu réactionnel a été ensuite chauffé à 50°C pendant deux heures et agité à température ambiante pendant 3 Jours. Le milieu réactionnel a été concentré. Le résidu a été repris avec de l'acétate d'éthyle et a été lavée deux fois avec une solution aqueuse de dihydrogénophosphate de sodium à 1M. La phase organique a été séchée sur sulfate de sodium anhydre, filtrée et concentrée. Le résidu a été chromatographié sur gel de silice (colonne prépackée de 25 g, éluant heptane/acétate d'éthyle, de 80% à 100% d'acétate d'éthyle). 0.23 g de produit ont été obtenus sous forme d'un solide jaune. Rendement = 64%, pureté HPLC = 98.01 %

RMN ¹H (DMSO-d6, 400 MHz) : 1.32 (s, 3H); 2.26 (s, 3H); 3.20-3.70 (m, 8H); 4.29 (dd, J=6.2 Hz, J=2.4 Hz, 2H); 4.58 (d, J=6.2 Hz, 1H); 4.65 (d, J=6.2 Hz, 1H); 5.65 (d, J=9.7 Hz, 1H); 6.06-6.07 (m, 1H); 6.26 (d, J=3.1 Hz, 1H); 6.82-6.93 (m, 2H); 7.79 (dd, J=7.7 Hz, J=1.7 Hz, 1H); 8.86 (d, J=9.8 Hz, 1H); 9.40-9.55 (m, 1H); 9.85-10.20 (m, 1H).

### EXEMPLE 16

### Préparation du 3-(4-hydroxy-pyrimidin-5-ylamino)-4-{[(5-méthyl-furan-2-yl)-(3-méthyl-oxetan-3-yl)-méthyl]-amino}-cyclobut-3-ène-1,2-dione

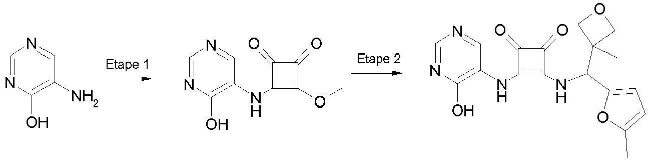

### Etape 1:

### 3-(4-Hydroxy-pyrimidin-5-ylamino)-4-méthoxy-cyclobut-3-ène-1,2-dione

A une suspension de 3.86 g (27.16 mmol, 4 éq) de 3,4-diméthoxy-cyclobut-3-ène-1,2-dione dans 20 mL de méthanol ont été rajoutés 1.00 g (6.78 mmol, 1 éq) de chlorhydrate de 5-amino-pyrimidin-4-ol et 0.95 mL (6.78 mmol, 1 éq) de triéthylamine. Le milieu réactionnel a été chauffé à 50°C pendant 18 heures. Le milieu réactionnel a été laissé à température ambiante. Le précipité a été filtré et lavé au méthanol. 1.19 g de produit ont été obtenus sous forme d'un solide jaune. Rendement = 79%.

### Etape 2:

### 3-(4-Hydroxy-pyrimidin-5-ylamino)-4-{[(5-méthyl-furan-2-yl)-(3-méthyl-oxetan-3-yl)-méthyl]-amino}-cyclobut-3-ène-1,2-dione

Un mélange de 300 mg (1.36 mmol, 1 éq) de 3-(4-hydroxy-pyrimidin-5-ylamino)-4-méthoxy-cyclobut-3-ène-1,2-dione et de 504 mg (2.78 mmol, 2 éq) de C-(5-méthyl-furan-2-yl)-C-(3-méthyl-oxetan-3-yl)-méthylamine dans 25 mL de méthanol a été chauffé à 50°C pendant 22 heures. Le milieu réactionnel a été filtré et l'insoluble a été lavé au méthanol et à l'éther diéthylique. 0.36 g de produit ont été obtenus sous forme d'un solide blanc. Rendement = 72%, pureté HPLC = 97.81%, Tf = 281°C (dégradation).

RMN ¹H (DMSO-d6, 400 MHz) : 1.30 (s, 3H); 2.25 (s, 3H); 4.27-4.29 (m, 2H); 4.57 (d, J=6.2 Hz, 1H); 4.62 (d, J=6.2 Hz, 1H); 5.60 (d, J=9.7 Hz, 1H); 6.05-6.06 (m, 1H); 6.25 (d, J=3.1 Hz, 1H); 7.99 (s, 1H); 8.52 (s, 1H); 8.98 (d, J=9.8 Hz, 1H); 9.58 (s, 1H); 12.88 (s, 1H).

### EXEMPLE 17

### Préparation du 3-{[(5-méthyl-furan-2-yl)-(3-méthyl-oxetan-3-yl)-méthyl]-amino}-4-(1-méthyl-2-oxo-1,2-dihydro-quinolin-3-ylamino)-cyclobut-3-ène-1,2-dione

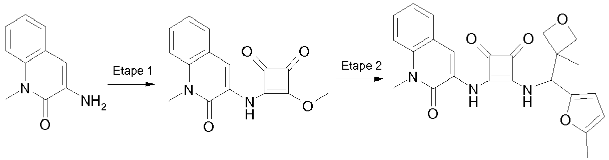

### Etape 1:

De manière analogue à l'EXEMPLE 1 (étape 6), 3-méthoxy-4-(1-méthyl-2-oxo-1,2-dihydro-quinolin-3-ylamino)-cyclobut-3-ène-1,2-dione a été préparée. Rendement = 67%.

### Etape 2:

### De manière analogue à l'EXEMPLE 1 (étape 7), (3-{[(5-méthyl-furan-2-yl)-(3-méthyl-oxetan-3-yl)-méthyl]-amino}-4-(1-méthyl-2-oxo-1,2-dihydro-quinolin-3-ylamino)-cyclobut-3-ène-1,2-dione a été préparée. Rendement = 31%, Tf= 155-161°C.

RMN ¹H (DMSO-d6, 400 MHz) : 1.33 (s, 3H); 2.27 (s, 3H); 3.76 (s, 3H); 4.30 (t, J=4.2-5.6 Hz, 2H); 4.58 (d, J=6.1 Hz, 1H); 4.65 (d, J=9.3 Hz, 1H); 5.65 (d, J=9.3 Hz, 1H); 6.07 (s, 1H); 7.27 (d, J=2.7 Hz, 1H); 7.30 (t, J=6.1-6.4 Hz, 1H); 7.45-7.65 (m, 3H); 8.38 (s, 1H); 9.16 (d, J=9.4 Hz, 1H); 9.88 (s, 1H).

### EXEMPLE 18

### Préparation du 3-{[(5-méthyl-furan-2-yl)-(3-méthyl-oxetan-3-yl)-méthyl]-amino}-4-(6-méthyl-2-oxo-2H-pyran-3-ylamino)-cyclobut-3-ène-1,2-dione

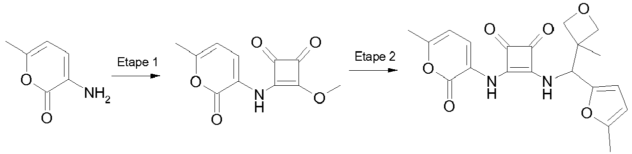

### Etape 1:

De manière analogue à l'EXEMPLE 1 (étape 6), 3-méthoxy-4-(6-méthyl-2-oxo-2H-pyran-3-ylamino)-cyclobut-3-ène-1,2-dione a été préparée.

### Etape 2:

De manière analogue à l'EXEMPLE 1 (étape 7), 3-{[(5-méthyl-furan-2-yl)-(3-méthyl-oxetan-3-yl)-méthyl]-amino}-4-(6-méthyl-2-oxo-2H-pyran-3-ylamino)-cyclobut-3-ène-1,2-dione a été préparée.

### EXEMPLE 19

### Préparation du 3-{[(5-méthyl-furan-2-yl)-(3-méthyl-oxetan-3-yl)-méthyl]-amino}-4-(1-méthyl-2-oxo-1,2-dihydro-pyridin-3-ylamino)-cyclobut-3-ène-1,2-dione

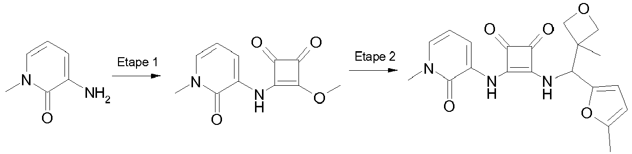

### Etape 1:

De manière analogue à l'EXEMPLE 1, (étape 6), 3-méthoxy-4-(1-méthyl-2-oxo-1,2-dihydro-pyridin-3-ylamino)-cyclobut-3-ène-1,2-dione a été préparée. Rendement = 50%.

### Etape 2:

De manière analogue à l'EXEMPLE 1 (étape 7), (3-{[(5-méthyl-furan-2-yl)-(3-méthyl-oxetan-3-yl)-méthyl]-amino}-4-(1-méthyl-2-oxo-1,2-dihydro-quinolin-3-ylamino)-cyclobut-3-ène-1,2-dione a été préparée. Rendement = 86%.

RMN ¹H (DMSO-d6, 400 MHz) : 1.31 (s, 3H); 2.26 (s, 3H); 3.53 (s, 3H); 4.27-4.29 (m, 2H); 4.57 (d, J=6.2 Hz, 1H); 4.63 (d, J=6.2 Hz, 1H); 5.62 (d, J=9.7 Hz, 1H); 6.05-6.06 (m, 1H); 6.24 (d, J=3.1 Hz, 1H); 6.29 (t, J=7.1 Hz, 1H); 7.41 (dd, J=6.7 Hz, J=1.6 Hz, 1H); 8.00 (dd, J=7.4 Hz, J=1.5 Hz, 1H); 9.08 (dl, J=9.8 Hz, 1H); 9.70 (s, 1H).

### EXEMPLE 20

### Préparation du 3-(2-hydroxy-pyridin-3-ylamino)-4-{[(5-méthyl-furan-2-yl)-(3-méthyl-oxetan-3-yl)-méthyl]-amino}-cyclobut-3-ène-1,2-dione

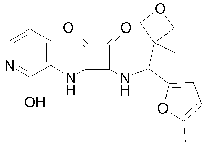

De manière analogue à l'EXEMPLE 1 (étapes 6 et 7), 3-(2-hydroxy-pyridin-3-ylamino)-4-{[(5-méthyl-furan-2-yl)-(3-méthyl-oxetan-3-yl)-méthyl]-amino}-cyclobut-3-ène-1,2-dione a été préparée à partir du 3-amino-pyridine-2-ol. HPLC 94.84%, ES- [369].

RMN ¹H (DMSO-d6, 400 MHz) : 1.31 (s, 3H) ; 2.25 (s, 3H) ; 4.27 (d, J=6.2 Hz, 2H) ; 4.57-4.65 (m, 2H) ; 5.63 (d, J=9.8 Hz, 1H) ; 6.02 (s, 1H) ; 6.20-6.25 (m, 2H) ; 7.05-7.06 (m, 2H) ; 8.03-8.05 (m, 1H) ; 9.05 (d, J=9.8 Hz, 1H) ; 9.64 (s, 1H)

### EXEMPLE 21

### Préparation du 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzènesulfonamide

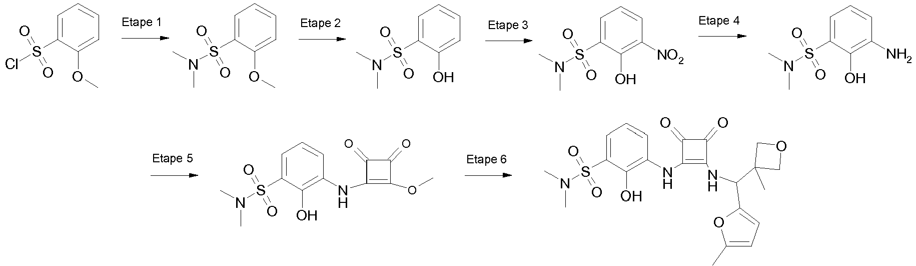

### Etape 1:

### 2-Méthoxy-N,N-diméthyl-benzènesulfonamide

A une solution de 9.90 g (47.9 mmol, 1 éq) de chlorure de 2-méthoxy-benzènesulfonyle dans 300 mL de tétrahydrofuranne à 0°C sous azote ont été rajoutés goutte à goutte 72 mL (144 mmol, 3 éq) de solution de diméthylamine 2M dans le tétrahydrofuranne. Après 30 minutes, le milieu réactionnel a été hydrolysé avec de l'eau et de l'acétate d'éthyle a été rajouté. La phase organique a été à nouveau lavée à l'eau puis séchée sur sulfate de sodium anhydre, filtrée et concentrée. 8.78 g de produit ont été obtenus sous forme d'une huile marron. Rendement = 85%.

### Etape 2:

### 2-Hydroxy-N,N-diméthyl-benzènesulfonamide

A une solution de 8.78 g (40.8 mmol, 1 éq) de 2-méthoxy-N,N-diméthyl-benzènesulfonamide dans 250 mL de dichlorométhane à -70 °C sous azote ont été rajoutés goutte à goutte 200 mL (200 mmol, 4.9 éq) d'une solution de tribromoboranne 1M dans le dichlorométhane. Le milieu réactionnel a été laissé une heure à - 70°C puis a été laissé revenir Jusqu'à 10 °C pendant 1 heure et demie. Le milieu réactionnel a été ensuite refroidi à -70°C et environ 100 mL de méthanol ont été rajoutés goutte à goutte (environ une heure d'addition). Le milieu réactionnel a été ensuite laissé revenir à témpérature ambiante et a été concentré. Le résidu obtenu a été filtré sur silice avec l'éluant heptane/acétate d'éthyle 6/4. 7.81 g de produit ont été obtenus sous forme d'un solide beige. Rendement = 95%.

### Etape 3:

### 2-Hydroxy-N,N-diméthyl-3-nitro-benzènesulfonamide

A un mélange de 7.17 g (35.6 mmol, 1 éq) de 2-hydroxy-N,N-diméthyl-benzènesulfonamide dans 35.5 mL d'acide acétique dans un bain à 5°C ont été rajoutés en 15 minutes 2.24 mL (47.4 mmol, 1.3 éq) d'acide nitrique fumant à 90%. Le milieu réactionnel a été ensuite agité à température ambiante. Après 1 heure et demie, le milieu réactionnel a été hydrolysé avec de l'eau et de la saumure. Le milieu réactionnel a été ensuite extrait avec un mélange d'acétate d'éthyle/heptane 4/1 (2 x 100 mL). Les phases organiques ont été rassemblées, séchées sur sulfate de sodium anhydre, filtrées et concentrées. Le solide obtenu (8.66 g) a été réuni avec GUL1537-178 et a été chromatographié sur gel de silice (colonne prépackée, éluant heptane/(acétate d'éthyle + 1% méthanol), de 20% à 65% en (acétate d'éthyle + 1% méthanol)). Une fraction enrichie en isomère ortho a été isolé soit 3.7 g d'un solide jaune. Ce solide a été recristallisé dans 40 mL d'éthanol. 1.93 g de produit ont été obtenus sous forme d'un solide jaune pâle. Rendement = 20%.

### Etape 4:

### 3-Amino-2-hydroxy-N,N-diméthyl-benzènesulfonamide

A une solution de 1.01 g (4.10 mmol) de 2-hydroxy-N,N-diméthyl-3-nitro-benzènesulfonamide dans 20 mL de méthanol et 20 mL de tétrahydrofuranne ont été rajouté 104 mg (10% en masse) de palladium sur charbon à 10%. Le milieu réactionnel a été agité sous atmosphère d'hydrogène pendant 17 heures. Le milieu réactionnel a été filtré sur célite et a été concentré. 0.94 g de produit ont été obtenus sous forme d'un solide marron. Rendement quantitatif.

### Etape 5:

### 2-Hydroxy-3-(2-méthoxy-3,4-dioxo-cyclobut-1-enylamino)-N,N-diméthyl-benzènesulfonamide

Un mélange de 0.94 g (4.10 mmol) de 3-amino-2-hydroxy-N,N-diméthyl-benzènesulfonamide et de 2.47 g (17.40 mmol) de 3,4-diméthoxy-cyclobut-3-ène-1,2-dione dans 20 mL de méthanol a été agité à température ambiante pendant 4 Jours. Le milieu réactionnel a été concentré. Le résidu (3.29 g) a été chromatographié sur gel de silice (colonne prépackée de 200 g, 100 mL/min, éluant heptane/acétate d'éthyle, de 50% à 85% en acétate d'éthyle). 0.86 g de produit ont été obtenus sous forme d'un solide jaune. Rendement = 64%.

### Etape 6:

### 2-Hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzènesulfonamide

Un mélange de 284 mg (0.87 mmol) de 2-hydroxy-3-(2-méthoxy-3,4-dioxo-cyclobut-1-enylamino)-N,N-diméthyl-benzènesulfonamide et de 318 mg (1.75 mmol) de C-(5-méthyl-furan-2-yl)-C-(3-méthyl-oxetan-3-yl)-méthylamine dans 20 mL de méthanol a été chauffé à 50°C pendant 3 Jours. Le milieu réactionnel a été concentré. Le résidu a été repris dans de l'acétate d'éthyle et a été lavé deux fois avec une solution aqueuse à 1M de dihydrogénophophate de sodium. La phase organique a été séchée sur sulfate de sodium anhydre, filtrée et concentrée. 0.29 g de produit ont été obtenus sous forme d'un solide ocre. Rendement =70%, HPLC = 98.68 %, Tf = 123°C.

RMN ¹H (DMSO-d6, 400 MHz) : 1.32 (s, 3H); 2.26 (s, 3H); 2.73 (s, 6H); 4.28-4.30 (m, 2H); 4.58 (d, J=6.2 Hz, 1H); 4.64 (d, J=6.2 Hz, 1H); 5.62 (d, J=9.8 Hz, 1H); 6.06-6.07 (m, 1H); 6.27 (d, J=3.1 Hz, 1H); 7.08 (t, J=8.0 Hz, 1H); 7.32 (dd, J=8.0 Hz, J=1.3 Hz, 1H); 7.91 (d, J=8.0 Hz, 1H); 8.80 (d, J=9.8 Hz, 1H); 9.54 (s, 1H); 9.66 (s, H).

### EXEMPLE 22

### Préparation du 2-Hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzènesulfonamide

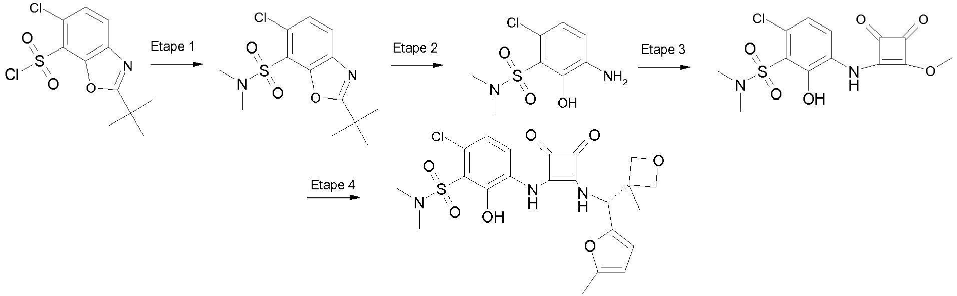

### Etape 1:

### 2-tert-Butyl-6-chloro-benzooxazole-7-sulfonic acid diméthylamide

A une solution de 3.0 g de 2-tert-butyl-6-chloro-benzooxazole-7-sulfonyl chloride (9.73 mmol; 1.0 éq) dans 90 mL de tétrahydrofurane sous azote à 0°C ont été rajoutés 4.05 mL de triéthylamine (29.20 mmol; 3.0 éq) et 29.2 mL de diméthylamine 2M (58.41 mmol; 6.0 éq) dans le tétrahydrofuranne. Après 2 heures et demie, le mileu réactionnel a été hydrolysé avec de l'eau et extrait deux fois à l'acétate d'éthyle. Les phases organiques ont été rassemblées, séchées sur sulfate de sodium anhydre, filtrées et concentrées.

2.96 g d'acide 2-tert-butyl-6-chloro-benzooxazole-7-sulfonique diméthylamide ont été obtenus. Rendement = 95.98%.

### Etape 2:

### 3-Amino-6-chloro-2-hydroxy-N,N-diméthyl-benzènesulfonamide

A une solution de 2.93 g d'acide 2-tert-butyl-6-chloro-benzooxazole-7-sulfonique diméthylamide (9.25 mmol; 1.0 eq) dans 13 mL de 1,4-dioxane ont été rajoutés 3.4 mL d'acide sulfurique (63.59 mmol; 1.16 V) et 3.4 mL d'eau. Le milieu réactionnel a été chauffé à reflux pendant 4 heures et demie. Le milieu réactionnel a été concentré. 130 mL de soude 1M ont été rajoutés dans le résidu (pH = 10) suivis de 400 mL d'eau. La solution a été extraite à l'acétate d'éthyle (2 x 250 mL). Les phases organiques ont été rassemblées, séchées sur sulfate de sodium anhydre, filtrées et concentrées. Le résidu a été chromatographié sur gel de silice (colonne prépackée de 250 g, éluant heptane/acétate d'éthyle, de 20% à 40% en acétate d'éthyle, débit 100 mL/min). 1.66 g de 3-amino-6-chloro-2-hydroxy-N,N-diméthyl-benzènesulfonamide ont été obtenus. Rendement = 72%).

### Etape 3:

### 6-Chloro-2-hydroxy-3-(2-méthoxy-3,4-dioxo-cyclobut-1-enylamino)-N,N-dimethbenzènesulfonamide

A une solution de 3,4-cyclobutane-1,2-dione (1.80 g; 12.68 mmol; 2.0 eq) dans 80 mL de méthanol a été rajouté du 3-amino-6-chloro-2-hydroxy-N,N-diméthyl-benzènesulfonamide (1.59 g; 6.34 mmol; 1.0 eq). Le milieu réactionnel a été chauffé à 50°C pendant 5 heures. Le précipité formé a été filtré. Le filtrat a été concentré et le résidu obtenu a été chromatographié sur gel de silice (colonne prépackée de 240 g, éluant heptane/acétate d'éthyle de 40% à 70% en acétate d'éthyle, 100 mL/min). 1.04 g de 6-chloro-2-hydroxy-3-(2-méthoxy-3,4-dioxo-cyclobut-1-enylamino)-N,N-diméthyl benzène-sulfonamide ont été obtenus. Rendement = 45%.

### Etape 4:

### 2-Hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzènesulfonamide

Un mélange de 6-chloro-2-hydroxy-3-(2-méthoxy-3,4-dioxo-cyclobut-1-enylamino)-N,N-diméthyl-benzènesulfonamide (0.25 g; 0.69 mmol; 1.0 eq) et de C-[(R)-C-(5-méthyl-furan-2-yl)-C-(3-méthyl-oxetan-3-yl)]-méthylamine (0.19 g; 1.04 mmol; 1.5 eq) dans 20 mL de méthanol a été chauffé à 50°C pendant 23 heures. Le milieu réactionnel a été concentré en laissant quelques millilitres de solvant et a été filtré sous vide. Le solide obtenu a été lavé au méthanol. 210.00 mg de 6-chloro-2-hydroxy-N,N-diméthyl-3-(2-{[(R)-(1-méthyl-cyclobutyl)-(5-méthyl-furan-2-yl)-méthyl]-amino}-3,4-dioxo-cyclobut-1-enylamino)-benzènesulfonamide ont été obtenus. Rendement = 59.43%, Tf = 152°C.

RMN ¹H (DMSO-d6, 400 MHz): 1.32 (s, 3H), 2.26 (s, 3H), 2.88 (s, 6H), 4.28-4.30 (m, 2H), 4.57 (d, J=6.2 Hz, 1H), 4.64 (d, J=6.2 Hz, 1H), 5.63 (d, J=9.8 Hz, 1H), 6.07 (dd, J=3.1 Hz, J=1.0 Hz, 1H), 6.26 (d, J=3.1 Hz, 1H), 7.22 (d, J=8.8 Hz, 1H), 7.99 (d, J=8.8 Hz, 1H), 8.90 (d, J=9.8 Hz, 1H), 9.58 (s, 1H), 10.58 (s, 1H)

### EXEMPLE 23

### Préparation du 3-(3H-benzotriazol-4-ylamino)-4-{[(5-méthyl-furan-2-yl)-(3-méthyl-oxetan-3-yl)-méthyl]-amino}-cyclobut-3-ène-1,2-dione

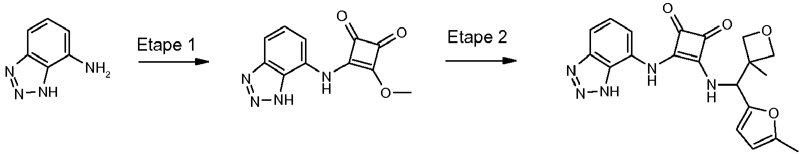

### Etape 1:

### 3-(3H-Benzotriazol-4-ylamino)-4-méthoxy-cyclobut-3-ène-1,2-dione

Un mélange de 2.045 g (15.2 mmol, 1 éq) de 1H-1,2,3-benzotriazol-4-amine et de 1.80 g (12.7 mmol, 1 éq) de 3,4-diméthoxy-3-cyclobutene-1,2-dione dans 30 mL de méthanol a été agité à température ambiante pendant 2 Jours. Le milieu réactionnel (pris en masse) a été filtré. 2.04 g de mélange de 3-(3H-benzotriazol-4-ylamino)-4-méthoxy-cyclobut-3-ène-1,2-dione et de 3,4-bis-(3H-benzotriazol-4-ylamino)-cyclobut-3-ène-1,2-dione ont été obtenus sous forme d'un solide jaune. Mélange de produits (29% bon produit). Rendement = 19%

### Etape 2:

### 3-(3H-Benzotriazol-4-ylamino)-4-{[(5-métliyl-furan-2-yl)-(3-méthyl-oxetan-3-yl)-méthyl]-amino}-cyclobut-3-ène-1,2-dione

Un mélange de 263 mg (1.45 mmol, 1.2 éq) de C-(5-méthyl-furan-2-yl)-C-(3-méthyl-oxetan-3-yl)-méthylamine et de 1.02 g (1.21 mmol, 1 éq) de 3-(3H-benzotriazol-4-ylamino)-4-méthoxy-cyclobut-3-ène-1,2-dione à 29% dans 25 mL de méthanol a été chauffé à 50°C pendant 24 heures. L'insoluble (dimère) a été filtré et le filtrat a été évaporé. Le résidu a été chromatographié sur gel de silice (colonne puriFlash PF-30SIHP/40G, Spot II) élué dichlorométhane/méthanol (gradient). Le solide a été repris avec de l'acétate d'éthyle et cette phase organique a été lavée plusieurs fois avec une solution de dihydrogénophosphate de sodium IN, séchée sur sulfate de magnésium, filtrée et évaporée. 205 mg de 3-(3H-benzotriazol-4-ylamino)-4-{[(5-méthyl-furan-2-yl)-(3-méthyl-oxetan-3-yl)-méthyl]-amino}-cyclobut-3-ène-1,2-dione ont été obtenus sous forme d'un solide jaune. (Tf = 178-180°C). Rendement = 43% ; LC/MS: 95.97% [393].

RMN ¹H (DMSO-d6, 400 MHz): 1.35 (s, 3H) ; 2.27 (s, 3H) ; 4.31 (t, J=5.8 Hz, 1H) ; 4.6 (dd, J=24.4 Hz, 2H) ; 5.67 (d, J=9.8 Hz, 1H) ; 6.08 (dd, J=3.1 Hz, 1H) ; 6.30 (d, J=3.1 Hz, 1H) ; 7.40 (d, J=8.0 Hz, 1H) ; 7.45-7.49 (t, J=8.0 Hz, 1H) ; 7.85 (d, J=7.5 Hz, 1H) ; 8.93 (d, J=9.2 Hz, 1H) ; 10.6 (s, 1H) ; 15.87 (s, 1H).

### EXEMPLE 24

### 3-{[(5-Méthyl-furan-2-yl)-(3-méthyl-oxetan-3-yl)-méthy]-amino}-4-(2-oxo-2,3-dihydro-benzooxazol-7-ylamino)-cyclobut-3-ène-1,2-dione

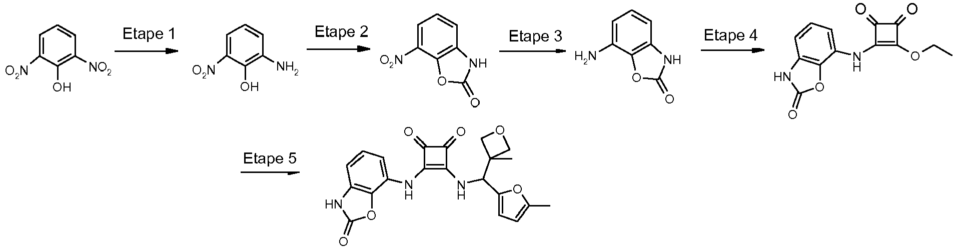

### Etape 1 :

### 2-Amino-6-nitro-phenol

8.6 g de 2,6-dinitro-phenol (46.71 mmol) ont été solubilisés dans 95 mL d'acétate d'éthyle. La solution a été dégazée puis 0.86 g (10% en masse) de palladium sur charbon à 10% ont été ajoutés. Le milieu réactionnel a été agité sous atmosphère d'hydrogène pendant 6 Jours à température ambiante. Le milieu réactionnel a été filtré sur célite et concentré à sec. Le résidu obtenu a été chromatographié sur une cartouche de gel de silice élué au dichlorométhane 100% à dichlorométhane/acétate d'éthyle 80/20. 3.43 g de produit ont été obtenus sous forme d'un solide cuivre foncé. Rendement = 48%.

### Etape 2:

### 7-Nitro-3H-benzooxazol-2-one.

2.27 g de N,N'-carbonyldiimidazole (14 mmoll.4 éq) en suspension homogène dans 6 mL d'acétate d'éthyle a été additionné sur 1.54 g de 2-amino-6-nitro-phenol (10 mmol, 1 éq) en solution dans 10 mL d'acétate d'éthyle. Le milieu réactionnel a été agité vivement pendant 3 heures, puis 10 mL d'eau ont été ajoutés. L'agitation a été poursuivie pendant 15 minutes avant d'évaporer environ ¾ d'acétate d'éthyle. Le milieu réactionnel a été amené à 0°C puis 2 mL d'acide chlorhydrique à 37% ont été ajoutés. L'agitation a été de nouveau poursuivie pendant 15 minutes. Le milieu réactionnel a été filtré. Le solide obtenu a été lavé avec une solution d'acide chlorhydrique IN, de l'eau et avec un mélange eau-éthanol (4:1) puis séché à l'étuve. 1.3 g de produit ont été obtenus sous forme d'un solide jaune pâle. Rendement = 73%.

### Etape 3:

### 7-Amino-3H-benzooxazol-2-one.

Une solution de 0.3 g de 7-nitro-3H-benzooxazol-2-one (1.67 mmol) dans 5 mL d'éthanol a été dégazée puis additionnée de 0.03 g (10% en masse) de palladium sur charbon (5%-50%wet). Le milieu réactionnel a été agité sous atmosphère d'hydrogène pendant 2 heures à température ambiante. Le milieu réactionnel a été filtré sur célite et concentré à sec. 0.25 g de produit a été obtenu sous forme d'une huile incolore. Rendement = 99%.

### Etape 4:

### 3-Ethoxy-4-(2-oxo-2,3-dihydro-benzooxazol-7-ylamino)-cyclobut-3-ène-1,2-dione

Un mélange de 0.247 g de 7-amino-3H-benzooxazol-2-one (1.6 mmol, 1 éq) et de 0.36 mL de 3,4-diéthoxy-cyclobut-3-ène-1,2-dione (2.5 mmol, 1.5 éq) dans 9 mL d'éthanol a été agité à température ambiante pendant 2 jours (formation d'un précipité). De l'éthanol a été additionné pour favoriser la chute du précipité qui a été filtré, lavé à l'éther diéthylique et séché sous vide à 45°C. 0.35 g de produit ont été obtenus sous forme d'un solide kaki clair. Rendement = 78%.

### Etape 5:

### 3-{[(5-Méthyl-furan-2-yl)-(3-méthyl-oxetan-3-yl)-méthyl]-amino}-4-(2-oxo-2,3-dihydro-benzooxazol-7-ylamino)-cyclobut-3-ène-1,2-dione

Un mélange de 0.35 g (1.28 mmol, 1 éq) de 3-éthoxy-4-(2-oxo-2,3-dihydro-benzooxazol-7-ylamino)-cyclobut-3-ène-1,2-dione et de 0.278 g (1.53 mmol, 1.2 éq) de C-(5-méthyl-furan-2-yl)-C-(3-méthyl-oxetan-3-yl)-méthylamine dans 6 mL de méthanol a été chauffé à 65°C pendant 5 heures. Le méthanol a été évaporé et le résidu a été chromatographié sur gel de silice, élué au dichlorométhane/méthanol 95/5. La pâte obtenue a été cristallisée dans l'éther éthylique, filtrée et séchée sous vide à 40°C. 0.24 g de produit a été obtenu sous forme d'un solide beige. Rendement = 46%. HPLC 96.4%, ES+ [410].

RMN ¹H (DMSO-d6, 400 MHz) : 1.33 (s, 3H) ; 2.27 (s, 3H) ; 4.29-4.31 (m, 2H) ; 4.58 ( d, J=6.3 Hz, 2H) ; 4.64 ( d, J=6.2 Hz, 2H) ; 5.62 (d, J=9.8 Hz, 1H) ; 6.07 (m, 1H) ; 6.28 (m, 1H) ; 6.78-6.80 (m, 1H) ; 7.13 (t, J=8.0 Hz, 1H) ; 7.70 (d, J=8.4 Hz, 1H) ; 8.62 (d, J=9.8 Hz, 1H) ; 9.84 (s, 1H) ; 11.79 (s, 1H).

### EXEMPLE 25

### Préparation du 3-hydroxy-4-(2-{[(R)-(5-méthyl-furan-2-yl)-(3-méthyl-oxetan-3-yl)-méthyl]-amino}-3,4-dioxcyclobut-1-enylamino)-thiophene-2-carboxylate de diméthylamide

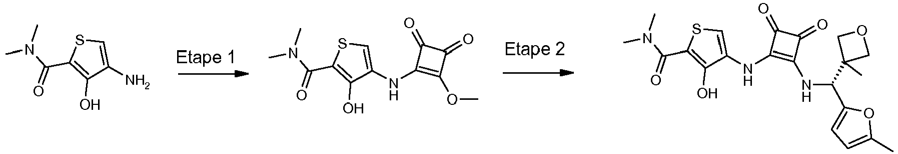

### Etape 1:

### 3-Hydroxy-4-(2-méthoxy-3,4-dioxo-cyclobut-1-enylamino)-thiophene-2-carboxylate de diméthylamide

Un mélange de 5.20 g (27.92 mmol, 1.0 éq) de 4-amino-3-hydroxy-thiophène-2-carboxylate de diméthylamide et de 5.95 g (41.88 mmol, 1.5 éq) de 3,4-diméthoxy-cyclobut-3-ène-1,2-dione dans 52 mL de méthanol a été chauffé à 50°C pendant 16 heures. L'insoluble a été filtré et séché à l'étuve sous vide à 45°C. 7.38 g de 3-hydroxy-4-(2-méthoxy-3,4-dioxo-cyclobut-1-énylamino)-thiophène-2-carboxylate de diméthylamide ont été obtenus. Rendement = 89%.

### Etape 2:

### 3-hydroxy-4-(2-{[(R)-(5-méthyl-furan-2-yl)-(3-méthyl-oxetan-3-yl)-méthyl]-amino}-3,4-dioxcyclobut-1-enylamino)-thiophene-2-carboxylate de diméthylamide

Un mélange de 300 mg (1.01 mmol, 1.0 éq) de 3-hydroxy-4-(2-méthoxy-3,4-dioxo-cyclobut-1-énylamino)-thiophène-2-carboxylate de diméthylamide et de 220 mg (1.21 mmol, 1.2 éq) de C-[(R)-C-(5-méthyl-furan-2-yl)-C-(3-méthyl-oxetan-3-yl)]-méthylamine dans 15 mL de méthanol a été agité à température ambiante pendant 2 Jours et demi puis a été chauffé à 50°C pendant 21 heures. Le milieu réactionnel a été évaporé et le résidu a été chromatographié sur gel de silice (colonne puriFlash IR-50SI/40G, spot II) élué au dichlorométhane/acétate d'éthyle (75/25). 350 mg de 3-hydroxy-4-(2-{[(R)-(5-méthyl-furan-2-yl)-(3-méthyl-oxetan-3-yl)-méthyl]-amino } -3,4-dioxo-cyclobut-1-énylamino)-thiophène-2-carboxylate de diméthylamide ont été obtenus. Rendement = 78%.

RMN ¹H (DMSO-d6, 400 MHz) : 1.30 (s, 3H), 2.25 (s, 3H), 3.15 (s, 6H), 4.29 (dd, J=2.9-6.2 Hz, 2H), 4.60 (dd, J=6.2-20.4 Hz, 2H), 5.60 (d, J=9.8 Hz, 1H), 6.06 (dd, J=1.0-3.0 Hz, 1H), 6.25 (d, J=3.0 Hz, 1H), 7.84 (s, 1H), 8.67 (d, J=9.8 Hz, 1H), 9.66 (s, 1H), 13.01 (s, 1H).

### EXEMPLE 26

### Préparation du 3-[[2-(3-Diméthylcarbamoyl-2-hydroxy-phenylamino)-3,4-dioxo-cyclobut-1-enylamino]-(5-méthyl-furan-2-yl)-méthyl]-3-méthyl-azetidine-1-carboxylate de tert-butyle

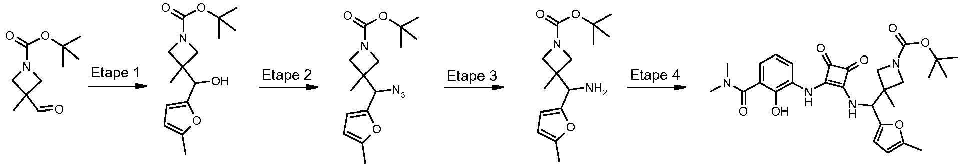

### Etape 1:

De manière analogue à l'EXEMPLE 1 (étape 1), 3-[hydroxy-(5-méthyl-furan-2-yl)-méthyl]-3-méthyl-azetidine-1-carboxylate de tert-butyle a été préparé. Rendement = 100%.

### Etape 2:

De manière analogue à l'EXEMPLE 1 (étape 2), 3-[azido-(5-méthyl-furan-2-yl)-méthyl]-3-méthyl-azetidine-1-carboxylate de tert-butyle a été préparé. Rendement = 48%.

### Etape 3:

De manière analogue à l'EXEMPLE 1 (étape 3), 3-[amino-(5-méthyl-furan-2-yl)-méthyl]-3-méthyl-azetidine-1-carboxylate de tert-butyle a été préparé. Rendement = 75%.

### Etape 4:

De manière analogue à l'EXEMPLE 1 (étape 7), 3-[[2-(3-Diméthylcarbamoyl-2-hydroxy-phenylamino)-3,4-dioxo-cyclobut-1-enylamino]-(5-méthyl-furan-2-yl)-méthyl]-3-méthyl azetidine-1- carboxylate de tert-butyle a été préparé. Rendement = 88%, Tf = 162-165°C, LC/MS : 99.56% [538].

RMN ¹H (DMSO-d6, 400 MHz)_: 1.27 (s, 3H) ; 1.36 (s, 9H) ; 2.27 (s, 3H) ; 2.94 (s, 6H) ; 3.55 (s, 2H) ; 3.9 (m, 2H) ; 5.45 (d, 1H) ; 6.07 (dd, J=3.0 Hz, 1H) ; 6.29 (d, J=3.1 Hz, 1H) ; 6.88 (m, 2H) ; 7.75 (d, J=2.9 Hz, 1H) ; 8.8 (d, 1H) ; 9.42 (s, 1H) ; 10.1 (s, 1H).

### EXEMPLE 27

### Préparation du 3-(2-{[(4,5-diméthyl-furan-2-yl)-(3-méthyl-oxetan-3-yl)-méthyl]-amino}-3,4-dioxo-cyclobut-1-enylamino)-2-hydroxy-N,N-diméthyl-benzamide

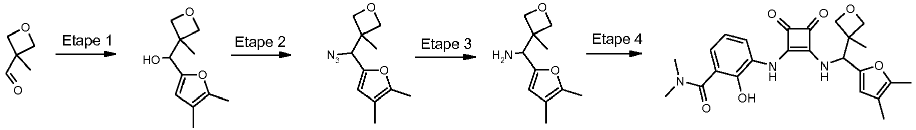

### Etape 1:

De manière analogue à l'EXEMPLE 1 (étape 1) et à partir de 2,3-diméthylfurane, (4,5-diméthyl-furan-2-yl)-(3-méthyl-oxetan-3-yl)-méthanol a été préparé. Rendement = 65%.

### Etape 2:

De manière analogue à l'EXEMPLE 1 (étape 2), 5-[azido-(3-méthyl-oxetan-3-yl)-méthyl]-2,3-diméthyl-furane a été préparé. Rendement = 60%.

### Etape 3:

De manière analogue à l'EXEMPLE 1 (étape 3), C-(4,5-diméthyl-furan-2-yl)-C-(3-méthyl-oxetan-3-yl)-méthylamine ester a été préparé. Rendement = 96%.

### Etape 4:

De manière analogue à l'EXEMPLE 1 (étape 7), 3-(2-{[(4,5-diméthyl-furan-2-yl)-(3-méthyl-oxetan-3-yl)-méthyl]-amino}-3,4-dioxo-cyclobut-1-enylamino)-2-hydroxy-N,N-diméthylbenzamide a été préparé. Rendement = 75%, Tf = 207-209°C, LC/MS: 97.42% [453]. RMN ¹H (DMSO-d6, 400 MHz)_: 1.32 (s, 3H) ; 1.88 (s, 3H) ; 2.17 (s, 3H) ; 2.94 (s, 6H) ; 4.28 (dd, J=6.2 Hz, 2H) ; 4.6 (dd, J=29.6 Hz, 2H) ; 5.59 (d, *J*=9.7 Hz, 1H) ; 6.15 (s, 1H) ; 6.88 (m, 2H) ; 7.76 (dd, J=6.6 Hz, 1H) ; 8.80 (d, J=9.8 Hz, 1H) ; 9.45 (s, 1H) ; 10.0 (s, 1H).

### EXEMPLE 28

### Préparation du 3-(2-{[(3-méthyl-oxetan-3-yl)-(3-méthoxy-phenyl)-méthyl]-amino}-3,4-dioxo-cyclobut-1-enylamino)-2-hydroxy-N,N-diméthyl-benzamide

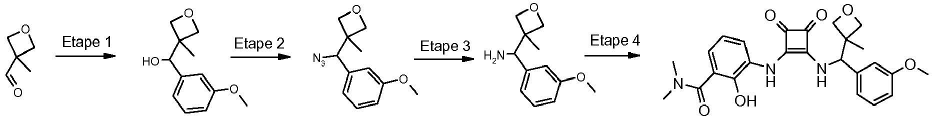

### Etape 1:

### (3-Méthoxy-phenyl)-(3-méthyl-oxetan-3-yl)-méthanol.

A une solution de 3-méthyl-oxetane-3-carbaldehyde (0.5 g, 5.0 mmol) dans 8 mL de tétrahydrofuranne refroidie à -70°C a été ajoutée goutte à goutte le bromure de m-méthoxybenzène magnésium 1.0 M dans le tétrahydrofuranne (25 mL, 25.0 mmol). Le milieu réactionnel a été agité pendant 4 heures à -70°C puis laissé remonter à 0°C, hydrolysé avec une solution saturée de chlorure d'ammonium et extrait à l'acétate d'éthyle. Les phases organiques ont été rassemblées, lavées avec une solution saturée de chlorure de sodium et évaporées. Le résidu obtenu a été purifié par chromatographie sur une cartouche de gel de silice (élué à l'heptane/acétate d'éthyle, gradient de 90/10 à 60/40). 0.45 g de produit a été obtenu sous forme d'une huile épaisse incolore. Rendement = 43%.

### Etape 2:

De manière analogue à l'EXEMPLE 1 (étape 2), 3-[azido-(3-méthoxy-phenyl)-méthyl]-3-méthyl-oxetane a été préparé. Rendement = 28%.

### Etape 3:

De manière analogue à l'EXEMPLE 1 (étape 3), C-(3-méthoxy-phenyl)-C-(3-méthyl-oxetan-3-yl)-méthylamine a été préparée. Rendement = 64%.

### Etape 4:

De manière analogue à l'EXEMPLE 1 (étape 7), 3-(2-{[(3-méthyl-oxetan-3-yl)-(3-méthoxy-phenyl)-méthyl]-amino}-3,4-dioxo-cyclobut-1-enylamino)-2-hydroxy-N,N-diméthyl-benzamide a été préparé. Rendement = 76%, HPLC 94.3%, ES+ [466].

RMN ¹H (DMSO-d6, 400 MHz) : 1.09 (s, 3H) ; 2.95 (s, 6H) ; 3.77 (s, 3H) ; 4.18 (d, 1H) ; 4.29 (d, 1H) ; 4.74 (t, 2H) ; 5.58 (d, 1H) ; 6.82-6.93 (m, 5H) ; 7.34 (t, 1H) ; 8.74 (d, 1H) ; 9.40 (s, 1H) ; 10.05 (s, 1H).

### EXEMPLE 29

### Préparation du 3-(2-{[(3-méthyl-oxetan-3-yl)-(4-méthoxy-phenyl)-méthyl]-amino}-3,4-dioxo-cyclobut-1-enylamino)-2-hydroxy-N,N-diméthyl-benzamide

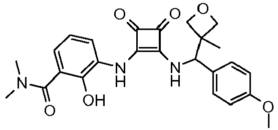

De manière analogue à l'EXEMPLE 28, (étapes 1 à 4), 3-(2-{[(3-méthyl-oxetan-3-yl)-(4-méthoxy-phenyl)-méthyl]-amino }-3,4-dioxo-cyclobut-1-enylamino)-2-hydroxy-N,N-diméthyl-benzamide a été préparé. HPLC 98.97%, ES+[465].

RMN ¹H (DMSO-d6, 400 MHz) : 1.28 (s, 3H) ; 2.95 (s, 6H) ; 3.75 (s, 3H) ; 4.18 ( d, 1H) ; 4.29 (d, 1H) ; 4.67 (d, 1H) ; 4.72 (d, 1H) ; 5.56 (d, 1H) ; 6.88 (d, 2H) ; 6.97 (d, 2H) ; 7.21 (d, 2H) ; 7.72-7.74 (m, 1H) ; 8.74 (d, 1H) ; 9.40 (s, 1H)

### EXEMPLE 30

### Préparation du 3-(2-{[benzo[1,3]dioxol-5-yl-(3-méthyl-oxetan-3-yl)-méthyl]-amino}-3,4-dioxo-cyclobut-1-enylamino)-2-hydroxy-N,N-diméthyl-benzamide

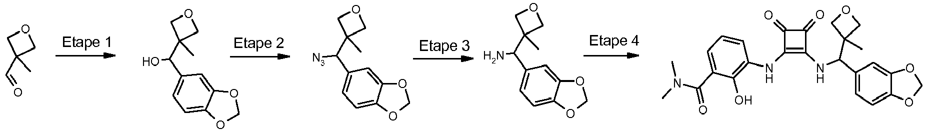

### Etape 1:

De manière analogue à l'EXEMPLE 28, (étape 1), benzo[1,3]dioxol-5-yl-(3-méthyl-oxetan-3-yl)-méthanol a été préparé. Rendement = 62%.

### Etape 2:

De manière analogue à l'EXEMPLE 1 (étape 2), C-benzo[1,3]dioxol-5-yl-C-(3-méthyl-oxetan-3-yl)-méthylazide a été préparé. Rendement = 33%.

### Etape 3:

De manière analogue à l'EXEMPLE 1 (étape 3), C-benzo[l,3]dioxol-5-yl-C-(3-méthyl-oxetan-3-yl)-méthylamine a été préparée. Rendement = 99%.

### Etape 4:

De manière analogue à l'EXEMPLE 1 (étape 7), 3-(2-{[benzo[1,3]dioxol-5-yl-(3-méthyl-oxetan-3-yl)-méthyl]-amino}-3,4-dioxo-cyclobut-1-enylamino)-2-hydroxy-N,N-diméthylbenzamide a été préparé. Rendement = 57%, HPLC 98.0%, ES+ [480].

RMN ¹H (DMSO-d6, 400 MHz) : 1.29 (s, 3H) ; 2.95 (s, 6H) ; 4.17 (d, J=6.0Hz, 2H) ; 4.28 (d, J=6.1Hz, 2H) ; 4.68-473 (m, 2H) ; ; 5.51 (d, J=9.8Hz, 2H) ; 6.03 (s, 2H) ; 6.74-6.77 (m, 1H) ; 6.84-6.95 (m, 4H) ; 7.71-7.73 (m, 1H) ; 8.70 (d, , 1H) 9.40 (s, 1H) ; 10.05 (s, 1H).

### EXEMPLE 31

### Préparation du 3-[3-(3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène-1,2-dione

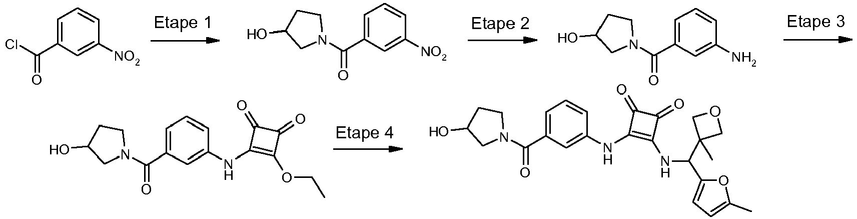

### Etape 1:

### (3-Hydroxy-pyrrolidin-1-yl)-(3-nitro-phényl)-méthanone

A une solution de 3.71 g de chlorure de 3-nitro-benzoyle (20 mmol) dans 40 mL de dichlorométhane refroidie à 0°C ont été ajoutés 2.22 g de triéthylamine (20 mmol) suivis de 1.74 g de pyrrolidin-3-ol (20 mmol). Le milieu réactionnel a été agité à température ambiante pendant 2 heures puis a été dilué avec 50 mL de dichloromethane. La phase organique a été lavée avec 50 mL d'une solution d'acide chlorhydrique IN puis avec 50 mL d'une solution saturée d'hydrogénocarbonate de sodium, séchée sur sulphate de magnésium et évaporée. 3.78 g de (3-hydroxy-pyrrolidin-1-yl)-(3-nitro-phenyl)-méthanone ont été obtenus et utilisés sans purification dans l'étape suivante. Rendement = 80%.

### Etape 2:

### (3-Amino-phényl)-(3-hydroxy-pyrrolidin-1-yl)-méthanone

Un mélange de 2.36 g de (3-hydroxy-pyrrolidin-1-yl)-(3-nitro-phenyl)-méthanone dans 40 mL d'éthanol et en présence de 300 mg de palladium sur charbon à 10% a été agité sous pression atmosphérique d'hydrogène pendant 2 Jours. Le milieu réactionnel a été filtré sur célite et lavé avec 50 mL d'éthanol. Le solvant a été concentré Jusqu'à 30 mL. Cette solution de (3-amino-phényl)-(3-hydroxy-pyrrolidin-1-yl)-méthanone a été utilisée dans l'étape suivante. Rendement supposé = 100%.

### Etape 3

### 3-Ethoxy-4-[3-(3-hydroxy-pyrrolidine-1-carbonyl)-phenylamino]-cyclobut-3-ène-1,2-dione:

Un mélange des 30 mL de la solution de (3-amino-phényl)-(3-hydroxy-pyrrolidin-1-yl)-méthanone dans l'éthanol et de 6.81 g de 3,4-diéthoxy-cyclobut-3-ène-1,2-dione (40.0 mmol, 2.5 éq) a été chauffé à 60°C pendant la nuit. Le milieu réactionnel a été évaporé et le résidu a été chromatographié sur gel de silice élué à l'heptane/acétate d'éthyle 4/1, 2:1 puis dichlorométhane/méthanol (6%). 3.03 g de 3-éthoxy-4-[3-(3-hydroxy-pyrrolidine-1-carbonyl)-phenylamino]-cyclobut-3-ène-1,2-dione ont été obtenus. Rendement = 63%.

### Etape 4

### 3-[3-(3-Hydroxy-pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione

Un mélange de 314 mg (1.73 mmol, 1.2 éq) de C-(5-méthyl-furan-2-yl)-C-(3-méthyl-oxetan-3-yl)-méthylamine et de 500 g (1.44 mmol, 1 éq) de 3-éthoxy-4-[2-hydroxy-3-(3-hydroxy-pyrrolidine-1-carbonyl)-phenylamino]-cyclobut-3-ène-1,2-dione dans 15 mL de méthanol a été chauffé à 50°C pendant 6 Jours. Le milieu réactionnel a été évaporé et le résidu a été chromatographié sur gel de silice HP (colonne Redi*Sep Rf* Gold 40g, Spot II) élué dichlorométhane/méthanol (gradient).

388 mg de 3-[3-(3-hydroxy-pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione ont été obtenus sous forme d'un solide jaune. Rendement = 58%, Tf= 242-245°C, LC/MS: 98.24% [465]

RMN ¹H (DMSO-d6, 400 MHz)_: 1.32 (s, 3H) ; 1.79-1.94 (m, 2H) ; 2.26 (s, 3H) ; 3.35-3.43 (m, 2H) ; 3.50-3.62 (m, 2H) ; 4.23 & 4.32 (d, J=37.7 Hz, 1H) ; 4.29 (dd, J=6.2 Hz, 2H) ; 4.6 (dd, J=24.9 Hz, 2H) ; 4.95 (d, 1H) ; 5.59 (d, J=9.6 Hz, 1H) ; 6.06 (dd, J=3.0 Hz, 1H) ; 6.28 (d, J=3.1 Hz, 1H) ; 7.16 (dd, J=7.3 Hz, 1H) ; 7.40-7.47 (m, 2H) ; 7.64 (s, 1H) ; 8.27 (d, J=6.6 Hz, 1H) ; 9.80 (s, 1H).

### EXEMPLE 32

### Préparation du 1-[3-(2-{[(5-méthyl-furan-2-yl)-(3-méthyl-oxetan-3-yl)-méthyl]-amino}-3,4-dioxo-cyclobut-1-enylamino)-benzoyl]-piperidine-2-carboxylate de méthyle

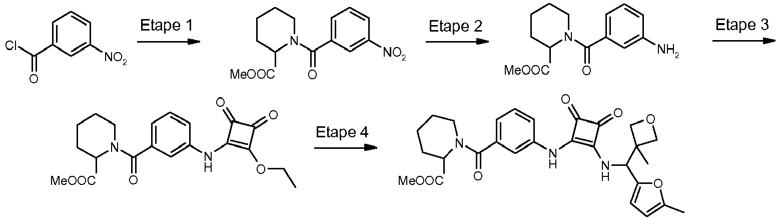

### Etape 1:

De manière analogue à l'EXEMPLE 31, (étape 1), 1-(3-nitro-benzoyl)-piperidine-2-carboxylate de méthyle a été préparé. Rendement = 84%.

### Etape 2:

De manière analogue à l'EXEMPLE 31, (étape 2), 1-(3-amino-benzoyl)-piperidine-2-carboxylate de méthyle a été préparé. Rendement = 99%.

### Etape 3:

### De manière analogue à l'EXEMPLE 31, (étape 3), 1-[3-(2-éthoxy-3,4-dioxo-cyclobut-1-enylamino)-benzoyl]-piperidine-2-carboxylate de méthyle a été préparé. Rendement = 81%.

### Etape 4:

De manière analogue à l'EXEMPLE 31, (étape 4), 1-[3-(2-{[(5-méthyl-furan-2-yl)-(3-méthyl-oxetan-3-yl)-méthyl]-amino}-3,4-dioxo-cyclobut-1-enylamino)-benzoyl]-piperidine-2-carboxylate de méthyle a été préparé. Rendement = 52%, Tf = 130-132°C, LC/MS: 97.12% [521]

RMN ¹H (DMSO-d6, 400 MHz)_: 1.27 (m, 1H) ; 1.32 (s, 3H) ; 1.41-1.52 (m, 2H) ; 1.70 (m, 2H) ; 2.26 (s, 3H) ; 3.66-3.73 (d, 3H) ; 4.30 (dd, J=6.2 Hz, 1H) ; 4.6 (dd, J=24.6 Hz, 2H) ; 5.58 (d, J=9.7 Hz, 1H) ; 6.06 (dd, J=3.0 Hz, 1H) ; 6.28 (d, J=3.1 Hz, 1H) ; 7.03 (m, 1H) ; 7.42-7.51 (m, 3H) ; 7.64 (s, 1H) ; 8.27 (d, J=9.8 Hz, 1H) ; 9.80 (s, 1H).

### EXEMPLE 33

### Préparation du 1-[3-(2-{[(5-Méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle

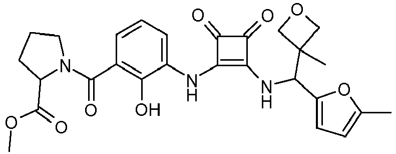

De manière analogue à l'EXEMPLE 31, (étapes 1 à 4), 1-[3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle a été préparé.

RMN ¹H (DMSO-d6, 400 MHz) : 1.33 (s, 3H) ; 1.86-1.92 (m, 3H) ; 2.20-2.30 (m, 1H) ; 2.26 (s, 3H) ; 3.54-3.59 (m, 2H) ; 367 (s, 3H) ; 4.29-4.30 (dd, 2H) ; 4.47-4.51 ( 1H) ; 4.58 (d, J=6.2 Hz, 1H) ; 4.64 (d, J=6.3 Hz, 1H) ; 5.58 (d, J=9.8 Hz, 1H) ; 6.06 (m, 1H) ; 6.28 (d, 1H) ; 7.18 (d, J=7.5 Hz, 1H) ; 7.42-7.44 (m, 1H) ; 7.52-7.54 (m, 1H) ; 7.64 (s, 1H) ; 7.09-7.11 (d, J=9.8 Hz, 1H) ; 9.81 (s, 1H).

### EXEMPLE 34

### Préparation du 2-hydroxy-N,N-diméthyl-3-(2-{[(3-méthyl-oxetan-3-yl)-thiophen-2-yl-méthyl]-amino}-3,4-dioxo-cyclobut-1-enylamino)-benzamide

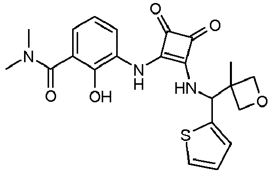

De manière analogue à l'EXEMPLE 28, (étapes 1 à 4), 2-hydroxy-N,N-diméthyl-3-(2-{[(3-méthyl-oxetan-3-yl)-thiophen-2-yl-méthyl]-amino } -3,4-dioxo-cyclobut-1-enylamino)-benzamide a été préparé. HPLC 96.6% ES+ [441]

RMN ¹H (DMSO-d6, 400 MHz) : 1.35 (s, 3H) ; 2.89 (s, 6H) ; 4.31-4.34 (m, 2H) ; 4.65-4.69 ( m, 2H) ; 5.95 (d, 1H) ; 6.88-6.92 (m, 2H) ; 7.02-7.07 (m, 2H) ; 7.53-7.55 (d, 1H) ; 7.74-7.76 (m, 1H) ; 8.82 (d 1H) ; 9.45 (s, 1H).

### EXEMPLE 35

### Préparation du 2-hydroxy-N,N-diméthyl-3-(2-{[(3-méthyl-oxetan-3-yl)-(5-méthyl-thiophen-2-yl)-méthyl]-amino}-3,4-dioxo-cyclobut-1-enylamino)-benzamide.

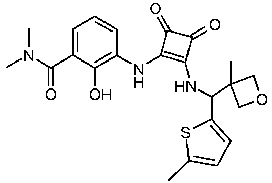

De manière analogue à l'EXEMPLE 1 (étapes 1 à 7) et à partir du 2-bromo-5-méthylthiophène, 2-hydroxy-N,N-diméthyl-3-(2-{[(3-méthyl-oxetan-3-yl)-(5-méthyl-thiophen-2-yl)-méthyl]-amino}-3,4-dioxo-cyclobut-1-enylamino)-benzamide a été préparé. HPLC 98.7%, ES+ [457].

RMN ¹H (DMSO-d6, 400 MHz) : 1.35 (s, 3H) ; 2.42 (s, 3H) ; 2.94 (s, 6H) ; 4.29-4.33 (m, 2H) ; 4.62-4.67 (m, 2H) ; 5.84 (d , J=9.7 Hz, 1H) ; 6.71-6.72 (m, 1H) ; 6.79 (m, 1H) ; 6.87-6.90 (d, 2H) ; 7.73-7.75 (m, 1H) ; 8.75 (d 1H) ; 9.43 (s, 1H) ; 10.00 (s, 1H) ;

### EXEMPLE 36

### Préparation du 3-(2-{[furan-2-yl-(3-méthyl-oxetan-3-yl)-méthyl]-amino}-3,4-dioxo-cyclobut-1 enylamino)-2-hydroxy-N,N-diméthyl-benzamide.

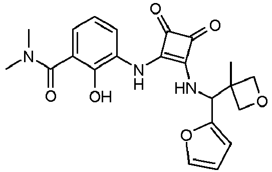

De manière analogue à l'EXEMPLE 1 (étapes 1 à 7), 3-(2-{[furan-2-yl-(3-méthyl-oxetan-3-yl)-méthyl]-amino}-3,4-dioxo-cyclobut-1 enylamino)-2-hydroxy-N,N-diméthyl-benzamide a été préparé.

HPLC 96.8%, ES- [424].

RMN ¹H (DMSO-d6, 400 MHz) : 1.30 (s, 3H) ; 2.94 (s, 6H) ; 4.31 (d, J=6.2 Hz, 2H) ; 4.62 (d, J=6.3Hz, 1H) ; 4.67 (d, J=6.2 Hz, 1H) ; 5.71 (d, J=9.7 Hz, 1H) ; 6.41 (d, J=3.2 Hz, 1H) ; 6.48 (m, 1H) ; 6.87-6.92 (m, 2H) ; 7.71(m, 1H) ; 7.75-7.78 (m, 1H) ; 8.85 (d, J=9.7 Hz, 1H) ; 9.46 (s, 1H) ; 10.00 (s, 1H) ;

### EXEMPLE 37a et 37b

### Préparation du (a) 2-hydroxy-N,N-diméthyl-3-(2-{[(4-méthyl-furan-2-yl)-(3-méthyl-oxetan-3-yl)-méthyl]-amino}-3,4-dioxo-cyclobut-1-enylamino)-benzamide et du (b) 2-Hydroxy-N,N-diméthyl-3-(2-{[(3-méthyl-furan-2-yl)-(3-méthyl-oxetan-3-yl)-méthyl]-amino}-3,4-dioxo-cyclobut-1-enylamino)-benzamide.

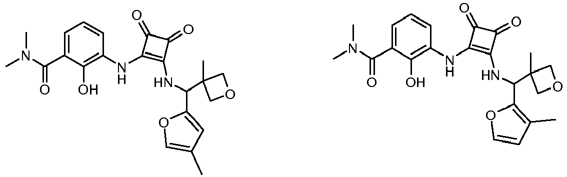

De manière analogue à l'EXEMPLE 1 (étapes 1 à 7), et à partir de la litiation du 3-méthylfurane qui n'a pas été régiosélective et qui se fait aux positions 5- et 2-, 2-hydroxy-N,N-diméthyl-3-(2-{[(4-méthyl-furan-2-yl)-(3-méthyl-oxetan-3-yl)-méthyl]-amino}-3,4-dioxo-cyclobut-1-enylamino)-benzamide et 2-hydroxy-N,N-diméthyl-3-(2-{[(3-méthyl-furan-2-yl)-(3-méthyl-oxetan-3-yl)-méthyl]-amino}-3,4-dioxo-cyclobut-1-enylamino)-benzamide ont été préparés. Sachant que la séparation des régioisomères a été faite à l'étape de l'azide par chromatographie sur gel de silice élué à l'heptane/acétate d'éthyle 95/5.

### 2-hydroxy-N,N-diméthyl-3-(2-{[(4-méthyl-furan-2-yl)₋(3-méthyl-oxetan-3-yl)-méthyl]-amino}-3A-dioxo-cyclobut-1-enylamino)-benzamide:

HPLC 93.8%, ES+ [440]

RMN ¹H (DMSO-d6, 400 MHz) : 1.30 (s, 3H) ; 1.97 (s, 3H) ; 2.95 (s, 6H) ; 4.30 (d, J=6.1 Hz, 1H) ; 4.60 ( d, J=6.1 Hz, 1H) ; 4.66 (d, J=6.0 Hz, 1H) ; 5.65 (d, J=12.2 Hz, 1H) ; 6.29 (s, 1H) ; 6.89-6.92 (m, 2H) ; 7.45 (s, 1H) ; 7.77 (m, 1H) ; 8.82 (d, J=9.6 Hz, 1H) ; 10.05 (s, 1H).

### 2-Hydroxy-N,N-diméthyl-3-(2-{[(3-méthyl-furan-2-yl)-(3-méthyl-oxetan-3-yl)-méthyl]-amino}-3,4-dioxo-cyclobut-1-enylamino)-benzamide:

HPLC 88.00% +12%, ES+ [440]

RMN ¹H (DMSO-d6, 400 MHz) : 1.30 (s, 3H) ; 2.02 (s, 3H) ; 2.94 (s, 6H) ; 4.27 (d, J=6.1 Hz, 1H) ; 4.31 ( d, J=6.3 Hz, 1H) ; 4.48 (d, J=6.1 Hz, 1H) ; 4.76 (d, J=6.1 Hz, 1H) ; 5.63 (d, J=9.7Hz, 1H) ; 6.36 (s, 1H) ; 6.87-6.91 (m, 2H) ; 7.61 (s, 1H) ; 7.75 (m, 1H) ; 9.00(d, J=9.6 Hz, 1H) ; 9.49 (s, 1H).

### EXEMPLE 38

### Préparation du 2-hydroxy-N,N-diméthyl-3-(2-{[(4-isopropyl-furan-2-yl)-(3-méthyl-oxetan-3-yl)-méthyl]-amino}-3,4-dioxo-cyclobut-1-enylamino)-benzamide

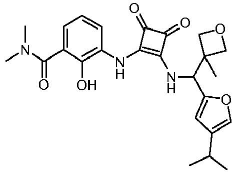

De manière analogue à l'EXEMPLE 1 (étapes 1 à 7), et à partir de 3-isopropylfurane, 2-hydroxy-3-(2-{[(4-isopropyl-furan-2-yl)-(3-méthyl-oxetan-3-yl)-méthyl]-amino}-3,4-dioxo-cyclobut-1-enylamino)-N,N-diméthyl-benzamide a été préparé. HPLC 97.2%, ES- [466]. RMN ¹H (DMSO-d6, 400 MHz) : 1.14 (d, J=6.8 Hz, 3H) ; 1.30 (s, 3H) ; 6.68-2.73 (m, 1H) ; 2.94 (s, 6H) ; 4.29-4.31 (m, 2H) ; 4.60 (d, J=6.3 Hz, 1H) ; 4.65 (d, J=6.2 Hz, 1H) ; 5.67 (d, J=9.7 Hz, 1H) ; 6.37 (s, 1H) ; 6.87-6.90 (m, 2H) ; 7.44 (s, 1H) ; 7.76-7.79 (m, 1H) ; 8.84 (d, J=9.7 Hz, 1H) ; 9.46 (se, 1H) ; 10.00 (s, 1H).

### EXEMPLE 39

### Préparation du 3-(2-hydroxy-6-méthoxy-pyridin-3-ylamino)-4-{[(5-méthyl-furan-2-yl)-(3-méthyl-oxetan-3-yl)-méthyl]-amino}-cyclobut-3-ène-1,2-dione

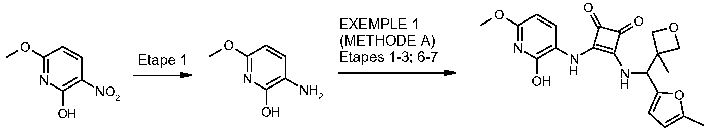

### Etape 1:

### 3-Amino-6-méthoxy-1H-pyridin-2-one.

1.0 g de 6-méthoxy-3-nitro-1H-pyridin-2-one (5.88 mmol) a été solubilisé dans 16 mL de méthanol. La solution a été dégazée puis additionnée de palladium sur charbon 10% (0.10 g, 10% en masse). Le milieu réactionnel a été agité sous atmosphère d'hydrogène à 40°C pendant 4 heures. Le milieu réactionnel a été filtré sur célite et concentré à sec. Le résidu a été chromatographié sur gel de silice, élué au dichlorométhane/méthanol 95/5. 0.30 g du composé a été isolé. Rendement = 36%.

De manière analogue à l'EXEMPLE 1 (étapes 1 à 3, 6 et 7), 3-(2-hydroxy-6-méthoxy-pyridin-3-ylamino)-4-{[(5-méthyl-furan-2-yl)-(3-méthyl-oxetan-3-yl)-méthyl]-amino}-cyclobut-3-ène-1,2-dione a été préparée. HPLC 98.8%, ES- [399].

RMN ¹H (DMSO-d6, 400 MHz) : 1.30 (s, 3H) ; 2.25 (s, 3H) ; 3.80 (s, 3H) ; 4.26-4.29 (m, 2H) ; 4.56 (d, J=6.2 Hz, 1H) ; 4.63 (d, J=6.2 Hz, 1H) ; 5.62 (d, J=9.8 Hz, 1H) ; 6.05-6.06 (m, 1H) ; 6.23-6.24 (m, 12H) ; 8.00 (d, J=8.2 Hz, 1H) ; 9.40 (s, 1H).

### EXEMPLE 40

### Préparation du 3-(6-chloro-2-hydroxy-pyridin-3-ylamino)-4-{[(5-méthyl-furan-2-yl)-(3-méthyl-oxetan-3-yl)-méthyl]-amino}-cyclobut-3-ène-1,2-dione

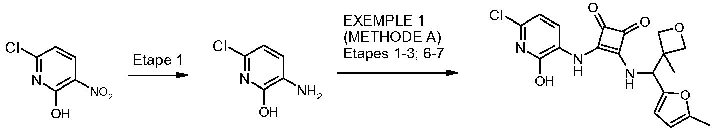

### Etape 1:

### 3-Amino-6-chloro-1H-pyridin-2-one

A une solution de 1.0 g de 6-chloro-3-nitro-1H-pyridin-2-one (5.73 mmol) dans 15 mL d'acétate d'éthyle a été ajouté par portions 9.2 g de chlorure d'étain dihydrate (40.7 mmol, 7 éq). Le milieu réactionnel a été chauffé à reflux pendant 3 heures, refroidit puis dilué avec 60 mL d'acétate d'éthyle et neutralisé avec 14 g de bicarbonate de sodium (poudre). Le milieu réactionnel a été filtré. Le solide a été lavé à l'acétate d'éthyle (2 x 15 mL) et le filtrat a été concentré à sec. Le résidu a été chromatographié sur gel de silice, élué au dichlorométhane/méthanol 95/5. 0.60 g de produit a été obtenu sous forme d'un solide beige. Rendement = 72%.

De manière analogue à l'EXEMPLE 1 (étapes 1 à 3, 6 et 7), et à partir de 6-chloro-3-nitro-1H-pyridin-2-one, 3-(6-chloro-2-hydroxy-pyridin-3-ylamino)-4-{[(5-méthyl-furan-2-yl)-(3-méthyl-oxetan-3-yl)-méthyl]-amino}-cyclobut-3-ène-1,2-dione a été préparée. HPLC 94.8%, ES- [402].

RMN ¹H (DMSO-d6, 400 MHz) : 1.37 (s, 3H) ; 2.32 (s, 3H) ; 4.33-4.35 (m, 2H) ; 4.62 (d, J=6.2 Hz, 1H) ; 4.68 (d, J=62 Hz, 1H) ; 5.68 (d, J=9.7 Hz, 1H) ; 6.12 (s, 1H) ; 6.31 (s, 1H) ; 8.18 (s, 2H) ; 9.00 (s, 1H) ; 9.59 (s, 1H) ; 12.70-13.00 (s, 1H).

### EXEMPLE 41

### Préparation du 3-{[(5-Méthyl-furan-2-yl)-(3-méthyl-oxetan-3-yl)-méthyl]-amino}-4-(2-oxo-2,3-dihydro-1H-benzoimidazol-4-ylamino)-cyclobut-3-ène-1,2-dione

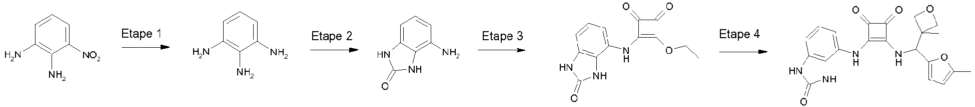

### Etape 1 :

### Benzène-1,2,3-triamine

Une solution de 3-nitro-benzène-1,2-diamine à 0.05 mol/L dans le méthanol a été passée dans le H-Cube sur une cartouche de palladium sur charbon avec un débit de 1mL/mn. Au bout d'une heure et demie, le produit a été complètement hydrogéné. Le solvant a été concentré. 450.7 g de benzène-1,2,3-triamine ont été obtenus sous forme d'une huile verte. Rendement >100%.

### Etape 2:

### 4-Amino-1,3-dihydro-benzoimidazol-2-one

0.53 g (6.5 mmol, 2 éq) de N-N'-carbonyliimidazole ont été additionnés par portions sur 450.7 mg (3.24 mmol, 1 éq) de benzène-1,2,3-triamine en solution dans 30 mL d'acétonitrile Le milieu réactionnel a été agité à température ambiante pendant 6 heures puis chauffé à 70°C pendant la nuit. La réaction a été arrêtée par l'ajout de 50 mL d'eau puis extraite avec de l'acétate d'éthyle. Les phases organiques ont été rassemblées et séchées sur sulfate de sodium. Les solvants ont été évaporés puis le résidu a été purifié par chromatographie sur gel de silice (dichlorométhane/méthanol/ammoniaque : 95/5/2). 229.9 mg de 4-amino-1,3-dihydro-benzoimidazol-2-one ont été obtenus. Rendement = 42%.

### Etape 3:

### 3-Ethoxy-4-(2-oxo-2,3-dihydro-1H-benzoimidazol-4-ylamino)-cyclobut-3-ène-1,2-dione

A une solution de 0.59 g de 4-amino-1,3-dihydro-benzoimidazol-2-one (4.0 mmol, 1 éq) dans 21 mL d'éthanol a été ajoutée 0.86 mL de 3,4-diéthoxy-cyclobut-3-ène-1,2-dione (5.9 mmol, 1.5 éq). Le milieu réactionnel a été agité à température ambiante pendant 2 jours (formation d'un précipité). De l'éthanol a été additionnée pour favoriser la chute du précipité qui a été filtré, lavé à l'éther diéthylique et séché sous vide à 45°C. Le résidu a été chromatographié sur gel de silice, élué au dichlorométhane/méthanol 90/10. 0.42 de produit a été obtenu sous forme d'un solide blanc. Rendement = 38.6%.

### Etape 4:

### 3-{[(5-Méthyl-furan-2-yl}-(3-méthyl-oxetan-3-yl}-méthyl]-amino}-4-(2-oxo-2,3-dihydro-1H-benzoimidazol-4-ylamino)-cyclobut-3-ène-1,2-dione.

A une solution de 0.41 g de 3-éthoxy-4-(2-oxo-2,3-dihydro-1H-benzoimidazol-4-ylamino)-cyclobut-3-ène-1,2-dione (1.50 mmol, 1 éq) dans 8 mL de méthanol a été ajoutée 0.33 g de C-(5-Méthyl-furan-2-yl)-C-(3-méthyl-oxetan-3-yl)-méthylamine (1.80 mmol, 1.2 éq). Le milieu réactionnel a été chauffé à 65°C pendant 18 heures. Le méthanol a été évaporé et le résidu a été chromatographié sur gel de silice, éluant dichlorométhane/méthanol 95/5 avec 0.1% de triéthylamine. Le produit reste scotché sur la silice. La pâte obtenue a été cristallisée dans l'éther diéthylique, filtrée et séchée sous vide à 40°C. 0.11 g de 3-{[(5-méthyl-furan-2-yl)-(3-méthyl-oxetan-3-yl)-méthyl]-amino}-4-(2-oxo-2,3-dihydro-1H-benzoimidazol-4-ylamino)-cyclobut-3-ène-1,2-dione a été obtenu. Rendement = 18%. HPLC 94.8%, ES- [407].

RMN ¹H (DMSO-d6, 400 MHz) : 1.32 (s, 3H) ; 2.26 (s, 3H) ; 4.28-4.30 (m, 2H) ; 4.59 (d, J=6.2 Hz, 1H) ; 4.66 (d, J=6.2 Hz, 1H) ; 5.59 (d, J=8.0 Hz, 1H) ; 6.07 (m, 1H) ; 6.27 (m, 1H) ; 6.75-6.77 (m, 1H) ; 6.88-6.94 (m, 2H) ; 8.16 (s, 1H) ; 9.28 (s,1H) ; 10.72-10.75 (m, 2H).

### EXEMPLE 42

### Préparation du 2-hydroxy-3-(2-{[(5-méthyl-furan-2-yl)-(3-méthyl-oxetan-3-yl)-méthyl]-amino}-3,4-dioxo-cyclobut-1-enylamino)-benzonitrile

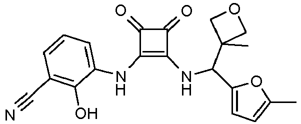

De manière analogue à l'EXEMPLE 1 (étapes 1 à 7), et à partir de 3-amino-2-hydroxybenzonitrile, 2-hydroxy-3-(2-{[(5-méthyl-furan-2-yl)-(3-méthyl-oxetan-3-yl)-méthyl]-amino}-3,4-dioxo-cyclobut-1-enylamino)-benzonitrile a été préparé. HPLC 96.13% ES- [393].

RMN ¹H (DMSO-d6, 400 MHz) : 1.32 (s, 3H) ; 2.26 (s, 3H) ; 4.28-4.30 (m, 1H) ; 4.57 (d, J=6.3 Hz, 1H) ; 4.64 (d, J=6.2 Hz, 1H) ; 5.63 (d, J=9.8 Hz, 1H) ; 6.02 (m, 1H) ; 6.26 (d, J=3.1 Hz, 1H) ; 7.00 (s, 1H) ; 7.30 (s, 1H) ; 8.03 (d, 1H) ; 9.85 (d, 1H) ; 9.50 (s, 1H).

### EXEMPLE 43

### Préparation du (R)-1-[3-(2-[{(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle

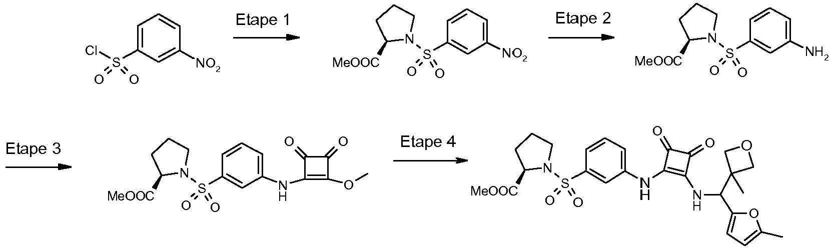

### Etape 1:

### (R)-1-(3-Nitro-benzènesulfonyl)-pyrrolidine-2-carboxylate de méthyle

A une solution de 2.56 g de chlorure de 3-nitrobenzène sulfonyle et de 1.91 g d'hydrochorure de (R)-pyrrolidine-2-carboxylate de méthyle dans 30 mL de dichlorométhane (30 mL) refroidie à 0°C ont été ajoutés goutte à goutte 3.38 mL de triéthylamine pendant 30 minutes. Le milieu réactionnel a été laissé à température ambiante et agité pendant 1 heure. Le milieu réactionnel a été dilué avec 50 mL de dichloromethane, lavé avec une solution hydrogénophosphate de sodium 1M (2 x 50 mL) et une solution saturée d'hydrogénocarbonate de sodium (50 mL). La phase organique a été séchée sur sulfate de magnésium et évaporée. 3.00 g de (R)-1-(3-nitro-benzènesulfonyl)-pyrrolidine-2-carboxylate de méthyle ont été obtenus et utilisés dans l'étape suivante sans purification.

### Etape 2:

### (R)-1-(3-Amino-4-chloro-benzènesulfonyl)-pyrrolidine-2-carboxylate de méthyle

Un mélange de 3.0 g de (R)-1-(3-nitro-benzènesulfonyl)-pyrrolidine-2-carboxylate de méthyle dans 40 mL de méthanol et en présence de 300 mg de palladium sur charbon à 10% (10% en masse) a été agité sous pression atmosphérique d'hydrogène pendant la nuit. Le milieu réactionnel a été filtré sur célite et lavé au méthanol. Le solvent a été évaporé. 2.80 g de (R)-1-(3-amino-4-chloro-benzènesulfonyl)-pyrrolidine-2-carboxylate de méthyle ont été obtenus et utilisés dans l'étape suivante sans purification. Rendement = 100%.

### Etape 3:

### (R)-1-[3-(2-Méthoxy-3,4-dioxo-cyclobut-1-enylamino)-benzènesulfonyl]-pyrrolidine-2-carboxylate de méthyle

Un mélange de 2.54 g de (R)-1-(3-amino-4-chloro-benzènesulfonyl)-pyrrolidine-2-carboxylate de méthyle et de 5.68 g de 3,4-diméthoxy-cyclobut-3-ène-1,2-dione dans 50 mL de méthanol a été chauffé à 60°C pendant la nuit. Le solvent a été évaporé et le résidu a été chromatographié sur gel de silice élué à l'heptane/acétate d'éthyle (4:1, 2:1 et acétate d'éthyle pur). 0.92 g de (R)-1-[3-(2-méthoxy-3,4-dioxo-cyclobut-1-enylamino)-benzènesulfonyl]-pyrrolidine-2-carboxylate de méthyle a été obtenu. Rendement = 23%.

### Etape 4:

### (R)-1-[3-(2-{[(5-Méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle

Un mélange de 408.4 mg de (R)-1-[3-(2-méthoxy-3,4-dioxo-cyclobut-1-enylamino)-benzènesulfonyl]-pyrrolidine-2-carboxylate de méthyle et de 350 mg de C-(5-méthyl-furan-2-yl)-C-(3-méthyl-oxetan-3-yl)-méthylamine dans 30 mL d'éthanol a été chauffé à 60°C pendant la nuit.. Le solvent a été évaporé et le résidu a été repris avec 50 mL d'acétate d'éthyle et lavé 2 fois avec une solution hydrogénophosphate de sodium 1M. La phase organique a été séchée sur sulfate de magnésium et évaporée. 432 mg de (R)-1-[3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle ont été obtenus. Rendement = 79%

### EXEMPLE 44

### Préparation du (S)-1-[4-chloro-3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle

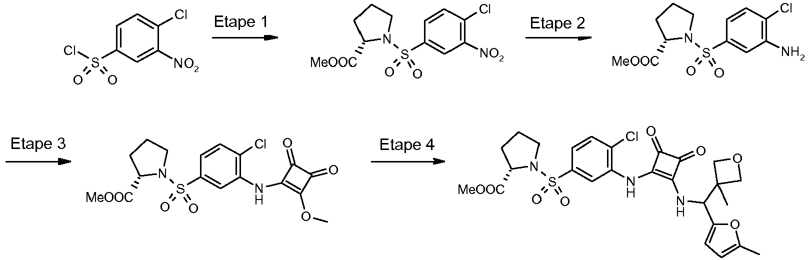

### Etape 1:

### (S)-1-(4-Chloro-3-nitro-benzènesulfonyl)-pyrrolidine-2-carboxylate de méthyle

A 0°C, une solution de 0.647 g (3.9 mmol) de chlorhydrate de (S)-pyrrolidine-2-carboxylate de méthyle dans 6 mL d'eau a été ajouté 0.83 g de carbonate de sodium (7.8 mmol, 2 éq) puis par portions 1.0 g (3.9 mmol) de 4-chloro-3-nitrobenzènesulfonyl chloride. Le milieu a été agité à 0°C pendant 4 heures. Le milieu réactionnel a été dilué avec de l'acétate d'éthyle et amené à pH5-6 avec HCl 2N. Après extraction, la phase aqueuse a été basifiée avec du bicarbonate de soude et extraite 2 fois à l'acétate d'éthyle. Les phases organiques ont été rassemblées, lavées avec une solution saturée de chlorure de sodium, séchées sur sulfate de magnésium et évaporées. 1.22 g (90%) de produit ont été obtenus. Rendement = 90%.

### Etape 2:

### (S)-1-(3-Amino-4-chloro-benzènesulfonyl)-pyrrolidine-2-carboxylate de méthyle

A une solution de 1.21 g de 4-chloro-N-N-diméthyl-3-nitro-benzènesulfonamide (3.47 mmol) dans 9 mL d'acétate d'éthyle ont été ajoutés par portions 5.56 g de chlorure d'étain dihydrate (24.63 mmol, 7 éq). Le milieu réactionnel a été chauffé à reflux pendant 3 heures, refroidit puis dilué avec 12 mL d'acétate d'éthyle et neutralisé avec 2.50 g de bicarbonate de sodium. Le milieu réactionnel a été filtré. Le solide a été lavé à l'acétate d'éthyle (2 x 15 mL) et le filtrat a été concentré à sec. Le résidu a été chromatographié sur gel de silice, élué au dichlorométhane/méthanol 50/50. 0.622 g de produit a été obtenu sous forme d'un solide beige. Rendement = 56%.

### Etape 3:

De manière analogue à l'EXEMPLE 44 (étape 3), (S)-1-[3-(2-méthoxy-3,4-dioxo-cyclobut-1-enylamino)-benzènesulfonyl]-pyrrolidine-2-carboxylate de méthyle a été préparé. Rendement = 32%.

### Etape 4:

De manière analogue à l'EXEMPLE 44 (étape 4), (S)-1-[4-chloro-3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle a été préparé. Rendement = 87%. HPLC 95.3%, ES- [576].

RMN ¹H (DMSO-d6, 400 MHz) : 1.34 (s, 3H) ; 1.55-1.70 (m, 1H) ; 1.75-1.93 (m, 2H) ; 1.95-2.10 (m, 1H) ; 2.27 (s, 3H) ; 3.20-3.33 (m, 1H) ; 3.35-3.50 (m, 1H) ; 3.67 (s, 1H) ; 4.29-4.32 (m, 2H) ; 4.45-4.55 (m, 1H) ; 4.59 (d, J=6.0 Hz, 1H) ; 4.65 (d, J=6.0 Hz, 1H) ; 5.65 (d, J=9.7 Hz, 1H) ; 6.09 (s, 1H) ; 6.30 (d, J=3.0 Hz, 1H) ; 7.50 (d, 1H) ; 7.75 (d, J=8.3 Hz, 1H) ; 8.00(d, 1H) ; 8.80 (d, 1H) ; 9.57 (s, 1H).

### EXEMPLE 45

### Préparation du (S)-1-[2,6-Difluoro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle

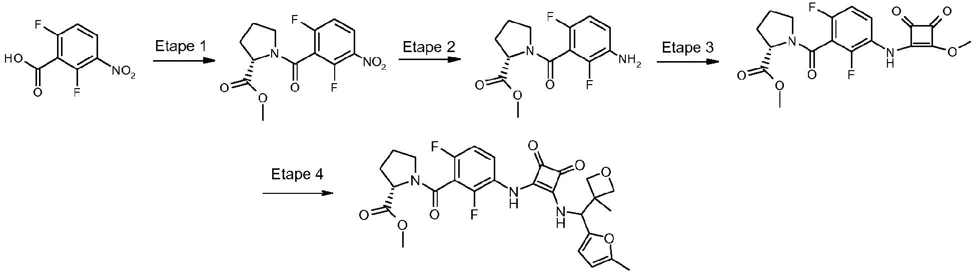

### Etape 1:

### (S)-1-(2,6-difluoro-3-nitro-benzoyl)-pyrrolidine-2-carboxylate de méthyle

Une solution de 1.82 g (8.94 mmol) d'acide 2,6-difluoro-3-nitro-benzoïque dans 36 mL de chlorure de thionyle a été chauffée au reflux pendant 3 heures et demie. Le milieu réactionnel a été concentré et a été coévaporé deux fois avec du toluène. Le résidu obtenu a été mis en solution dans 50 mL de dichlorométhane sous azote. 1.48 g (8.94 mmol) de chlorhydrate de (S)-pyrrolidine-2-carboxylate de méthyle a été rajouté et le milieu réactionnel a été refroidi à 0°C. 2.75 mL (19.67 mmol, 2.2 éq) de triéthylamine ont été alors rajoutés goutte à goutte. Après 22 heures, une solution aqueuse saturée d'hydrogénocarbonate de sodium a été ajouté. La phase organique a été à nouveau lavée avec une solution aqueuse saturée d'hydrogénocarbonate de sodium puis deux autres fois avec une solution aqueuse à 1 M d'acide chlorhydrique. La phase organique a été séchée sur sulfate de sodium anhydre, filtrée et concentrée. 2.52 g ont été obtenus. Rendement = 83%.

### Etape 2:

### (S)-1-(3-Amino-2,6-difluoro-benzoyl)-pyrrolidine-2-carboxylate de méthyle

A une solution de 2.52 g de (S)-1-(2,6-difluoro-3-nitro-benzoyl)-pyrrolidine-2-carboxylate de méthyle sous azote a été rajouté 0.71 g (28% en masse) de palladium sur charbon à 10%. Le milieu réactionnel a été agité sous atmosphère d'hydrogène pendant 2 heures. Le milieu réactionnel a été filtré sur célite et concentré. Le résidu a été utilisé tel quel dans l'étape suivante.

### Etape 3:

### (S)-1-[2,6-difluoro-3-(2-méthoxy-3,4-dioxo-cyclobut-1-enylamino)-benzoyl]-pyrrolidine-2-carboxylate de méthyle

Un mélange de (S)-1-(3-amino-2,6-difluoro-benzoyl)-pyrrolidine-2-carboxylate de méthyle (préparé ci-dessus) et de 4.20 g (29.55 mmol) de 3,4-diméthoxy-cyclobut-3-ène-1,2-dione en solution dans 50 mL de méthanol a été chauffé à 60°C pendant une heure puis agité à température ambiante pendant 4 jours. Le milieu réactionnel a été concentré et le résidu (6.29 g) a été chromatographié sur gel de silice (colonne prépackée de 300 g, élué à l'heptane/acétate d'éthyle de 70% à 100% d'acétate d'éthyle). 2.30 g de (S)-1-[2,6-difluoro-3-(2-méthoxy-3,4-dioxo-cyclobut-1-enylamino)-benzoyl]-pyrrolidine-2-carboxylate de méthyle ont été obtenus. Rendement = 79 %.

### Etape 4 :

### (S)-1-[2,6-difluoro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle

Un mélange de 500 mg (1.27 mmol) de (S)-1-[2,6-difluoro-3-(2-méthoxy-3,4-dioxo-cyclobut-1-enylamino)-benzoyl]-pyrrolidine-2-carboxylate de méthyle et de 344 mg (1.90 mmol, 1.5 éq) de C-(5-méthyl-furan-2-yl)-C-(3-méthyl-oxetan-3-yl)-méthylamine dans 25 mL de méthanol a été chauffé à 60°C puis a été agité à température ambiante pendant la nuit. Le milieu réactionnel a été concentré. Le résidu a été repris avec l'acétate d'éthyle et a été lavé deux fois avec une solution aqueuse de dihydrogénophophate de sodium 1M. La phase organique a été séchée sur sulfate de sodium anhydre, filtrée et concentrée. 0.64 g de (S)-1-[2,6-difluoro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle a été obtenu. Rendement = 93%, Tf= 122°C.

RMN ¹H (DMSO-d6, 400 MHz) : (présence de deux conformères, description du conformère principal) : 1.33 (s, 3H); 1.80-2.10 (m, 3H); 2.15-2.35 (m, 4H); 3.30-3.50 (m, 2H); 3.68 (s, 3H); 4.28-4.31 (m, 2H); 4.53-4.58 (m, 2H); 4.64 (d, J=6.2 Hz, 1H); 5.61 (d, J=9.7 Hz, 1H); 6.07 (d, J=2.0 Hz, 1H); 6.28 (d, J=2.9 Hz, 1H); 7.28 (t, J=8.4 Hz, 1H); 7.95-8.15 (m, 1H); 8.55-8.65 (m, 1H); 9.68 (m, 1H).

### EXEMPLE 46

### Préparation du (S)-1-[2-chloro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle

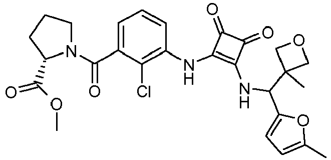

### Etape 1:

De manière analogue à l'EXEMPLE 46 (étape 1), (S)-1-(2-chloro-3-nitro-benzoyl)-pyrrolidine-2-carboxylate de méthyle a été préparé.

### Etape 2:

Une solution de 2.64 g de (S)-1-(2-chloro-3-nitro-benzoyl)-pyrrolidine-2-carboxylate de méthyle et de 10.0 g de chlorure d'étain dans 60 mL de méthanol a été chauffé à reflux pendant 1 heure. Le milieu réactionnel a été filtré et le filtrat a été concentré de la moitié de son volume et extrait à l'acétate d'éthyle (3 x 50 mL). Les phases organiques ont été rassemblées, séchées sur sulfate de magnésium et évaporées. 2.20 g de (S)-1-(3-amino-2-chloro-benzoyl)-pyrrolidine-2-carboxylate de méthyle ont été obtenus. Rendement = 92%.

### Etapes 3:

De manière analogue à l'EXEMPLE 46 (étape 3), (S)-1-[2-chloro-3-(2-méthoxy-3,4-dioxo-cyclobut-1-enylamino)-benzoyl]-pyrrolidine-2-carboxylate de méthyle a été préparé.

### Etapes 4:

De manière analogue à l'EXEMPLE 46 (étape 4), (S)-1-[2-chloro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle a été préparé. Rendement = 83%. Tf = 148-158°C,
LC/MS: 99.47% [541].

RMN ¹H (DMSO-d6, 400 MHz) : (présence de deux conformères, description du conformère principal) : 1.35 (s, 3H); 1.80-2.05 (m, 3H); 2.15-2.35 (m, 4H); 3.20-3.30 (m, 2H); 3.70 (s, 3H); 4.30 (t, J=6.2 Hz, 2H); 4.49-4.52 (m, 1H); 4.58 (d, J=6.2 Hz, 1H); 4.65 (d, J=6.2 Hz, 1H); 5.65 (d, J= .7 Hz, 1H); 6.08 (dd, J=3.0-0.9 Hz, 1H); 6.29 (d, J=3.0 Hz, 1H); 7.06 (d, J=7.4 Hz, 1H); 7.43 (t, J=7.9 Hz, 1H); 7.67 (d, J=8.2 Hz, 1H); 8.77 (d, J=9.9 Hz, 1H); 9.46 (s, 1H).

### EXEMPLE 47

### Préparation du (R)-1-[2-hydroxy-3-(2-{[(R)-(5-méthyl-furan-2-yl)-(3-méthyl-oxetan-3-yl)-méthyl]-amino}-3,4-dioxo-cyclobut-1-énylamino)-benzoyl]-pyrrolidine-2-carboxylate de méthyle :

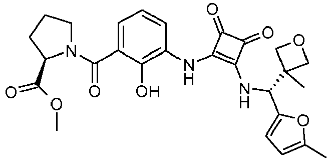

De manière analogue à l'EXEMPLE 7 (étape 1 à 4), (R)-1-[2-hydroxy-3-(2-{[(R)-(5-méthyl-furan-2-yl)-(3-méthyl-oxetan-3-yl)-méthyl]-amino}-3,4-dioxo-cyclobut-1-énylamino)-benzoyl]-pyrrolidine-2-carboxylate de méthyle a été préparé. Tf = 140°C, HPLC : 99.31%, ES+ [524]

RMN ¹H (DMSO-d6, 400 MHz) : présence de deux conformères, description du conformère majoritaire à environ 80 % : 1.32 (s, 3H), 1.80-2.00 (m, 3H), 2.15-2.35 (m, 4H), 3.55-3.75 (m, 5H), 4.27-4.30 (m, 2H), 4.50-4.60 (m, 2H), 4.65 (d, J = 6.2 Hz, 1H), 5.64 (d, J = 9.8 Hz, 1H), 6.06 (dd, J = 3.1 Hz, J = 1.0 Hz, 1H), 6.26 (d, J = 3.1 Hz, 1H), 6.93 (t, J = 7.9 Hz, 1H), 7.14 (dl, J = 7.52 Hz, 1H), 7.86 (dl, J = 7.8 Hz, 1H), 8.84 (dl, J = 9.72 Hz, 1H), 9.49 (sl, 1H).

### EXEMPLE 48

### Préparation du 2-hydroxy-N-méthyl-3-(2-{[(R)-(5-méthyl-furan-2-yl)-(3-méthyl-oxetan-3-yl)-méthyl]-amino}-3,4-dioxo-cyclobut-1-énylamino)-N-(2,2,2-trifluoro-éthyl)-benzamide :

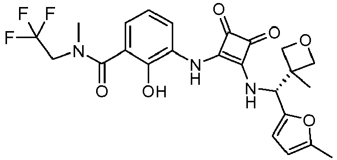

De manière analogue à l'EXEMPLE 7 (étape 1 à 4), 2-hydroxy-N-méthyl-3-(2-{[(R)-(5-méthyl-furan-2-yl)-(3-méthyl-oxetan-3-yl)-méthyl]-amino}-3,4-dioxo-cyclobut-1-énylamino)-N-(2,2,2-trifluoro-éthyl)-benzamide_a été préparé. Tf = 123°C, HPLC : 99.53%, ES+ [508]

RMN ¹H (DMSO-d6, 400 MHz) : 1.32 (s, 3H), 2.26 (s, 3H), 2.85-3.15 (m, 3H), 3.90-4.45 (m, 4H), 4.58 (d, J = 6.2 Hz, 1H), 4.65 (d, J = 6.2 Hz, 1H), 5.64 (d, J = 9.8 Hz, 1H), 6.06 (dd, J = 3.0, J = 3.0 Hz, 1H), 6.26 (d, J = 3.1 Hz, 1H), 6.84 (dd, J = 7.6 Hz, J = 1.5 Hz, 1H), 6.94 (t, J = 7.8 Hz, 1H), 7.76 (dd, J = 8.0 Hz, J = 1.1 Hz, 1H), 8.79 (d, J = 9.8 Hz, 1H), 9.45-9.55 (m, 1H), 9.75-10.00 (m, 1H).

### EXEMPLE 49

### Préparation du 2-Hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide

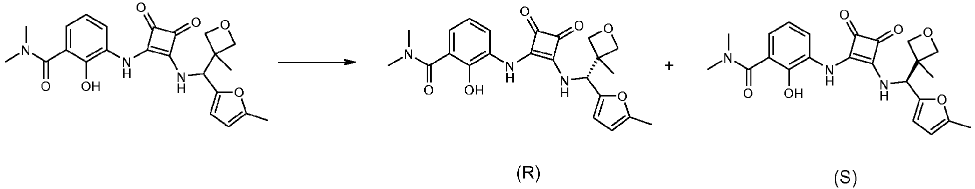

A partir du 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzamide obtenu à l'exemple 1, une séparation chirale a été effectuée sur 130 mg de solubilisés dans 4 mL d'éthanol.

La séparation s'effectue sur la colonne Chiralpack IA éluée avec heptane/éthanol (90/10) sur 80 minutes avec un débit de 12 mL/min. Les injections ont été de 800 µL (15-20 mg). Deux fractions de 23 mg de chaque énantiomère ont été obtenues :
- énantiomère (R)- : 27.1 min , LC/MS: 99.8% [439],
- énantiomère (S)- : 33.7 min , LC/MS: 99.5% [439].

RMN ¹H (DMSO-d6, 400 MHz) énantiomère (R)- ou (S)- : 1.32 (s, 3H) ; 2.26 (s, 3H) ; 2.94 (s, 6H) ; 4.29 (dd, *J*=6.2 Hz, 2H) ; 4.6 (dd, *J*=28.8 Hz, 2H) ; 5.6 (d, *J*=9.7 Hz, 1H) ; 6.06 (d, *J*=2.1 Hz, 1H), 6.25 (d, *J*=3.1 Hz, 1H) ; 6.88 (m, 2H) ; 7.76 (q, *J*=9.5 Hz, 1H) ; 8.83 (d, *J*=9.8 Hz, 1H) ; 9.46 (s, 1H).

### EXEMPLE 50

### Préparation du 2-hydroxy-N,N-diméthyl-3-(2-{[((R)-5-méthyl-furan-2-yl)-(3-méthyl-oxetan-3-yl)-méthyl]-amino}-3,4-dioxo-cyclobut-1-enylamino)-benzamide

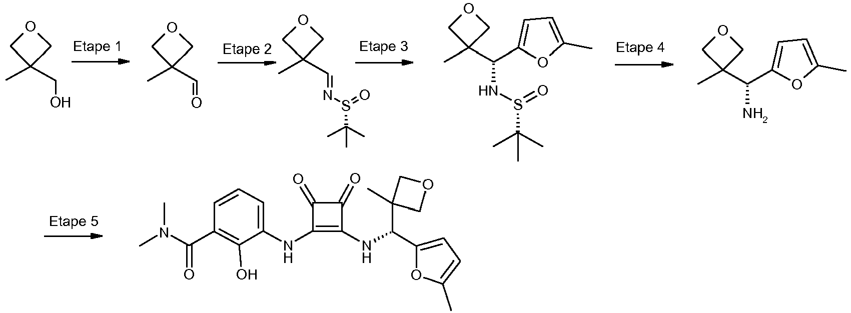

### Etape 1 (Méthode a)

### 3-Méthyl-oxetane-3-carbaldéhyde

6.8 mL (80 mmol, 1.6 éq) de chlorure d'oxalyle ont été ajoutés goutte à goutte sur une solution de 135 mL de dichlorométhane refroidie à -78 °C (bain acétone/carboglace) suivi de 12 mL de diméthylsulfoxide (0.17 mol, 3.3 éq) goutte à goutte (attention, fort dégagement de gaz toxique de monoxide de carbone). Le milieu réactionnel a été agité 15 minutes à -78°C. Une solution de 5.0 mL (50 mmol, 1 éq) de (3-méthyloxetan-3-yl)-méthanol dans 110 mL de dichlorométhane a été ajoutée goutte à goutte en 30 minutes à -78 °C (la température du milieu réactionnel atteint -55°C) et le milieu réactionnel a été agité à -70°C pendant 1 heure et demie. 44 mL de *N,N*-diisopropyléthylamine (0.25 mol, 5.0 éq) ont été ajoutés goutte à goutte (la température du milieu réactionnel atteint -25°C). Le bain refroidissant a été enlevé et le milieu réactionnel a été doucement ramené à température ambiante et agité pendant 30 minutes (rendement similaire en laissant agiter toute la nuit). La réaction a été arrêtée par addition de 150 mL d'une solution de bisulfate de sodium à 10% sous une forte agitation et en s'assurant que le pH de la phase aqueuse soit inférieur à 5. Le milieu réactionnel a été versé dans 200 mL de dichloromethane et décanté. 100 mL d'une solution saturée de chlorure de sodium ont été ajoutés à la phase aqueuse puis celle-ci a été extraite au dichlorométhane (3 x 100 mL). Les phases organiques ont été rassemblées, séchées sur sulfate de magnésium, filtrées et évaporées (260 mm Hg, bain marie à 40°C). 41.5 g de produit brut ont été obtenus. (Il a été important de ne pas trop concentrer car l'aldéhyde formé a été très volatile.

### Etape 1 (Méthode b)

### 3-Méthyl-oxetane-3-carbaldéhyde

Une solution de (3-méthyloxetan-3-yl)-méthanol (20.4 g, 0.2 mol) dans 200 mL de dichlorométhane a été ajoutée goutte à goutte sur un mélange de pyridinium chlorochromate (68.9 g, 0.32 mol) et de célite (20.4 g) dans 700 mL de dichlorométhane. Le milieu réactionnel a été agité à température ambiante pendant 5 heures. Le milieu réactionnel a été filtré sur 250 g de silice et élués au dichlorométhane. Les phases organiques ont été rassemblées et évaporées à 30°C sous ~500 mBar puis 3 fois 30 secondes 10°C sous ~30 mBar. 12.42 g de 3-méthyl-oxetane-3-carbaldéhyde ont été obtenus sous forme d'une huile volatile vert pâle. Rendement = 62%.

### Etape 2:

### (R)-2-Méthyl-propane-2-sulfinic acid 1-(3-méthyl-oxetan-3-yl)-meth-(E)-ylideneamide

18 mL d'éthoxide de Titanium (IV) (86 mmol, 2 éq) ont été ajoutés sur 46 g de 3-méthyl-oxetane-3-carbaldéhyde à 9.7% (45 mmol, 1éq) refoidis à 5°C. Le milieu réactionnel a été agité pendant 10 minutes. 5.73 g de (R)-(+)-2-méthyl-2-propanesulfinamide (47.3 mmol, 1.1 éq) ont été ajoutés et le milieu réactionnel a été agité pendant 16 heures. 5.54 g de sulfate de sodium décahydrate (17.2 mmol) ont été broyés en poudre et ajoutés au milieu réactionnel. Le milieu réactionnel a été agité vigoureusement pendant 20 minutes et 400 mL d'acétate d'éthyle ont été ajoutés et l'agitation a été maintenue pendant 30 minutes. 100 mL de celite et 20 g de sulphate de sodium ont été ajoutés et le milieu réactionnel a été agité pendant 10 minutes. La suspension a été filtrée sur 100 mL de celite et le gateau a été lave avec de l'acétate d'éthyle (2 x 100 mL). Le filtrat a été évaporé sous pression réduite pour donner 19.2 g d'une huile brune qui a été purifiée sur colonne de silice éluée à l'heptane / acétate d'éthyle. 8.38 g de (R)-2-Méthyl-propane-2-sulfinic acid 1-(3-méthyl-oxetan-3-yl)-meth-(E)-ylideneamide ont été obtenus sous forme d'un solide jaune pâle. Rendement = 90%, 81% sur 2 étapes).

### Etape 3:

### (R)- 2-Méthyl-propane-2-sulfinic acid [(R)-(5-méthyl-furan-2-yl)-(3-méthyl-oxetan-3-yl)-méthyl]-amide

23 mL de butyllithium à 2.7 M dans l'hexane (62 mmol, 1.4 éq) ont été ajoutés goutte à goutte en 5 minutes sur une solution de 6.8 mL de 2-méthylfurane (75 mmol, 1.7 éq) dans 38 mL d'éther diéthylique refroidie à 5°C. Le bain de glace a été enlevé et la solution orange a été agitée pendant 1 heure et demie à température ambiante. Le milieu réactionnel a été refroidi à 5°C et 16.0 g de magnesium bromide ethyl etherate solide (62.1 mmol, 1.4 éq) ont été ajoutés. Le bain de glace a été enlevé et la suspension obtenue a été agitée à température ambiante pendant 45 minutes et séparée en 2 phases une fois l'agitation arrêtée (phase supérieure Jaune pâle et phase inférieure marron avec quelques cristaux blancs insolubles). Dans un tricol de 1 L équipé d'un système d'agitation mécanique ont été introduits 9.0 g de sulfinylimine (44.3 mmol, 1 éq) et 240 mL de toluène. Le mélange a été refroidi à -70°C (bain acétone-carboglace) et le réactif de Grignard a été transféré sur la sulfinylimine via une canule en 30 minutes.

Le milieu réactionnel a été laissé remonter à température ambiante lentement et agité pendant 16 heures. Le milieu réactionnel a été refroidi à 5°C et 100 mL d'une solution saturée de chlorure d'ammonium ont été ajoutés pour hydrolyser le réactif de Grignard. Le milieu réactionnel a été laissé remonter à température ambiante et 10 mL d'eau ont été ajoutés pour dissoudre les sels présents. Le milieu réactionnel a été décanté et la phase aqueuse a été extraite avec *t*-Butyl méthyléther (100 mL). Les phases organiques ont été rassemblées, séchées sur sulphate de sodium, filtrées et évaporées. 13.5 g ont été obtenus sous forme d'huile orange. L'analyse du produit brut par RMN ¹H montre qu'il y a moins de 5% de diastéréoisomère non désiré. La purification sur colonne de silice éluée à l'heptane / acétate d'éthyle permet d'obtenir 11.5 g de (R)-2-méthyl-propane-2-sulfinic acid [(R)-(5-méthyl-furan-2-yl)-(3-méthyl-oxetan-3-yl)-méthyl]-amide sous forme d'une huile orange qui cristallise plus tard. Rendement = 91%).

### Etape 4:

### C-[(R)-C-(5-Méthyl-furan-2-yl)-C-(3-méthyl-oxetan-3-yl)]-méthylamine

Dans un tricol de 1 L équipé d'un système d'agitation mécanique ont été introduits 14.5 g d'amine protégée (51.0 mmol, 1 éq) et 255 mL de cyclopentyl méthyl ether (CPME). Le mélange a été refroidi à -40°C (bain acétone-carboglace) et 78 mL d'une solution de chlorure d'hydrogène à 1.2M dans CPME (94 mmol, 1.85 éq) ont été ajoutés goutte à goutte via une canule en 30 minutes.

Le milieu réactionnel a été agité pendant 1 heure et demie (température montée à 10°C). Un contrôle CCM (acetate d'éthyle / heptane 2/1) a montré qu'il restait de la matière première. Le milieu réactionnel a été filtré sur célite (300 mL) et le gâteau a été lavé avec CPME (4 x 50 mL). Le réceptacle a été changé et le gâteau a été lavé au méthanol (5 x 100 mL). Le filtrat a été évaporé pour donner 15 g d'une pâte marron clair. L'analyse du produit brut par RMN ¹H (CD₃OD) montre qu'il y a 10-15% de produit secondaire provenant de l'ouverture de l'oxétane (2 diastéréoisomères). 200 mL d'isopropanol ont été ajoutés, le solide a été cassé en petits morceaux et la suspension a été partiellement concentrée pour enlever 75 mL de solvent. La suspension a été alors agitée à température ambiante pendant 3 heures et filtrée. Le gâteau a été lavé à l' isopropanol (2 x 20 mL). 8.9 g de solide humide blanc ont été obtenus sous forme de sel d'hydrochlorure, déterminé par analyse RMN ¹H (CD₃OD). L'amine libre a été obtenue en agitant le sel d'hydrochlorure dans une solution de soude 2M (80 mL) et de *t*-butyl méthyléther (100 mL) pendant 30 minutes. Le milieu hétérogène a été décanté et la phase aqueuse a été extraite au *t*-butyl méthyléther (3 x 100 mL). Les phases organiques ont été rassemblées, séchées sur sulphate de sodium, filtrées et évaporées. Le résidu a été séché sous vide (ca. 1 mm Hg) pendant 1 heure. 5.22 g de produit ont été obtenus sous forme d'une huile jaune. Rendement = 56%). [α]_{D} -12.8 (c = 1.9, CHCl₃).

### Etape 5:

### 2-Hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide

471 mg (2.6 mmol, 1.3 éq) de C-(R)-(5-méthyl-furan-2-yl)-C-(3-méthyl-oxetan-3-yl)-méthylamine ont été ajoutés sur 609 mg (2.0 mmol, 1 éq) de 3-(2-éthoxy-3,4-dioxo-cyclobut-1-enylamino)-2-hydroxy-N,N-diméthyl-benzamide solubilisés à chaud dans 40 mL de méthanol. Le milieu réactionnel a été chauffé à 50°C pendant 17 heures. Le méthanol a été évaporé et le résidu (huile verte) a été chromatographié sur gel de silice (colonne puriFlash IR-50SI/80G, Spot II) élué au dichlorométhane/méthanol (97/3). Le solide amorphe a été repris avec de l'éther diéthylique pour donner une poudre Jaune qui a été recristallisée dans le n-propanol. 519 mg de 2-hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide ont été obtenus. Rendement = 70%.

### EXEMPLE 51 :

### Préparation du {[2-hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-méthyl-amino}-acétate de méthyle

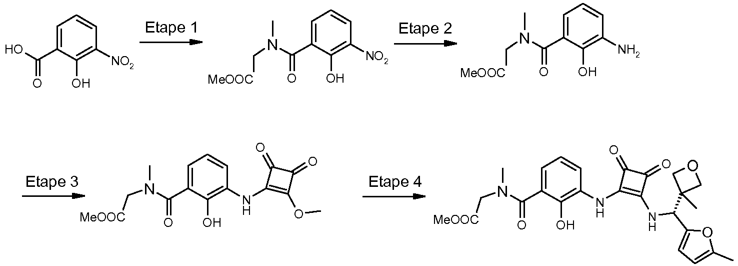

### Etape 1:

### [(2-Hydroxy-3-nitro-benzoyl)-méthyl-amino]-acétate de méthyle

A un mélange de 25.46 g (54.6 mmol, 2éq) de bromo-tris-pyrrolidino-phosphonium hexafluorophosphate et de 5.0 g (27.30 m mol, 1 éq) d'acide 3-nitrosalicylique dans 75.00 ml de dichlorométhane sous azote ont été rajoutés 18.8 ml (109.2 mmol, 4 éq) de N,N-diisopropylamine puis 7.62 g (54.6 mmol, 2 éq) de chlorhydrate de méthylamino-acétate de méthyle. Le milieu réactionnel a été agité à température ambiante pendant 22 heures. Le milieu réactionnel a été lavé trois fois avec une solution aqueuse à 1 M d'acide chlorhydrique. La phase organique a été séchée sur sulfate de sodium anhydre, filtrée et concentrée. Le résidu (30 g) a été chromatographié sur gel de silice (colonne prépackée de 800 g, éluant heptane/acétate d'éthyle, de 40 à 100% d'acétate d'éthyle, 200 ml/min).

5.85 g de [(2-hydroxy-3-nitro-benzoyl)-méthyl-amino]-acétate de méthyle ont été obtenus sous forme d'une huile jaune. Rendement = 79.88 %.

### Etape 2:

### [(3-Amino-2-hydroxy-benzoyl)-méthyl-amino]-acétate de méthyle

Une solution de 5.78 g (21.5 mmol, 1éq) de [(2-hydroxy-3-nitro-benzoyl)-méthyl-amino]-acétate de méthyle dans 75 mL de méthanol en présence de 0.52 g (10% en masse) de Pd/C 10% a été agité sous pression atmosphérique d'hydrogène pendant 2 heures et demie. Le milieu réactionnel a été filtré sur célite et le filtrat a été évaporé. Le résidu (4.78 g) a été chromatographié sur gel de silice (colonne prépackée de 300 g, éluant heptane/acétate d'éthyle, de 40 à 90% en acétate d'éthyle, 120 ml/min). 3.92 g de [(3-amino-2-hydroxy-benzoyl)-méthyl-amino]-acétate de méthyle ont été obtenus sous forme d'un huile jaune. Rendement = 76%.

### Etape 3:

### {[2-Hydroxy-3-(2-méthoxy-3,4-dioxo-cyclobut-1-enylamino)-benzoyl]-méthyl-amino}-acétate de méthyle

Un mélange de 3.92 g (16.4 mmol, 1éq) de [(3-amino-2-hydroxy-benzoyl)-méthyl-amino]-acétate de méthyle et de 4.68 g (32.9 mmol, 1éq) de 3.4-diméthoxy-3-cyclobutène-1.2-dione a été agité à température ambiante pendant 24 heures. Le solvant a été évaporé et le résidu a été chromatographié sur gel de silice (colonne prépackée de 200 g, éluant heptane/acétate d'éthyle 20/80 puis 0/100). 3.49 g de {[2-hydroxy-3-(2-méthoxy-3,4-dioxo-cyclobut-1-enylamino)-benzoyl]-méthyl-amino}-acétate de méthyle ont été obtenus sous forme d'un solide blanc. Rendement = 61%.

### Etape 4:

### {[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-méthyl-amino}-acétate de méthyle

Un mélange de 371 mg (2.07 mmol, 1.2 éq) de C-(R)-(5-méthyl-furan-2-yl)-C-(3-méthyl-oxetan-3-yl)-méthylamine (préparée dans l'EXEMPLE 50, étapes 1 à 4) et de 600 mg (1.7 mmol, 1 éq) de {[2-hydroxy-3-(2-méthoxy-3,4-dioxo-cyclobut-1-enylamino)-benzoyl]-méthyl-amino}-acétate de méthyle dans 24 mL de méthanol a été chauffé à 60°C pendant 16 heures. Le méthanol a été évaporé et le résidu a été chromatographié sur gel de silice, éluant dichlorométhane/acétate d'éthyle (75/25). La pâte obtenue a été cristallisée dans l'éther diéthylique et l'heptane et séchée sous vide à 40°C. 660 mg de {[2-hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-méthyl-amino}-acétate de méthyle ont été obtenus sous forme d'un solide jaune pâle. Rendement = 76%.

RMN ¹H (DMSO-d6, 400 MHz) : 1.33 (s, 3H) ; 2.26 (s, 3H) ; 2.96 (se,3H) ; 3.67 (s,3H) ; 4.80 (se, 1H) ; 4.28 (d, j=6.2Hz, 2H) ; 4.29 (d, j=6.2Hz, 1H) ; 4.88 (d, j=6.2Hz, 1H) ; 4.65 (d, j=6.2Hz, 1H) ; 5.65 (d, j=9.8Hz, 1H) ; 6.06 (m, 1H) ; 6.26 (m, 1H) ; 6.85 (se,1H) ; 6.88-6.93 (m, 1H) ; 7.78 (d, j=7.7Hz, 1H) ; 8.83 (d, j=9.8Hz, 1H) ; 9.46 (s, 1H) ; 9.87 (se, 1H).

### EXEMPLE 52 :

### Préparation du 6-chloro-2-hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide

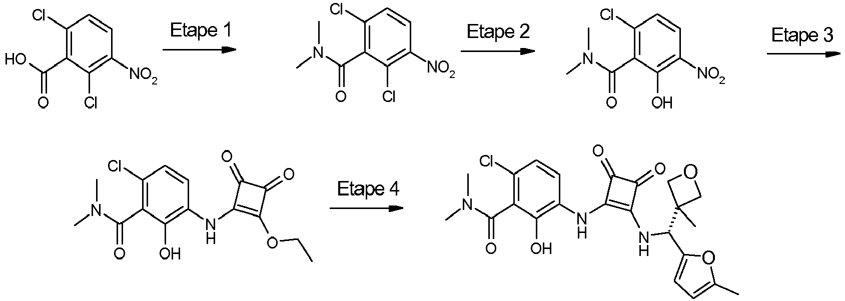

### Etape 1:

### 2,6-Dichloro-N,N-diméthyl-3-nitro-benzamide

Une solution de 10.0 g (42.4 mmol, 1éq) d'acide 2.6-dichloro-3-nitrobenzoïque dans 50 ml de chlorure de thionyle a été chauffée à reflux pendant 2 heures. Le milieu réactionnel a été concentré et coévaporé avec du toluène. Le résidu a été repris dans 35 ml de tétrahydrofuranne puis 48 ml d'une solution de diméthylamime dans le tétrahydrofuranne a été ajoutée goutte à goutte. Après 20 minutes d'agitation à température ambiante, de l'eau a été rajoutée ainsi que de l'acétate d'éthyle. La phase organique a été lavée à l'eau, séchée sur sulfate de sodium anhydre, filtrée et concentrée. 11.36 g de 2,6-dichloro-N,N-diméthyl-3-nitro-benzamide ont été obtenus sous forme d'une huile jaune. Rendement quantitatif.

### Etape 2:

**6-Chloro-2-hydroxy-N,N-diméthyl-3-nitro-benzamide**A une suspension de 7.16 g (179.01 mmol; 4.3 éq) d'hydrure de sodium dans 250 ml de tétrahydrofuranne refroidie à 0°C ont été rajoutés 3.2 ml (177.6 mmol, 4.2 éq) d'eau et 11.04 g (41.96 mmol; 1.0 éq) de 2,6-dichloro-N,N-diméthyl-3-nitro-benzamide (41.96 mmol; 1.00 eq.) en solution dans 130.00 ml de tétrahydrofuranne. Après 10 minutes, le milieu réactionnel a été agité à température ambiante pendant 19 heures. Le milieu réactionnel a été hydrolysé avec une solution aqueuse d'acide chlorhydrique IN et extrait à l'acétate d'éthyle. La phase organique a été lavée avec une solution aqueuse d'acide chlorhydrique IN, séchée sur sulfate de sodium anhydre, filtrée et concentrée. Le résidu (11.82 g) a été chromatographié sur gel de silice (colonne prépackée de 300 g, éluant heptane/acétate d'éthyle, de 40 à 80% en acétate d'éthyle, 150 ml/min). 6.10 g de 6-chloro-2-hydroxy-N,N-dimethyl-3-nitro-benzamide ont été obtenus sous forme d'un solide jaune. Rendement = 59%

### Etape 3:

### 6-Chloro-3-(2-éthoxy-3,4-dioxo-cyclobut-1-enylamino)-2-hydroxy-N,N-diméthyl-benzamide

Une solution de 5.96 g (24.4 mmol, 1éq) de 6-chloro-2-hydroxy-N,N-diméthyl-3-nitro-benzamide dans 100 mL de méthanol en présence de 0.58 g d'oxyde de platinium hydraté a été agité sous pression atmosphérique d'hydrogène pendant 3 heures. Le milieu réactionnel a été filtré sur célite et le filtrat a été concentré. La solution obtenue a été ajoutée goutte à goutte sur 8.0 g (48.8 mmol, 2 éq) de 3.4-diéthoxy-3-cyclobutène-1.2-dione en solution dans 50 mL de méthanol. Le milieu réactionnel a été agité à température ambiante pendant 18 heures. Le solvant a été évaporé et le résidu a été chromatographié sur gel de silice (colonne prépackée de 300 g, éluant heptane/acétone, de 50 à 100% d'acétone). 4.42 g de 6-chloro-3-(2-éthoxy-3,4-dioxo-cyclobut-1-enylamino)-2-hydroxy-N,N-diméthyl-benzamide ont été obtenus sous forme d'un solide beige. Rendement = 54%

### Etape 4:

### 6-Chloro-2-hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide

Un mélange de 412 mg (2.27 mmol, 1.1 éq) de C-(R)-(5-méthyl-furan-2-yl)-C-(3-méthyl-oxetan-3-yl)-méthylamine (préparée dans l'EXEMPLE 50, étapes 1 à 4) et de 700 mg (2.07 mmol, 1 éq) de 6-chloro-3-(2-éthoxy-3,4-dioxo-cyclobut-1-enylamino)-2-hydroxy-N,N-diméthyl-benzamide dans 45 mL de méthanol a été agité à température ambiante pendant 3 jours. Le méthanol a été évaporé et le résidu a été repris avec de l'acétate d'éthyle et lavé

avec une solution aqueuse de dihydrogénophosphate de sodium 1M. La phase organique a été séchée sur sulfate de sodium anhydre, filtrée et concentrée. Le résidu (0.90 g) a été chromatographié sur gel de silice (colonne prépackée de 80 g, éluant dichlorométhane/méthanol, de 0 à 10% en méthanol. 520 mg de 6-chloro-2-hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide ont été obtenus sous forme d'un solide ocre. Rendement = 53%. Tf = 165-167°C.

RMN ¹H (DMSO-d6, 400 MHz) : 1.31 (s, 3H), 2.21 (s, 3H), 2.79 (s, 3H), 3.00 (s, 3H), 4.28 (d, J = 6.1 Hz, 2H), 4.55-4.58 (m, 1H), 4.64 (d, J = 6.2 Hz, 1H), 5.64 (d, J = 9.6 Hz, 1H), 6.05 (m, 1H), 6.24 (m, 1H), 6.85-7.05 (m, 1H), 7.69-7.74 (m, 1H), 8.75-8.90 (m, 1H), 9.35-9.60 (m, 1H), 9.90-10.30 (m, 1H)

### EXEMPLE 53 :

### Préparation du 3-[4-chloro-2-hydroxy-3-(4-méthyl-pipérazine-1-sulfonyl)-phénylamino]-4-{[(R)-(5-méthyl-furan-2-yl)-(3-méthyl-oxetan-3-yl)-méthyl]-amino}-cyclobut-3-ene-1,2-dione

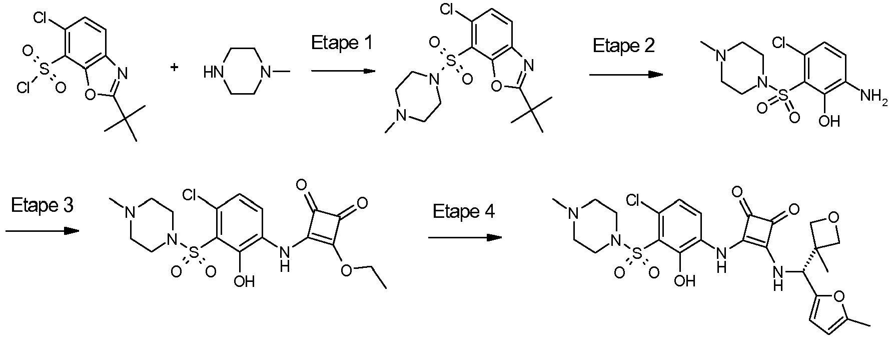

### Etape 1 :

### 2-tert-Butyl-6-chloro-7-(4-méthyl-pipérazine-1-sulfonyl)-benzooxazole

1.62 ml (11.68 mmol; 1.2 éq) de triéthylamine suivis de 1.20 ml (10.71 mmol; 1.1 éq) de 1-méthylpipérazine ont été ajoutés sur une solution de 3.0 g (9.73 mmol; 1.0 éq) de chlorure de 2-tert-butyl-6-chloro-benzooxazole-7-sulfonyle dans 45 ml de tétrahydrofuranne. Le milieu réactionnel a été agité à température ambiante pendant 2 heures. De l'eau a été rajoutée et le mileu réactionnel a été extrait à l'acétate d'éthyle. Les phases organiques ont été réunies, séchées sur sulfate de magnésium, filtrées et évaporées. 3.57 g de 2-tert-butyl-6-chloro-7-(4-méthyl-pipérazine-1-sulfonyl)-benzooxazole ont été obtenus sous forme d'une mousse collante brune. Rendement = 98%

### Etape 2 :

### 6-Amino-3-chloro-2-(4-méthyl-pipérazine-1-sulfonyl)-phénol

4.27 ml (0.08 mol; 1.20 V) d'acide sulfurique dilué dans 4.3 ml d'eau ont été ajoutés goutte à goutte sur 3.56 g de 2-tert-butyl-6-chloro-7-(4-méthyl-pipérazine-1-sulfonyl)-benzooxazole (0.01 mol; 1.0 éq) en solution dans 15 ml de 1,4-dioxanne. Le milieu réactionnel a été chauffé à reflux pendant 6 heures et demie. Le milieu réactionnel a été concentré et de la soude IN a été ajoutés (jusqu'à pH 7). La solution a été extraite au dichlorométhane. Les phases organiques ont été rassemblées, séchées sur sulfate de magnésium, filtrées et évaporées. Le résidu obtenu a été chromatographié sur gel de silice, éluant acétate d'éthyle/dichlorométhane 95/5. 2.0 g de 6-amino-3-chloro-2-(4-méthyl-pipérazine-1-sulfonyl)-phénol ont été obtenus sous forme d'une épaisse huile brune. Rendement = 68%.

### Etape 3 :

### 3-[4-Chloro-2-hydroxy-3-(4-méthyl-pipérazine-1-sulfonyl)-phénylamino]-4-éthoxy-cyclobut-3-ène-1,2-dione

Un mélange de 1.98 g (6.5 mmol, 1 éq) de 6-amino-3-chloro-2-(4-méthyl-pipérazine-1-sulfonyl)-phénol et de 2.20 g (48.8 mmol, 2 éq) de 3.4-diéthoxy-3-cyclobutène-1.2-dione en solution dans 20 mL d'éthanol. Le milieu réactionnel a été chauffé à 50°C pendant 16 heures. L'insoluble a été filtré, lavé à l'éthanol et séché sous vide à 45°C. 2.05 g de 3-[4-chloro-2-hydroxy-3-(4-méthyl-pipérazine-1-sulfonyl)-phénylamino]-4-éthoxy-cyclobut-3-ène-1,2-dione ont été obtenus sous forme d'un solide jaune. Rendement = 74%

### Etape 4 :

### 3-[4-Chloro-2-hydroxy-3-(4-méthyl-pipérazine-1-sulfonyl)-phénylamino]-4-{[(R)-(5-méthyl-furan-2-yl)-(3-méthyl-oxetan-3-yl)-méthyl]-amino}-cyclobut-3-ène-1,2-dione

Un mélange de 250 mg (1.4 mmol, 1.2 éq) de C-(R)-(5-méthyl-furan-2-yl)-C-(3-méthyl-oxetan-3-yl)-méthylamine (préparée dans l'EXEMPLE 50, étapes 1 à 4) et de 500 mg (1.16 mmol, 1 éq) de 3-[4-chloro-2-hydroxy-3-(4-méthyl-pipérazine-1-sulfonyl)-phénylamino]-4-éthoxy-cyclobut-3-ène-1,2-dione dans 20 mL de méthanol a été chauffé à 50°C pendant 16 heures. Le méthanol a été évaporé et le résidu a été chromatographié sur gel de silice élué au dichlorométhane/méthanol (98/2). Le résidu a été lavé avec une solution aqueuse de dihydrogénophosphate de sodium 1 M. La phase organique a été séchée sur sulfate de sodium anhydre, filtrée et évaporée. 450 mg de 3-[4-chloro-2-hydroxy-3-(4-méthyl-pipérazine-1-sulfonyl)-phénylamino]-4-{[(R)-(5-méthyl-furan-2-yl)-(3-méthyl-oxetan-3-yl)-méthyl]-amino}-cyclobut-3-ène-1,2-dione ont été obtenus sous forme d'un solide jaune sombre. Rendement = 68%.

RMN ¹H (DMSO-d6, 400 MHz) :1.31 (s, 3H) ; 2.26 (s, 3H) ; 2.42 (se, 3H) ; 2.70 (se, 4H) ; 3.37 (se, 4H) ; 4.28 (d, j=6.1Hz, 1H) ; 4.29 (d, j=6.1Hz, 1H) ; 4.57 (d, j=6.2Hz, 1H) ; 4.65 (d, j=6.2Hz, 1H) ; 5.62 (d, j=9.7Hz, 1H) ;6.06 (m, 1H) ; 6.24 (m, 1H) ; 6.70 (se, 1H) ; 7.85 (d, j=8.5Hz, 1H) ; 8.97 (d, j=9.8Hz, 1H) ; 9.60 (s, 1H) ; 10.00 (se, 1H).

### TESTS BIOLOGIQUES

### EXEMPLE 54 : Affinité in vitro

L'affinité *In Vitro* des composés de la présente invention pour les récepteurs CXCR1 et CXCR2 a été déterminée sur un test fonctionnel de type recrutement de la β-arrestine après activation du récepteur.

Il a été démontré que l'activation par CXCL28 du récepteur CXCR2 dans les cellules de la lignée PathHunter HEK293-CXCR2 ou du récepteur CXCR1 dans les cellules de la lignée U2OS h CXCR1 β-arrestine conduit au recrutement de la β-arrestine (Richardson et al. 2003 Role of the cytoplasmic tails of CXCR1 and CXCR2 in mediating leukocyte migration, activation, and regulation. J. Immunol. 170 : 2904.- 2911.)

Pour évalauer l'interaction directe du récepteur CXCR2 ou CXCR1 avec la β-arrestine 2, un test de recrutement β-arrestine 2 pour CXCR2 ou CXCR1 basée sur la complémentation de l'enzyme β-galactosidase (Olson KR, Eglen RM. Beta galactosidase complementation: a cell-based luminescent assay platform for drug discovery. Assay Drug Dev Technol. 2007 Feb; 5(1) ; 137-44), tel qu'établi par DiscoveRx Corporation a été utilisé. La stimulation de ces deux lignées cellulaires avec CXCL8 (10 nM) induit le recrutement de la β-arrestine 2, comme indiqué par une augmentation significative du facteur d'induction. Tous les antagonistes CXCR2 ont été testés de manière dose-depente et la concentration correspondant à 50% d'inhibition de la réponse a été déterminée (IC₅₀ = concentration de demi inhibition).

### Assay Recrutement β-arrestines :

Les cellules « PathHunter HEK293-CXCR2 » ou « U2OS hCXCR1 b-arrestine » (DiscoveRx Corporation) ont été ensemencées une nuit à 10 000 cellules / puits (384 puits format) dans 20 µl de milieu Opti MEM I. Une préincubation avec l'antagoniste ou le véhicule de 30 min à 37 °C et 5% de CO2 a été suivie par 60 minutes de stimulation par CXCL8 à 37 °C et 5% de CO2. Les cellules ont ensuite été placées à température ambiante pendant 30 minutes. Le réactif de détection PathHunter (DiscoveRx Corporation) a été ajouté. Après une incubation de 60 min à température ambiante, la β-galactosidase induite par la luminescence lors de l'interaction β-arrestine-CXCR2 a été mesurée pour 0,3 s dans un Envision 2102 Multilabel Reader (PerkinElmer Life and Analytical Sciences). Les données ont été analysées par une procédure de courbe non linéaire utilisant le logiciel d'exploitation XLFit4 (IDBS) et les IC50 ont été déterminées.

| **Composé (Exemple n°)** | **CXCR1 (nM)** | **CXCR2 (nM)** |
|---|---|---|
| 38 | 322 | 25 |
| 48 | 566 | 26 |
| 22 | 117 | 31 |
| 37a | 827 | 32 |
| 8 | 604 | 37 |
| 9 | 666 | 60 |
| 50 | 312 | 72 |
| 25 | 3899 | 96 |
| 7 | 2412 | 100 |
| 12 | 9999 | 100 |
| 34 | 1611 | 106 |
| 3 | 1150 | 106 |
| 35 | 3031 | 146 |
| 36 | 1706 | 198 |
| 37b | 7685 | 207 |
| 27 | 1541 | 244 |
| 21 | 1442 | 257 |
| 14 | 1300 | 262 |
| 2 | 1595 | 272 |
| 42 | 9999 | 311 |
| 20 | 6621 | 335 |
| 5 | 2620 | 341 |
| 30 | 2245 | 349 |
| 19 | 4957 | 394 |
| 29 | 9999 | 433 |
| 28 | 5541 | 475 |
| 40 | 5225 | 502 |
| 11 | 6510 | 549 |
| 15 | 3171 | 626 |
| 31 | 3533 | 716 |
| 49 (énantiomère S) | 7386 | 733 |
| 33 | 9999 | 962 |
| 39 | 9999 | 1035 |
| 6 | 2841 | 1088 |
| 51 | 650 | 72 |
| 52 | 897 | 373 |
| 53 | 115 | 30 |

### EXEMPLE 55 : Polypharmacologie : « profiling réceptoriel »

**Mesure du flux calcique sur cellules:** Les expériences ont été réalisées sur la plateforme FLIPR TETRA® de Molecular Devices. Après une lecture du niveau basal, les composés ont été ajoutés aux cellules exprimant le récepteur aux chimiokines d'intérêt et l'activité agoniste a été lue à 10 secondes. Après une incubation supplémentaire de 10 minutes, les cellules ont été activées, avec une concentration équivalente à l'AC80, par un agoniste de référence afin de détecter si ce composé présente une activité antagoniste.

Chaque lignée cellulaire exprimant un récepteur aux chimiokines a été établie sur la base de cellule Chem-1 exprimant de manière stable la forme recombinante du récepteur aux chimiokines ainsi qu'une protéine G associée, dans le but de coupler le récepteur à la voie de signalisation du calcium.

21 recepeturs appartenant à la famille des récepteurs aux chémokines (CCRs et CXCRs) ont été analysés. Tous les antagonistes CXCR2 ont été testés de manière dose-depente et la concentration correspondant à 50% d'hinibition de la réponse a été déterminée (IC₅₀).

| | IC50 (nM) | | |
|---|---|---|---|
| Antagoniste | CCR4 | CCR6 | CXCR3 |
| Exemple 50 | IA | 1.4 | 700 |
| Exemple 22 | 1500 | 3.4 | 3300 |
| Example 53 | 450 | 1.9 | 920 |
| Exemple 8 | IA | 5.8 | 240 |

| | | | |
|---|---|---|---|
| IA : Inactif | | | |

### EXEMPLE 56 : Constante de dissociation

La détermination des constantes de demi-dissociation des antagonistes CXCR2 a été basée sur le modèle *InVitro* de recrutement de la β-Arrestin précédemment décrit : les cellules « PathHunter HEK293-CXCR2 » (DiscoveRx Corporation) ont été ensemencées une nuit à 20 000 cellules/puits (au format 96 puits) dans 100µL/puits de milieu de culture OptiMEM-1% SVF. Une préincubation avec l'antagoniste ou le véhicule a été réalisée pendant 1 heure à 37°C-5% CO₂. Les cellules ont été ensuite lavées 3 fois avec 100µL/puits de milieu OptiMEM-1% SVF puis une incubation variable (0h-0.5h-6h-12h-24h) des cellules à 37°C-5% CO₂ a été réalisée. Les cellules ont été ensuite stimulées par 4nM de CXCL8 à 37°C-5% CO₂ pendant 1h30. Le réactif de détection PathHunter (DiscoveRx Corporation) a été ajouté à raison de 50µL/puits. Après une incubation de 60 minutes à température ambiante, la luminescence émise, par l'hydrolyse du substrat par la β-galactosidase complémentée lors de l'interaction β-Arrestin-CXCR2, a été mesurée pendant 0,3 secondes/puits avec un Envision Multilabel Reader (PerkinElmer Life and Analytical Sciences). Les données ont été analysées par une procédure de courbe non linéaire utilisant le logiciel d'exploitation XLFit4 (IDBS) et les IC50 ont été déterminées. Le temps de demi-dissociation a été déterminé sur une régression de type y=(A*(1-exp(((-1)*B)*x))) (où x=temps et y=luminescence normalisée) à concentration saturante en antagoniste.

Résultats : Les molécules décrites dans la présente invention ont été comparées à la molécule SCH-527123 (décrite pour avoir une dissociation pseudo-irréversible) (Pharmacological Characterization of SCH-527123, a Potent Allosteric CXCR1/CXCR2 Antagonist. JPET 322:477-485, 2007).

| Antagoniste | Temps de demi-dissociation (heures) |
|---|---|
| DMSO (véhicule) | nd |
| SCH-527123 | >96 |
| Exemple 50 | 11 |

### EXEMPLE 57 : A/ Stabilités métaboliques en microsomes hépatiques

Des microsomes hépatiques (Becton Dickinson) ont été incubés à une concentration de protéine de 0.5 mg/mL dans le milieu réactionnel.

Le milieu réactionnel des microsomes a été composé de tampon Phosphate pH : 7.4 à 100 mM, de MgCl₂ à 100 mM. (50/50), d'un système de génération d'ATP composé d'un mélange de Nicotinamide Adénine Di-Phosphate (NADP) et de Glucose-6-Phosphate (G6P) à 1 mg/mL. de Glucose-6-Phosphate Deshydrogénase (G6PDH) à 4 U/mL. Les composés ont été testés à 1 µM (DMSO 0.1%)

Les prélèvements de milieu d'incubation après ajout des microsomes ont été réalisés aux temps 5, 10, 15, 30 et 60 minutes. A chaque temps, la réaction métabolique a été stoppée par ajout de méthanol (1 volume milieu incubation/3 volumes de méthanol). La disparition du produit parent a été mesurée par analyse LC/MS/MS. Le temps pour lequel 50% de produit parent a disparu (T1/2) a été calculé à partir de la cinétique de disparition du produit parent en fonction du temps.

| Antagoniste | Temps de demi-vie (min) |
|---|---|
| SCH-527123 | Stable (>60min) |
| Exemple 21 | 7.7 |
| Exemple 22 | 57 |
| Exemple 53 | 47 |

### B/ Stabilités métaboliques en hépatocytes.

Les Hépatocytes humains ont été fournis par Biopredic en plaque 24 puits. Après 48 h d'adaptation en culture, les hépatocytes ont été placés dans un milieu de traitement contenant 0.1% Sérum Albumine Bovine et les composés ont été testés à 1 µM (DMSO de 0.1%)

Les prélèvements de milieu d'incubation après ajout du composé à tester ont été réalisés aux temps t=0, 1, 2, 4, 6 et 24 heures.

A chaque temps, la réaction métabolique a été stoppée par ajout de méthanol (1 volume milieu incubation/3 volumes de méthanol). La disparition du produit parent a été mesurée par analyse LC/MS/MS. Le temps pour lequel 50% (T1/2) de produit parent a disparu a été calculé à partir de la cinétique de disparition du produit parent en fonction du temps.

| Antagoniste | Temps de demi-vie (min) |
|---|---|
| SCH-527123 | 900 |
| Exemple 22 | 445 |
| Exemple 53 | 186 |

## Revendications

1. Composé di-substitués de la diamino-3,4- cyclobutène-3-dione-1,2 répondant à la formule générale (I) suivante ou un de ses sels ou solvates pharmaceutiquement acceptable: 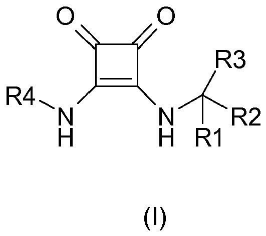 dans laquelle,
R1 représente un atome d'hydrogène ou un méthyle,
R2 représente un cycle à quatre atomes choisi parmi les structures (1) et (2) suivantes: 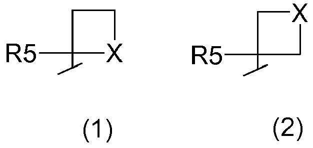 dans lesquelles R5 et X ont la signification donnée ci-après,
R3 représente un cycle aromatique ou heteroaromatique sélectionné dans le groupe constitué par les cycles répondant aux formules (a) à (o) suivantes : 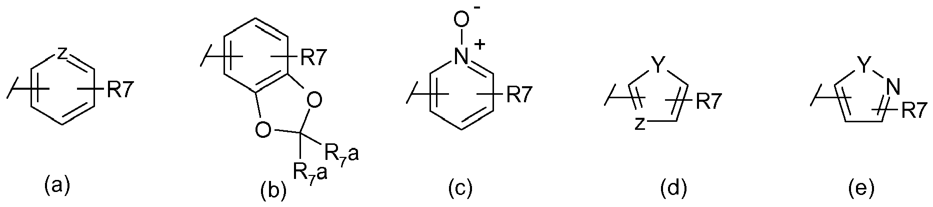 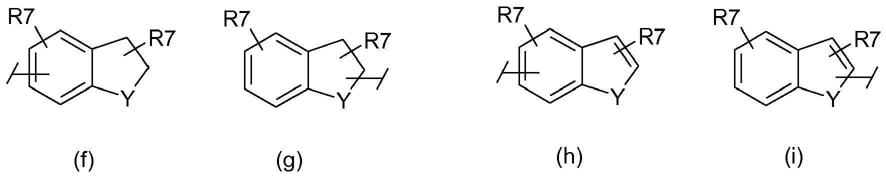 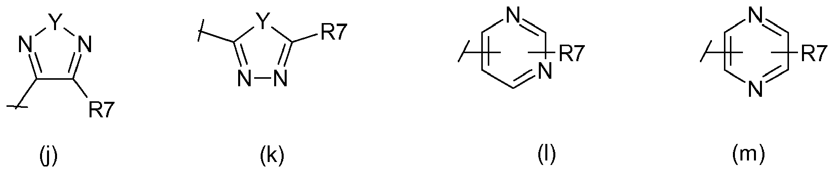 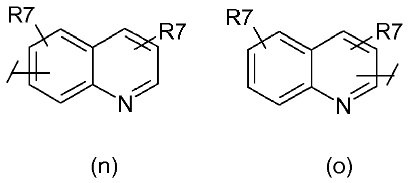 dans lesquelles R7, R7a, Y et Z ont la signification donnée ci-après, étant précisé que les cycles (a) à (o) peuvent éventuellement porter plusieurs groupes R7, identiques ou différents, le nombre total de tels groupes R7 étant au maximum égal au nombre d'atomes substituables du cycle ;
R4 représente un cycle aromatique ou hétéroaromatique sélectionné dans le groupe constitué par les cycles répondant aux formules (p) à (z) et (aa) à (ak) suivantes : 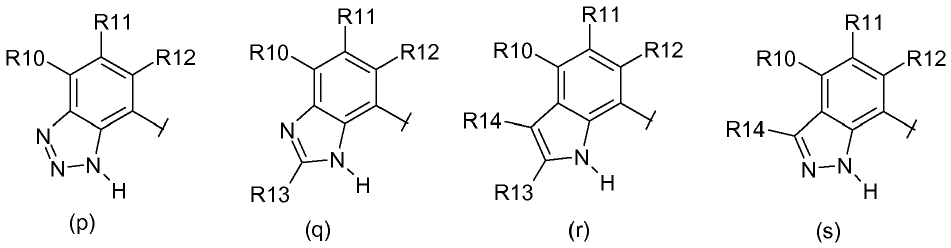 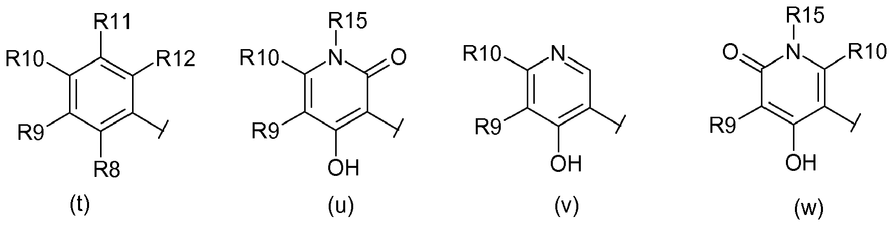 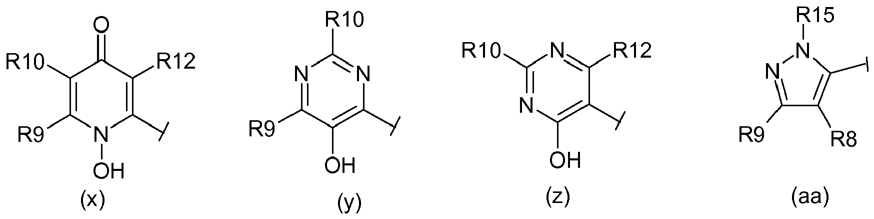 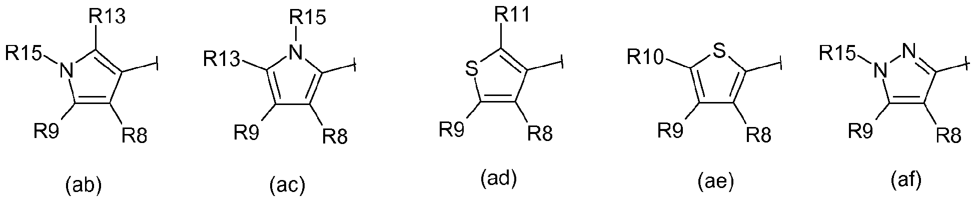 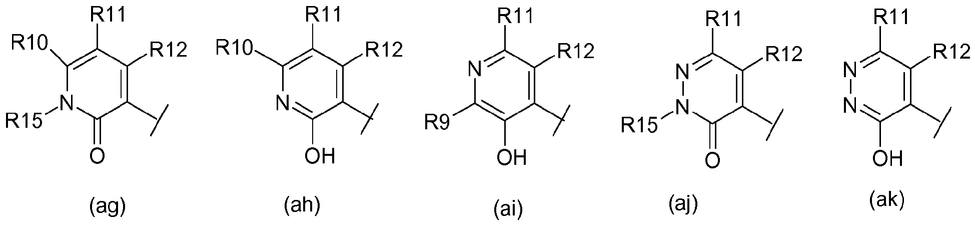 dans lesquelles R7, R8, R9, R10, R11, R12, R13, R14 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un radical R16, un halogène, -CF3, -COR16, -OR16, -NR16R17, -NO2, -CN, - SO2R16, -SO2NR16R17, -NR16COR17, -CONR16R17, -NR16CO2R17 ou -CO2R16,
R7a représente un atome d'hydrogène ou bien un radical alkyle ayant de 1 à 5 atomes de carbones,
R8 représente un atome d'hydrogène, un atome d'halogène, un radical -OH, -SH, - CONHOR16, -CONR16OH, -NR16R17, -SO3H, -OCOR16, -NHSO2R16, -SO2NR16R17, - NHCOR16, -CONR16R17, -NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un hydrogène, un atome d'halogène, un radical alkyle, alkoxy, -CF3, - OCF3, -OH, -NO2, -CN, -SO2R16, -SO2NR16R17, -NR16COR17, -NR16CO2R17, - CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p) à (z) et (aa) à (ak) ci-dessus, alors ils peuvent former ensemble, avec la liaison qui les réunit, un cycle aryle, hétéroaryle, cycloalkyle ou hétérocycloalkyle,
R13 et R14 sont identiques ou différents et sont indépendamment choisis dans le groupe constitué par un atome d'hydrogène, un atome d'halogène, un alkyle, -CF3, -OCF3, -OH, -SH, -CN, -SO2R16, -SO2NR16R17, -NHSO2NR16R17, -NR16R17, -NR16CONR16R17,-NR16COR17, -NR16CO2R17, -CONR16R17, -COR16 ou -CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, hétéroaryle, arylalkyle, hétéroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

2. Composé selon la revendication 1, **caractérisé en ce que** dans la formule (I) précitée:
R1 représente un atome d'hydrogène,
R2 représente un cycle à quatre chainons répondant à la structure (2) suivante: 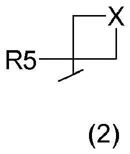 dans laquelle R5 et X ont la signification donnée ci-après,
R3 représente un cycle aromatique ou hétéroaromatique sélectionné dans le groupe constitué par les cycles répondant aux formules (a), (b) et (d) suivantes : 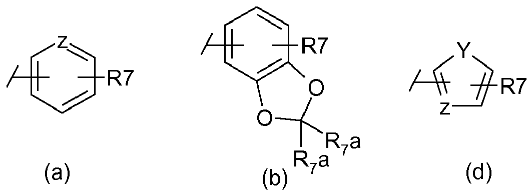 dans lesquelles R7, R7a, Y et Z ont la signification donnée ci-après, étant précisé que les cycles (a), (b) et (d) peuvent éventuellement porter plusieurs groupes R7, identiques ou
différents, le nombre total de tels groupe R7 étant au maximum égal au nombre d'atomes substituables du cycle ;
R4 représente un cycle aromatique ou hétéroaromatique sélectionné dans le groupe constitué par les cycles répondant aux formules (p), (q), (t), (z), (ad), (ag) et (ah) suivantes : 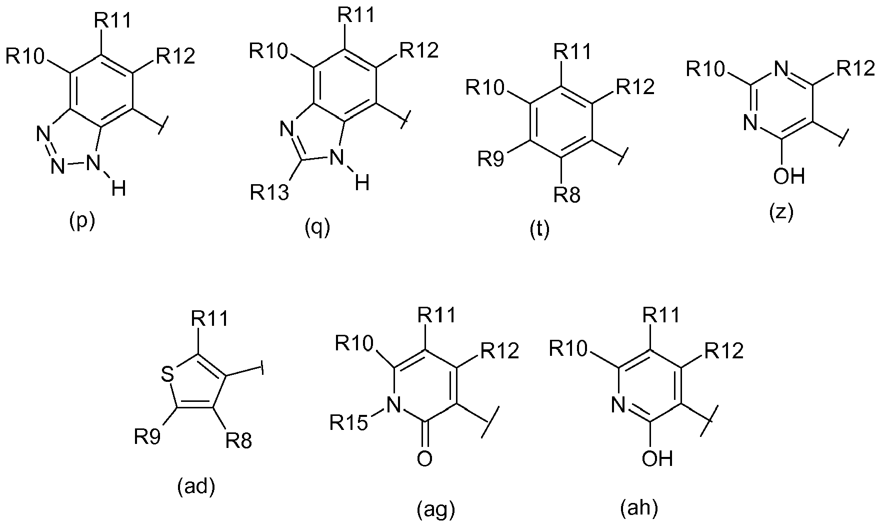 dans lesquelles R8, R9, R10, R11, R12, R13 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un halogène, un radical R16, -CF3, -COR16, -OR16, -NR16R17, -NO2, -CN, - SO2R16, -SO2NR16R17, -NR16COR17, -CONR16R17, -NR16CO2R17 ou -CO2R16,
R7a représente un atome d'hydrogène ou bien un radical alkyle ayant de 1 à 5 atomes de carbones,
R8 représente un atome d'hydrogène, un radical -OH, -SH, -CONHOR16, -CONR16OH, - NR16R17, -SO3H, -OCOR16, -NHSO2R16, -SO2NR16R17, -NHCOR16, -CONR16R17, - NR16CO2R17, -NHSO2NR16R17, -CO2R16, pyrrolyle, imidazolyle, triazolyle ou tétrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17, - CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p), (q), (t), (z), (ad), (ag) et (ah) ci-dessus, alors ils peuvent former ensemble, avec la liaison qui les réunit, un cycle aryle, hétéroaryle, cycloalkyle ou hétérocycloalkyle,
R13 est choisi dans le groupe constitué par un atome d'hydrogène, un atome d'halogène, un radical alkyle, -CF3, -OCF3, -OH, -SH, -CN, -SO2R16, -SO2NR16R17, -NHSO2NR16R17, - NR16R17, -NR16CONR16R17, -NR16COR17, -NR16CO2R17, -CONR16R17, -COR16 ou-CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou ycloalkylalkyle,
R16 et R17 sont identiques ou différents et sont indépendamment choisis dans le groupe constitué par un atome d'hydrogène, un radical aryle, hétéroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote, ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

3. Composé selon la revendication 1 ou 2, **caractérisé en ce que** dans la formule (I) précitée:
R1 représente un atome d'hydrogène,
R2 représente un cycle à quatre atomes répondant à la structure (2) suivante: 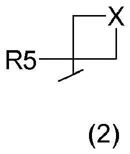 dans laquelle R5 et X ont la signification donnée ci-après,
R3 représente un cycle hétéroaromatique répondant à la formule (d) suivante : 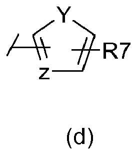
dans laquelle R7, Y et Z ont la signification donnée ci-après, étant précisé que le cycle (d) peut éventuellement porter plusieurs groupes R7, identiques ou différents, le nombre total de tels groupe R7 étant au maximum égal au nombre d'atomes substituables du cycle ;
R4 représente un cycle aromatique ou heteroaromatique sélectionné dans le groupe constitué par les cycles répondant aux formules (p), (q), (t), (z), (ad), (ag) et (ah) suivantes : 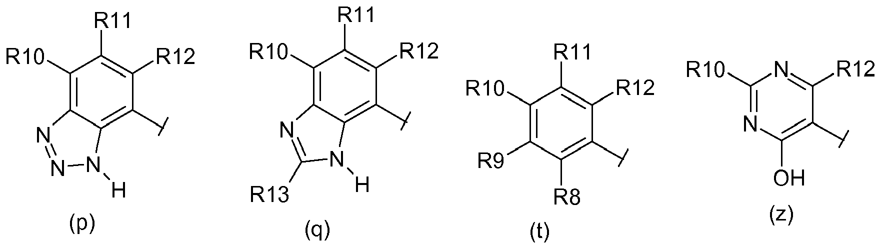 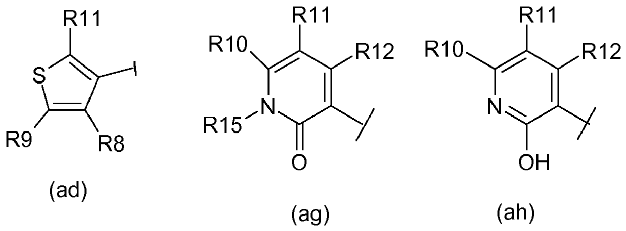 dans lesquelles R8, R9, R10, R11, R12, R13 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un atome d'halogène, un radical R16, -CF3, -COR16, -OR16, -NR16R17, - NO2, -CN, -SO2R16, -SO2NR16R17, -NR16COR17, -CONR16R17, -NR16CO2R17 ou - CO2R16,
R8 représente un atome d'hydrogène, un radical -OH, -SH, -CONHOR16, -CONR160H,-NR16R17, -SO3H, -OCOR16, -NHSO2R16, -SO2NR16R17, -NHCOR16, -CONR16R17, - NR16CO2R17, -NHSO2NR16R17, -CO2R16, pyrrolyle, imidazolyle, triazolyle ou tétrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p), (q), (t), (z), (ad), (ag) et (ah) ci-dessus, ils peuvent former ensemble, avec la liaison qui les réunit, un cycle aryle, hétéroaryle, cycloalkyle ou hétérocycloalkyle,
R13 est choisi dans le groupe constitué par un atome d'hydrogène, un atome d'halogène, un radical alkyle, -CF3, -OCF3, -OH, -SH, -CN, -SO2R16, -SO2NR16R17, -NHSO2NR16R17, - NR16R17, -NR16CONR16R17, -NR16COR17, -NR16CO2R17, -CONR16R17, -COR16 ou-CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

4. Composé selon l'une des revendications 1 à 3, **caractérisé en ce que** dans la formule (I) précitée:
R1 représente un atome d'hydrogène,
R2 représente un cycle à quatre atomes répondant à la structure (2) suivante: 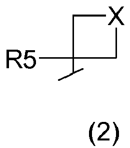 dans laquelle R5 et X ont la signification donnée ci-après,
R3 représente un cycle hétéroaromatique répondant à la formule (d) suivante : 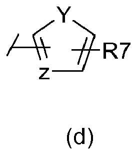 dans laquelle R7, Y et Z ont la signification donnée ci-après, étant précisé que le cycle (d) peut éventuellement porter plusieurs groupes R7, identiques ou différents, le nombre total de tels groupe R7 étant au maximum égal au nombre d'atomes substituables du cycle ;
R4 représente un cycle aromatique répondant à la formule (t) suivante : 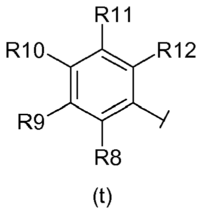 dans lesquelles R8, R9, R10, R11 et R12 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un radical R16, un halogène, -CF3, -COR16, -OR16, -NR16R17, -NO2, -CN, - SO2R16, -SO2NR16R17, -NR16COR17, -CONR16R17, -NR16CO2R17 ou -CO2R16,
R8 représente un atome d'hydrogène, un radical -OH, -SH, -CONHOR16, -CONR160H,-NR16R17, -SO3H, -OCOR16, -NHSO2R16, -SO2NR16R17, -NHCOR16, -CONR16R17, - NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17, - CONR16R17, -COR16 ou -CO2R16,
ou bien encore, lorsque deux des radicaux R9, R10, R11 et R12 se trouvent en position ortho sur le cycle aromatique (t), ils peuvent former ensemble, avec la liaison qui les réunit, un cycle aryle, hétéroaryle, cycloalkyle ou hétérocycloalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

5. Composition pharmaceutique comprenant une quantité efficace d'un composé ou d'un sel pharmaceutiquement acceptable duduit composé selon la revendication 1 en association avec un solvant ou un support pharmaceutiquement acceptable.

6. Composé selon la revendication 1 ou composition pharmaceutique selon la revendication 5 pour son utilisation en tant que médicament.

7. Composé selon la revendication 1 ou composition pharmaceutique selon la revendication 5 pour son utilisation dans le traitement de maladies médiées par les α-chimiokines.

8. Composé ou composition pharmaceutique selon la revendication 7 pour son utilisation dans le traitement de maladies du groupe comprenant les dermatoses neutrophiliques, notamment le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn, le rejet de greffe, la mucoviscidose et les cancers cutanés.

9. Composé ou composition pharmaceutique selon la revendication 7 pour son utilisation dans le traitement de maladies dermatologiques telles que les dermatoses neutrophiliques, notamment le psoriasis, les dermatites atopiques, l'acné et la rosacée.

10. Composé selon la revendication 1 choisi dans la liste constituée par:
1/ 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzamide
2/ 3-(2-{[(3-Fluorométhyl-oxetan-3-yl)-(5-méthyl-furan-2-yl)-méthyl]-amino}-3,4-dioxo-cyclobut-1-enylamino)-2-hydroxy-N,N-diméthyl-benzamide
3/ 3-(2-{[(3-Ethyl-oxetan-3-yl)-(5-méthyl-furan-2-yl)-méthyl]-amino}-3,4-dioxo-cyclobut-1-enyl-amino)-2-hydroxy-N,N-diméthyl-benzamide
4/ acide 2-hydroxy-3-(2-{[(5-méthyl-furan-2-yl)-(3-méthyl-oxetan-3-yl)-méthyl]-amino}-3,4-dioxo-cyclobut-1-enylamino)-benzoïque
5/ 3-[2-hydroxy-3-((R)-3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène- 1,2-dione
6/ 3-[2-hydroxy-3-((S)-3-hydroxy-pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione
7/ (R)-1-[2-hydroxy-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylic acid tert-butyl ester
8/ (R)-1-[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
9/ (S)-1-[2-Hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
10/ acide (R)-1-[2-hydroxy-3-(2-{[(5-méthyl-furan-2-yl)-(3-méthyl-oxetan-3-yl)-méthyl]-amino}-3,4-dioxocyclobut-1-enylamino)-benzoyl]-pyrrolidine-2-carboxylique
11/ 3-[2-hydroxy-3-(1-hydroxy-ethyl)-phenylamino]-4-{[(5-méthyl-furan-2-yl)-(3-méthyl-oxetan-3-yl)-méthyl]-amino}-cyclobut-3-ène-1,2-dione
12/ 3-(2-hydroxy-3-isobutyryl-phenylamino)-4-{[(5-méthyl-furan-2-yl)-(3-méthyl-oxetan-3-yl)-méthyl]-amino}-cyclobut-3-ène-1,2-dione
13/ 3-(4-hydroxy-2-méthyl-3-oxo-2,3-dihydro-1H-isoindol-5-ylamino)-4-{[(5-méthyl-furan-2-yl)-(3-méthyl-oxetan-3-yl)-méthyl]-amino}-cyclobut-3-ène-1,2-dione
14/ 3-[2-hydroxy-3-(pyrrolidine-1-carbonyl)-phenylamino]-4-{[(5-méthyl-furan-2-yl)-(3-méthyl-oxetan-3-yl)-méthyl]-amino}-cyclobut-3-ène-1,2-dione
15/ 3-[2-hydroxy-3-(morpholine-4-carbonyl)-phenylamino]-4-{[(5-méthyl-furan-2-yl)-(3-méthyl-oxetan-3-yl)-méthyl]-amino}-cyclobut-3-ène-1,2-dione
16/ 3-(4-hydroxy-pyrimidin-5-ylamino)-4-{[(5-méthyl-furan-2-yl)-(3-méthyl-oxetan-3-yl)-méthyl]-amino}-cyclobut-3-ène-1,2-dione
17/ 3-{[(5-méthyl-furan-2-yl)-(3-méthyl-oxetan-3-yl)-méthyl]-amino}-4-(1-méthyl-2-oxo-1,2-dihydro-quinolin-3-ylamino)-cyclobut-3-ène-1,2-dione
18/ 3-{[(5-méthyl-furan-2-yl)-(3-méthyl-oxetan-3-yl)-méthyl]-amino}-4-(6-méthyl-2-oxo-2H-pyran-3-ylamino)-cyclobut-3-ène-1,2-dione
19/ 3-{[(5-méthyl-furan-2-yl)-(3-méthyl-oxetan-3-yl)-méthyl]-amino}-4-(1-méthyl-2-oxo-1,2-dihydro-pyridin-3-ylamino)-cyclobut-3-ène-1,2-dione
20/ 3-(2-hydroxy-pyridin-3-ylamino)-4-{[(5-méthyl-furan-2-yl)-(3-méthyl-oxetan-3-yl)-méthyl]-amino}-cyclobut-3-ène-1,2-dione
21/ 2-hydroxy-N,N-diméthyl-3-(2-{[(5-méthyl-furan-2-yl)-(3-méthyl-oxetan-3-yl)-méthyl]-amino}-3,4-dioxo-cyclobut-1-enylamino)-benzènesulfonamide
22/ 2-Hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzènesulfonamide
23/ 3-(3H-benzotriazol-4-ylamino)-4-{[(5-méthyl-furan-2-yl)-(3-méthyl-oxetan-3-yl)-méthyl]-amino}-cyclobut-3-ène-1,2-dione
24/ 3-{[(5-Méthyl-furan-2-yl)-(3-méthyl-oxetan-3-yl)-méthyl]-amino}-4-(2-oxo-2,3-dihydro-benzooxazol-7-ylamino)-cyclobut-3-ène-1,2-dione
25/ 3-hydroxy-4-(2-{[(R)-(5-méthyl-furan-2-yl)-(3-méthyl-oxetan-3-yl)-méthyl]-amino}-3,4-dioxcyclobut-1-enylamino)-thiophene-2-carboxylate de diméthylamide
26/ 3-[[2-(3-Diméthylcarbamoyl-2-hydroxy-phenylamino)-3,4-dioxo-cyclobut-1-enylamino]-(5-méthyl-furan-2-yl)-méthyl]-3-méthyl-azetidine-1-carboxylate de tert-butyle
27/ 3-(2-{[(4,5-diméthyl-furan-2-yl)-(3-méthyl-oxetan-3-yl)-méthyl]-amino}-3,4-dioxo-cyclobut-1-enylamino)-2-hydroxy-N,N-diméthyl-benzamide
28/ 3-(2-{[(3-méthyl-oxetan-3-yl)-(3-méthoxy-phenyl)-méthyl]-amino}-3,4-dioxo-cyclobut-1-enylamino)-2-hydroxy-N,N-diméthyl-benzamide
29/ 3-(2-{[(3-méthyl-oxetan-3-yl)-(4-méthoxy-phenyl)-méthyl]-amino}-3,4-dioxo-cyclobut-1-enylamino)-2-hydroxy-N,N-diméthyl-benzamide
30/ 3-(2-{[benzo[1,3]dioxol-5-yl-(3-méthyl-oxetan-3-yl)-méthyl]-amino}-3,4-dioxo-cyclobut -1-enylamino)-2-hydroxy-N,N-diméthyl-benzamide
31/ 3-[3-(3-hydroxy-pyrrolidine-1-carbonyl)-phénylamino]-4-{[(5-méthyl-furan-2-yl)-(3-méthyl-oxetan-3-yl)-méthyl]-amino}-cyclobut-3-ène-1,2-dione
32/ 1-[3-(2-{[(5-méthyl-furan-2-yl)-(3-méthyl-oxetan-3-yl)-méthyl]-amino}-3,4-dioxo-cyclobut-1-enylamino)-benzoyl]-piperidine-2-carboxylate de méthyle
33/ 1-[3-(2-{[(5-Méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
34/ 2-hydroxy-N,N-diméthyl-3-(2-{[(3-méthyl-oxetan-3-yl)-thiophen-2-yl-méthyl]-amino}-3,4-dioxo-cyclobut-1-enylamino)-benzamide
35/ 2-hydroxy-N,N-diméthyl-3-(2-{[(3-méthyl-oxetan-3-yl)-(5-méthyl-thiophen-2-yl)-méthyl]-amino}-3,4-dioxo-cyclobut-1-enylamino)-benzamide
36/ 3-(2-{[furan-2-yl-(3-méthyl-oxetan-3-yl)-méthyl]-amino}-3,4-dioxo-cyclobut-1 enylamino)-2-hydroxy-N,N-diméthyl-benzamide
37a/ 2-hydroxy-N,N-diméthyl-3-(2-{[(4-méthyl-furan-2-yl)-(3-méthyl-oxetan-3-yl)-méthyl]-amino}-3,4-dioxo-cyclobut-1-enylamino)-benzamide
37b/ 2-Hydroxy-N,N-diméthyl-3-(2-{[(3-méthyl-furan-2-yl)-(3-méthyl-oxetan-3-yl)-méthyl]-amino}-3,4-dioxo-cyclobut-1-enylamino)-benzamide
38/ 2-hydroxy-N,N-diméthyl-3-(2-{[(4-isopropyl-furan-2-yl)-(3-méthyl-oxetan-3-yl)-méthyl]-amino}-3,4-dioxo-cyclobut-1-enylamino)-benzamide
39/ 3-(2-hydroxy-6-méthoxy-pyridin-3-ylamino)-4-{[(5-méthyl-furan-2-yl)-(3-méthyl-oxetan-3-yl)-méthyl]-amino}-cyclobut-3-ène-1,2-dione
40/ 3-(6-chloro-2-hydroxy-pyridin-3-ylamino)-4-{[(5-méthyl-furan-2-yl)-(3-méthyl-oxetan-3-yl)-méthyl]-amino}-cyclobut-3-ène-1,2-dione
41/ 3-{[(5-Méthyl-furan-2-yl)-(3-méthyl-oxetan-3-yl)-méthyl]-amino}-4-(2-oxo-2,3-dihydro-1H-benzoimidazol-4-ylamino)-cyclobut-3-ène-1,2-dione
42/ 2-hydroxy-3-(2-{[(5-méthyl-furan-2-yl)-(3-méthyl-oxetan-3-yl)-méthyl]-amino}-3,4-dioxo-cyclobut-1-enylamino)-benzonitrile
43/ (R)-1-[3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle
44/ (S)-1-[4-chloro-3-(2-{[(5-méthyl-furan-2-yl)-(3-méthyl-oxetan-3-yl)-méthyl]-amino}-3,4-dioxo-cyclobut-1-enylamino)-benzènesulfonyl]-pyrrolidine-2-carboxylate de méthyle
45/ (S)-1-[2,6-Difluoro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
46/ (S)-1-[2-chloro-3-(2-{[(5-méthyl-furan-2-yl)-(3-méthyl-oxetan-3-yl)-méthyl]-amino}-3,4-dioxo-cyclobut-1-enylamino)-benzoyl]-pyrrolidine-2-carboxylate de méthyle
47/ (R)-1-[2-hydroxy-3-(2-{[(R)-(5-méthyl-furan-2-yl)-(3-méthyl-oxetan-3-yl)-méthyl]-amino}-3,4-dioxo-cyclobut-1-énylamino)-benzoyl]-pyrrolidine-2-carboxylate de méthyle
48/ 2-hydroxy-N-méthyl-3-(2-{[(R)-(5-méthyl-furan-2-yl)-(3-méthyl-oxetan-3-yl)-méthyl]-amino}-3,4-dioxo-cyclobut-1-énylamino)-N-(2,2,2-trifluoro-éthyl)-benzamide
49/ 2-hydroxy-N,N-diméthyl-3-(2-{[((R)-5-méthyl-furan-2-yl)-(3-méthyl-oxetan-3-yl)-méthyl]-amino}-3,4-dioxo-cyclobut-1-enylamino)-benzamide
51/ {[2-hydroxy-3-(2-{[(R)-(5-méthyl-furan-2-yl)-(3-méthyl-oxetan-3-yl)-méthyl]-amino}-3,4-dioxo-cyclobut-1-enylamino)-benzoyl]-méthyl-amino}-acétate de méthyle
52/ 6-chloro-2-hydroxy-N,N-diméthyl-3-(2-{[(R)-(5-méthyl-furan-2-yl)-(3-méthyl-oxetan-3-yl)-méthyl]-amino}-3,4-dioxo-cyclobut-1-enylamino)-benzamide
53/ 3-[4-chloro-2-hydroxy-3-(4-méthyl-pipérazine-1-sulfonyl)-phénylamino]-4-{[(R)-(5-méthyl-furan-2-yl)-(3-méthyl-oxetan-3-yl)-méthyl]-amino}-cyclobut-3-ene-1,2-dione

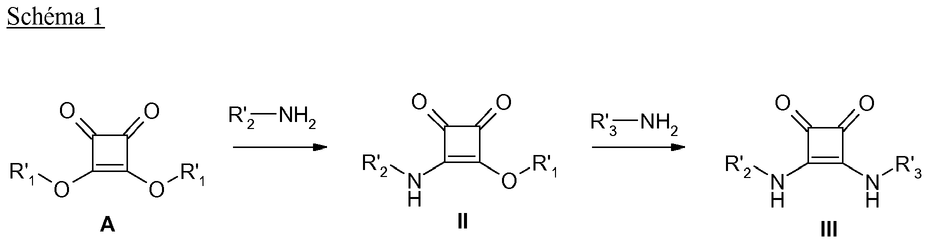

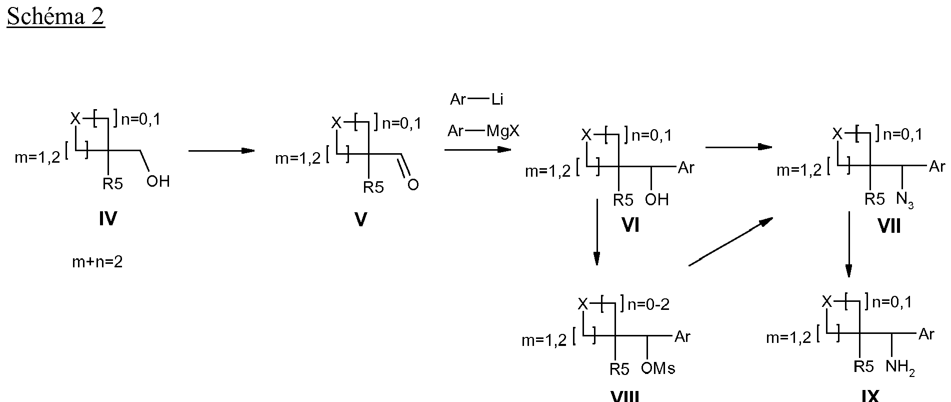

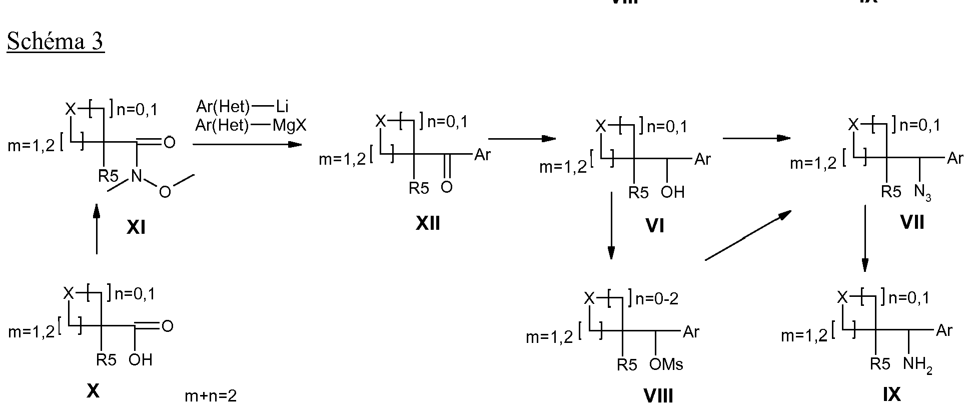

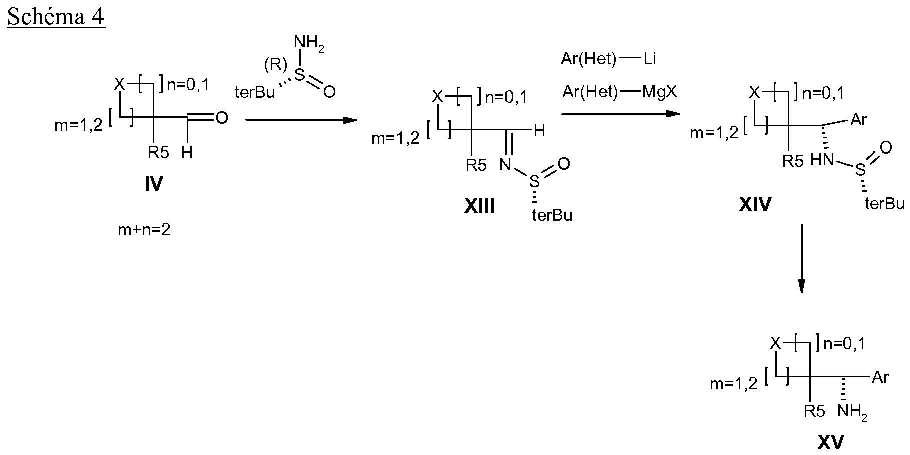

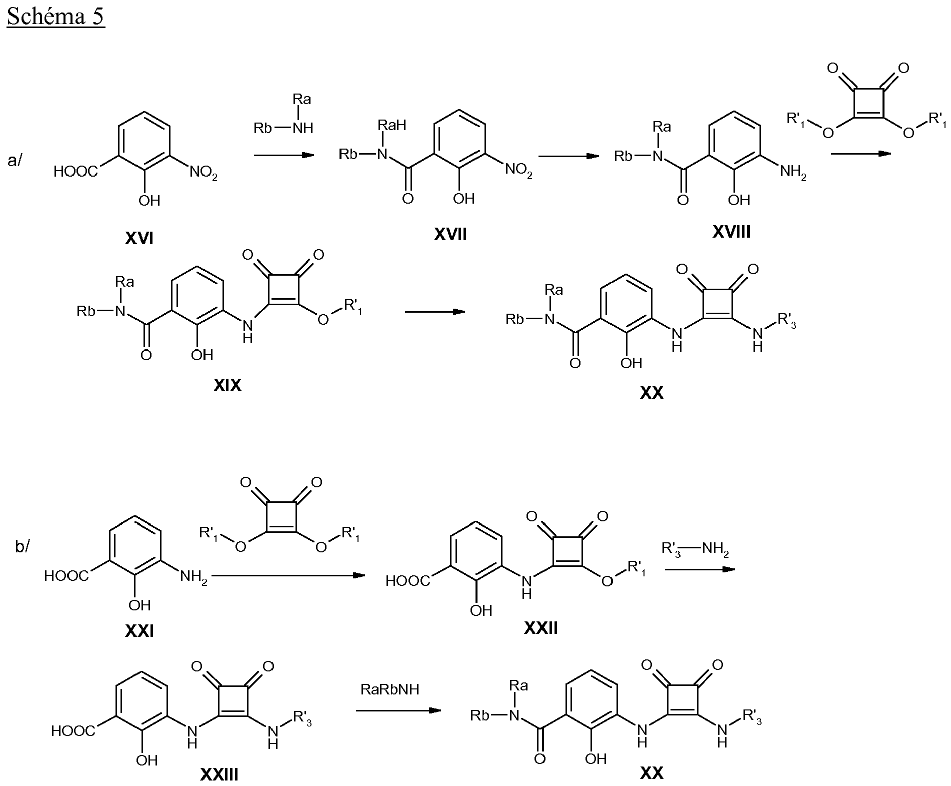

## Patentansprüche

1. Disubstituierte 3,4-Diamino-3-cyclobuten-1,2-dion-verbindung der folgenden allgemeinen Formel (I) oder eines ihrer pharmazeutisch unbedenklichen Salze oder Solvate: 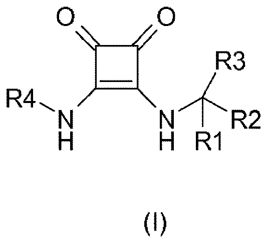 wobei
R1 für ein Wasserstoffatom oder ein Methyl steht, R2 für einen vieratomigen Ring steht, der aus den folgenden Formeln (1) und (2) ausgewählt ist: 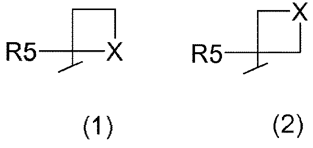 wobei R5 und X die nachstehend angegebene Bedeutung besitzen,
R3 für einen aromatischen oder heteroaromatischen Ring steht, der aus der Gruppe der Ringe der folgenden Formeln (a) bis (o) ausgewählt ist: 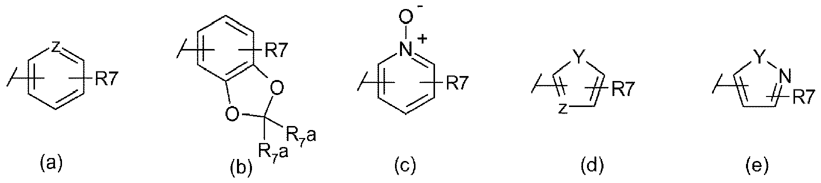 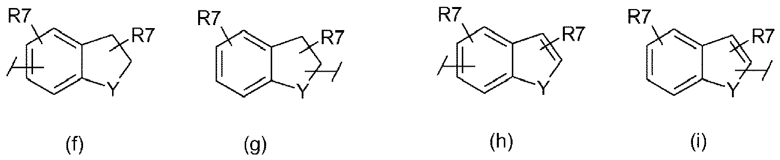 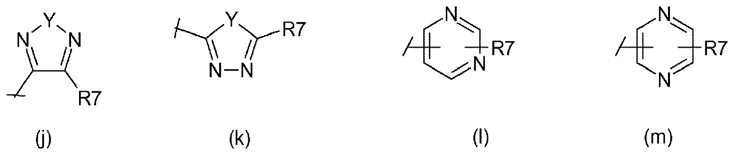 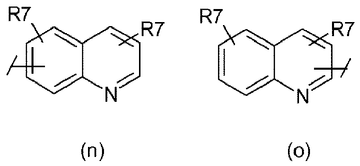 wobei R7, R7a, Y und Z die nachstehend angegebene Bedeutung besitzen, wobei die Ringe (a) bis (o) gegebenenfalls mehrere gleiche oder verschiedene R7-Gruppen tragen können, wobei die Gesamtzahl derartiger R7-Gruppen höchstens gleich der Zahl substituierbarer Atome des Rings ist;
R4 für einen aromatischen oder heteroaromatischen Ring steht, der aus der Gruppe der Ringe der folgenden Formeln (p) bis (z) und (aa) und (ak) ausgewählt ist: 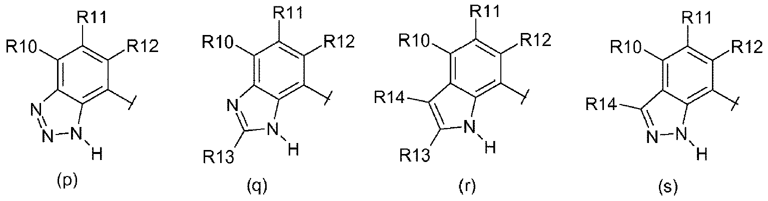 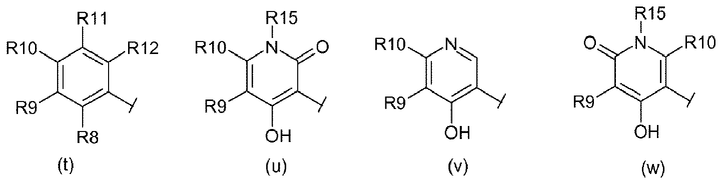 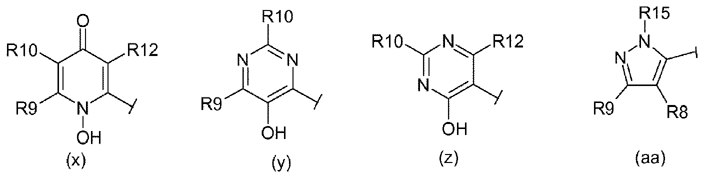 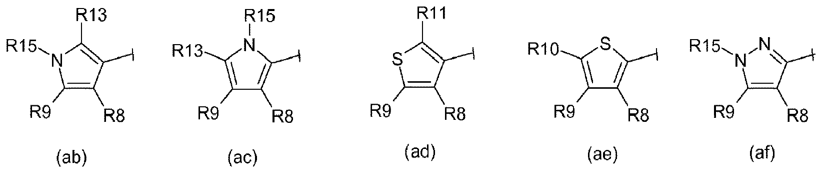 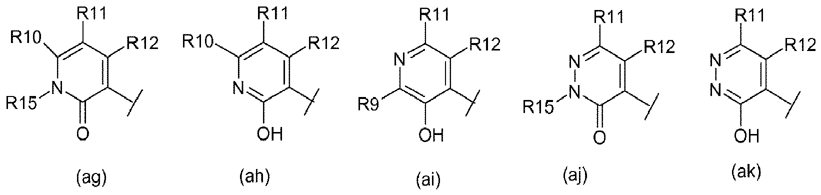 wobei R7, R8, R9, R10, R11, R12, R13, R14 und R15 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für einen Rest R16, ein Halogen, -CF₃, -COR16, -OR16, -NR16R17, -NO₂, -CN, -SO₂R16, -SO₂NR16R17, -NR16COR17, -CONR16R17, -NR16CO₂R17 oder -CO₂R16 steht,
R7a für ein Wasserstoffatom oder einen Alkylrest mit 1 bis 5 Kohlenstoffatomen steht,
R8 für ein Wasserstoffatom, ein Halogenatom oder einen -OH-, -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoff, einem Halogenatom und einem Alkyl-, Alkoxy-, -CF₃-, -OCF₃-, -OH-, -NO₂-, -CN-, -SO₂R16-, -SO₂NR16R17-, -NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an einem aromatischen oder heteroaromatischen Ring, der aus der Gruppe bestehend aus den Ringen der obigen Formeln (p) bis (z) und (aa) bis (ak) ausgewählt ist, stehen, zusammen mit der sie verknüpfenden Bindung einen Aryl-, Heteroaryl-, Cycloalkyl- oder Heterocycloalkylring bilden können,
R13 und R14 gleich oder verschieden sind und unabhängig voneinander aus der Gruppe bestehend aus einem Wasserstoffatom, einem Halogenatom, einem Alkyl, -CF₃, -OCF₃, -OH, -SH, -CN, -SO₂R16, -SO₂NR16R17, -NHSO₂NR16R17, -NR16R17, -NR16CONR16R17, -NR16COR17, -NR16CO₂R17, -CONR16R17, -COR16 oder -CO₂R16 ausgewählt sind,
R15 für ein Wasserstoffatom oder einen -OH-, -SO₂R16-, -COR16-, -CO₂R16-, Aryl-, Heteroaryl-, Arylalkyl-, Heteroarylalkyl-, Alkyl-, Cycloalkyl-oder Cycloalkylalkylrest steht,
R16 und R17 gleich oder verschieden sind und unabhängig voneinander aus der Gruppe bestehend aus einem Wasserstoffatom, einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkylalkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoff, an den sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X für ein Sauerstoffatom, ein Schwefelatom oder ein durch einen Rest R6 substituiertes Stickstoffatom steht,
Y für ein Sauerstoffatom, ein Schwefelatom oder ein durch einen Rest R15 substituiertes Stickstoffatom steht und
Z für ein Kohlenstoff- oder Stickstoffatom steht.

2. Verbindung nach Anspruch 1, **dadurch gekennzeichnet, dass** in der obigen Formel (I):
R1 für ein Wasserstoffatom steht,
R2 für einen viergliedrigen Ring der folgenden Struktur (2) steht: 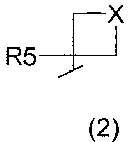 wobei R5 und X die nachstehend angegebene Bedeutung besitzen,
R3 für einen aromatischen oder heteroaromatischen Ring steht, der aus der Gruppe der Ringe der folgenden Formeln (a), (b) und (d) ausgewählt ist: 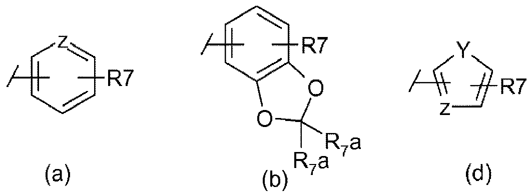 wobei R7, R7a, Y und Z die nachstehend angegebene Bedeutung besitzen, wobei die Ringe (a), (b) und (d) gegebenenfalls mehrere gleiche oder verschiedene R7-Gruppen tragen können, wobei die Gesamtzahl derartiger R7-Gruppen höchstens gleich der Zahl substituierbarer Atome des Rings ist;
R4 für einen aromatischen oder heteroaromatischen Ring steht, der aus der Gruppe der Ringe der folgenden Formeln (p), (q), (t), (z), (ad), (ag) und (ah) ausgewählt ist: 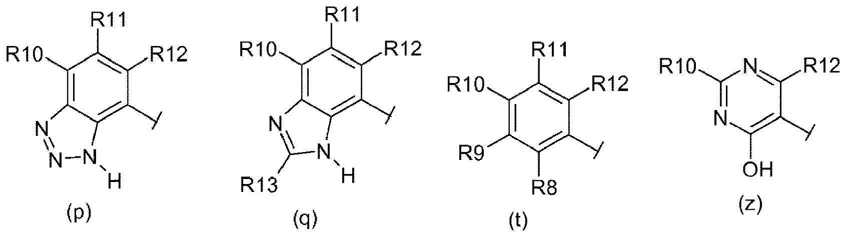 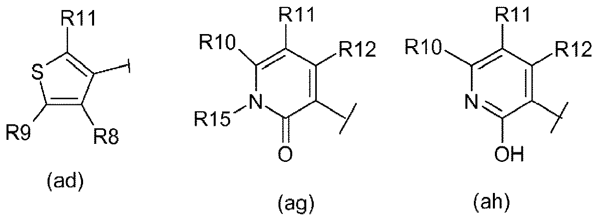 wobei R8, R9, R10, R11, R12, R13 und R15 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für ein Halogen oder einen R16-, -CF₃-, -COR16-, -OR16-, -NR16R17-, -NO₂-, -CN-, -SO₂R16-, -SO₂NR16R17-, -NR16COR17-, -CONR16R17-, -NR16CO₂R17- oder -CO₂R16-Rest steht,
R7a für ein Wasserstoffatom oder einen Alkylrest mit 1 bis 5 Kohlenstoffatomen steht,
R8 für ein Wasserstoffatom oder einen -OH-, -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoffatom, einem Halogenatom und einem Alkyl-, Alkoxy-, -CF₃-,-OCF₃-, -OH-, -NO₂-, -CN-, -SO₂R16-, -SO₂NR16R17-,-NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an einem aromatischen oder heteroaromatischen Ring, der aus der Gruppe bestehend aus den Ringen der obigen Formeln (p), (q), (t), (z), (ad), (ag) und (ah) ausgewählt ist, stehen, zusammen mit der sie verknüpfenden Bindung einen Aryl-, Heteroaryl-, Cycloalkyl- oder Heterocycloalkylring bilden können,
R13 aus der Gruppe bestehend aus einem Wasserstoffatom, einem Halogenatom und einem Alkyl-, -CF₃-, -OCF₃-, -OH-, -SH-, -CN-, -SO₂R16-, -SO₂NR16R17-, -NHSO₂NR16R17-, -NR16R17-, -NR16CONR16R17-, -NR16COR17-, -NR16CO₂R17-, -CONR16R17-, -COR16- oder -CO₂R16-Rest ausgewählt ist,
R15 für ein Wasserstoffatom oder einen -OH-, -SO₂R16-, -COR16-, -CO₂R16-, Aryl-, Heteroaryl-, Arylalkyl-, Heteroarylalkyl-, Alkyl-, Cycloalkyl-oder Cycloalkylalkylrest steht,
R16 und R17 gleich oder verschieden sind und unabhängig voneinander aus der Gruppe bestehend aus einem Wasserstoffatom, einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkylalkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoffatom, an das sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X für ein Sauerstoffatom, ein Schwefelatom oder ein durch einen Rest R6 substituiertes Stickstoffatom steht,
Y für ein Sauerstoffatom, ein Schwefelatom oder ein durch einen Rest R15 substituiertes Stickstoffatom steht und
Z für ein Kohlenstoff- oder Stickstoffatom steht.

3. Verbindung nach Anspruch 1 oder 2, **dadurch gekennzeichnet, dass** in der obigen Formel (I):
R1 für ein Wasserstoffatom steht,
R2 für einen vieratomigen Ring der folgenden Struktur (2) steht: 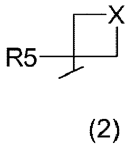 wobei R5 und X die nachstehend angegebene Bedeutung besitzen,
R3 für einen heteroaromatischen Ring der folgenden Formel (d) steht: 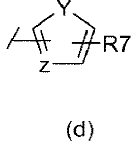 wobei R7, Y und Z die nachstehend angegebene Bedeutung besitzen, wobei der Ring (d) gegebenenfalls mehrere gleiche oder verschiedene R7-Gruppen tragen kann, wobei die Gesamtzahl derartiger R7-Gruppen höchstens gleich der Zahl substituierbarer Atome des Rings ist;
R4 für einen aromatischen oder heteroaromatischen Ring steht, der aus der Gruppe der Ringe der folgenden Formeln (p), (q), (t), (z), (ad), (ag) und (ah) ausgewählt ist: 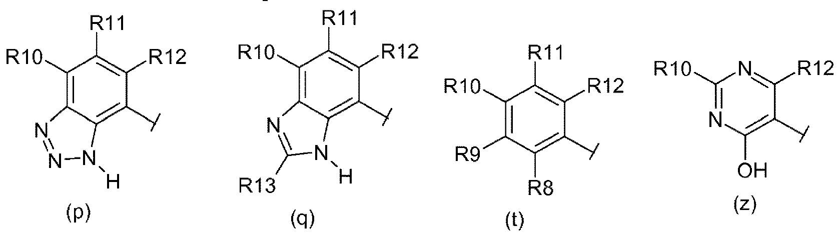 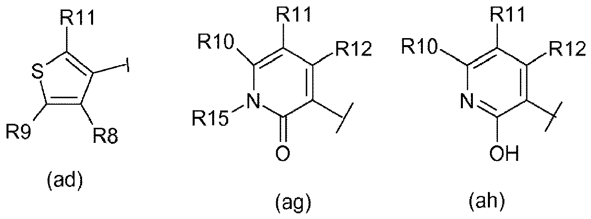 wobei R8, R9, R10, R11, R12, R13 und R15 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für ein Halogenatom oder einen R16-, -CF₃-, -COR16-, -OR16-, -NR16R17-, -NO₂-, -CN-, -SO₂R16-, -SO₂NR16R17-, -NR16COR17-, -CONR16R17-, -NR16CO₂R17- oder -CO₂R16-Rest steht,
R8 für ein Wasserstoffatom oder einen -OH-, -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoffatom, einem Halogenatom und einem Alkyl-, Alkoxy-, -CF₃-,-OCF₃-, -OH-, -NO₂-, -CN-, -SO₂R16-, -SO₂NR16R17-,-NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an einem aromatischen oder heteroaromatischen Ring, der aus der Gruppe bestehend aus den Ringen der obigen Formeln (p), (q), (t), (z), (ad), (ag) und (ah) ausgewählt ist, stehen, zusammen mit der sie verknüpfenden Bindung einen Aryl-, Heteroaryl-, Cycloalkyl- oder Heterocycloalkylring bilden können,
R13 aus der Gruppe bestehend aus einem Wasserstoffatom, einem Halogenatom und einem Alkyl-, -CF₃-, -OCF₃-, -OH-, -SH-, -CN-, -SO₂R16-, -SO₂NR16R17-, -NHSO₂NR16R17-, -NR16R17-, -NR16CONR16R17-, -NR16COR17-, -NR16CO₂R17-, -CONR16R17-, -COR16- oder -CO₂R16-Rest ausgewählt ist,
R15 für ein Wasserstoffatom oder einen -OH-, -SO₂R16-, -COR16-, -CO₂R16-, Aryl-, Heteroaryl-, Arylalkyl-, Heteroarylalkyl-, Alkyl-, Cycloalkyl-oder Cycloalkylalkylrest steht,
R16 und R17 gleich oder verschieden sind und unabhängig voneinander aus der Gruppe bestehend aus einem Wasserstoffatom, einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkylalkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoffatom, an das sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X für ein Sauerstoffatom, ein Schwefelatom oder ein durch einen Rest R6 substituiertes Stickstoffatom steht,
Y für ein Sauerstoffatom, ein Schwefelatom oder ein durch einen Rest R15 substituiertes Stickstoffatom steht und
Z für ein Kohlenstoff- oder Stickstoffatom steht.

4. Verbindung nach einem der Ansprüche 1 bis 3, **dadurch gekennzeichnet, dass** in der obigen Formel (I) :
R1 für ein Wasserstoffatom steht,
R2 für einen vieratomigen Ring der folgenden Struktur (2) steht: 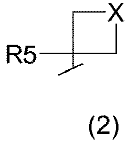 wobei R5 und X die nachstehend angegebene Bedeutung besitzen,
R3 für einen heteroaromatischen Ring der folgenden Formel (d) steht: 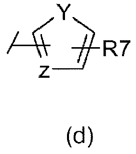 wobei R7, Y und Z die nachstehend angegebene Bedeutung besitzen, wobei der Ring (d) gegebenenfalls mehrere gleiche oder verschiedene R7-Gruppen tragen kann, wobei die Gesamtzahl derartiger R7-Gruppen höchstens gleich der Zahl substituierbarer Atome des Rings ist;
R4 für einen aromatischen Ring der folgenden Formel (t) steht: 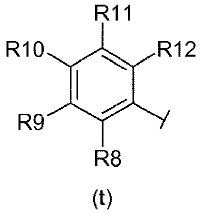 wobei R8, R9, R10, R11 und R12 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für einen Rest R16, ein Halogen, -CF₃, -COR16, -OR16, -NR16R17, -NO₂, -CN, -SO₂R16, -SO₂NR16R17, -NR16COR17, -CONR16R17, -NR16CO₂R17 oder -CO₂R16 steht,
R8 für ein Wasserstoffatom oder einen -OH-, -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoffatom, einem Halogenatom und einem Alkyl-, Alkoxy-, -CF₃-,-OCF₃-, -OH-, -NO₂-, -CN-, -SO₂R16-, -SO₂NR16R17-,-NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an dem aromatischen Ring (t) stehen, zusammen mit der sie verknüpfenden Bindung einen Aryl-, Heteroaryl-, Cycloalkyl- oder Heterocycloalkylring bilden können,
R16 und R17 gleich oder verschieden sind und unabhängig voneinander aus der Gruppe bestehend aus einem Wasserstoffatom und einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkylalkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoffatom, an das sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X für ein Sauerstoffatom, ein Schwefelatom oder ein durch einen Rest R6 substituiertes Stickstoffatom steht,
Y für ein Sauerstoffatom, ein Schwefelatom oder ein durch einen Rest R15 substituiertes Stickstoffatom steht und
Z für ein Kohlenstoff- oder Stickstoffatom steht.

5. Pharmazeutische Zusammensetzung, umfassend eine wirksame Menge einer Verbindung oder eines pharmazeutisch unbedenklichen Salzes der Verbindung nach Anspruch 1 in Kombination mit einem pharmazeutisch unbedenklichen Lösungsmittel oder Träger.

6. Verbindung nach Anspruch 1 oder pharmazeutische Zusammensetzung nach Anspruch 5 zur Verwendung als Medikament.

7. Verbindung nach Anspruch 1 oder pharmazeutische Zusammensetzung nach Anspruch 5 zur Verwendung bei der Behandlung von durch α-Chemokine vermittelten Erkrankungen.

8. Verbindung oder pharmazeutische Zusammensetzung nach Anspruch 7 zur Verwendung bei der Behandlung von Erkrankungen aus der Gruppe umfassend neutrophile Dermatosen, insbesondere Psoriasis, atopische Dermatiden, Akne, Rosacea, Asthma, chronisch-obstruktive Lungenerkrankungen, Atemwegserkrankungen bei Erwachsenen, Arthritis, entzündliche Darmerkrankungen, Morbus Crohn, Transplantatabstoßung, Mukoviszidose und Hautkrebserkrankungen.

9. Verbindung oder pharmazeutische Zusammensetzung nach Anspruch 7 zur Verwendung bei der Behandlung von dermatologischen Erkrankungen wie neutrophilen Dermatosen, insbesondere Psoriasis, atopischen Dermatiden, Akne und Rosacea.

10. Verbindung nach Anspruch 1, ausgewählt aus der Liste bestehend aus:
1/ 2-Hydroxy-N,N-dimethyl-3-(2-{[(5-methylfuran-2-yl)-(3-methyloxetan-3-yl)methyl]amino}-3,4-dioxocyclobut-1-enylamino)benzamid
2/ 3-(2-{[(3-Fluormethyloxetan-3-yl)-(5-methyl-furan-2-yl)methyl]amino}-3,4-dioxocyclobut-1-enylamino)-2-hydroxy-N,N-dimethylbenzamid
3/ 3-(2-{[(3-Ethyloxetan-3-yl)-(5-methylfuran-2-yl)methyl]amino}-3,4-dioxocyclobut-1-enylamino)-2-hydroxy-N,N-dimethylbenzamid
4/ 2-Hydroxy-3-(2-{[(5-methylfuran-2-yl)-(3-methyloxetan-3-yl)methyl]amino}-3,4-dioxocyclobut-1-enylamino)benzoesäure
5/ 3-[2-Hydroxy-3-((R)-3-hydroxypyrrolidin-1-carbonyl)phenylamino]-4-{[(5-methylfuran-2-yl)-(3-methyloxetan-3-yl)methyl]amino}cyclobut-3-en-1,2-dion
6/ 3-[2-Hydroxy-3-((S)-3-hydroxypyrrolidin-1-carbonyl)phenylamino]-4-{[(5-methylfuran-2-yl)-(3-methyloxetan-3-yl)methyl]amino}cyclobut-3-en-1,2-dion
7/ (R)-1-[2-Hydroxy-3-(2-{[(5-methylfuran-2-yl)-(3-methyloxetan-3-yl)methyl]amino}-3,4-dioxocyclobut-1-enylamino)benzoyl]pyrrolidin-2-carbonsäure-tert-butylester
8/ (R)-1-[2-Hydroxy-3-(2-{[(R)-(5-methylfuran-2-yl)-(3-methyloxetan-3-yl)methyl]amino}-3,4-dioxocyclobut-1-enylamino)benzoyl]pyrrolidin-2-carbonsäuremethylester
9/ (S)-1-[2-Hydroxy-3-(2-{[(R)-(5-methylfuran-2-yl)-(3-methyloxetan-3-yl)methyl]amino}-3,4-dioxocyclobut-1-enylamino)benzoyl]pyrrolidin-2-carbonsäuremethylester
10/ (R)-1-[2-Hydroxy-3-(2-{[(5-methylfuran-2-yl)-(3-methyloxetan-3-yl)methyl]amino}-3,4-dioxocyclobut-1-enylamino)benzoyl]pyrrolidin-2-carbonsäure
11/ 3-[2-Hydroxy-3-(1-hydroxyethyl)phenylamino]-4-{[(5-methylfuran-2-yl)-(3-methyloxetan-3-yl)methyl]amino}cyclobut-3-en-1,2-dion
12/ 3-(2-Hydroxy-3-isobutyrylphenylamino)-4-{[(5-methylfuran-2-yl)-(3-methyloxetan-3-yl)methyl]amino}cyclobut-3-en-1,2-dion
13/ 3-(4-Hydroxy-2-methyl-3-oxo-2,3-dihydro-1H-isoindol-5-ylamino)-4-{[(5-methylfuran-2-yl)-(3-methyloxetan-3-yl)methyl]amino}cyclobut-3-en-1,2-dion
14/ 3-[2-Hydroxy-3-(pyrrolidin-1-carbonyl)-phenylamino]-4-{[(5-methylfuran-2-yl)-(3-methyloxetan-3-yl)methyl]amino}cyclobut-3-en-1,2-dion
15/ 3-[2-Hydroxy-3-(morpholin-4-carbonyl)phenylamino]-4-{[(5-methylfuran-2-yl)-(3-methyloxetan-3-yl)methyl]amino}cyclobut-3-en-1,2-dion
16/ 3-(4-Hydroxypyrimidin-5-ylamino)-4-{[(5-methylfuran-2-yl)-(3-methyl-oxetan-3-yl)methyl]-amino}cyclobut-3-en-1,2-dion
17/ 3-{[(5-Methylfuran-2-yl)-(3-methyloxetan-3-yl)methyl]amino}-4-(1-methyl-2-oxo-1,2-dihydro-chinolin-3-ylamino)cyclobut-3-en-1,2-dion
18/ 3-{[(5-Methylfuran-2-yl)-(3-methyloxetan-3-yl)methyl]amino}-4-(6-methyl-2-oxo-2H-pyran-3-ylamino)cyclobut-3-en-1,2-dion
19/ 3-{[(5-Methylfuran-2-yl)-(3-methyloxetan-3-yl)methyl]amino}-4-(1-methyl-2-oxo-1,2-dihydro-pyridin-3-ylamino)cyclobut-3-en-1,2-dion
20/ 3-(2-Hydroxypyridin-3-ylamino)-4-{[(5-methylfuran-2-yl)-(3-methyloxetan-3-yl)methyl]amino}cyclobut-3-en-1,2-dion
21/ 2-Hydroxy-N,N-dimethyl-3-(2-{[(5-methylfuran-2-yl)-(3-methyloxetan-3-yl)methyl]amino}-3,4-dioxocyclobut-1-enylamino)benzolsulfonamid
22/ 2-Hydroxy-N,N-dimethyl-3-(2-{[(R)-(5-methylfuran-2-yl)-(3-methyloxetan-3-yl)methyl]amino}-3,4-dioxocyclobut-1-enylamino)benzolsulfonamid
23/ 3-(3H-Benzotriazol-4-ylamino)-4-{[(5-methylfuran-2-yl)-(3-methyloxetan-3-yl)methyl]amino}cyclobut-3-en-1,2-dion
24/ 3-{[(5-Methylfuran-2-yl)-(3-methyloxetan-3-yl)methyl]amino}-4-(2-oxo-2,3-dihydrobenzooxazol-7-ylamino)cyclobut-3-en-1,2-dion
25/ 3-Hydroxy-4-(2-{[(R)-(5-methylfuran-2-yl)-(3-methyloxetan-3-yl)methyl]amino}-3,4-dioxcyclobut-1-enylamino)thiophen-2-carbonsäuredimethylamid
26/ 3-[[2-(3-Dimethylcarbamoyl-2-hydroxyphenylamino)-3,4-dioxo-cyclobut-1-enylamino]-(5-methylfuran-2-yl)methyl]-3-methylazetidin-1-carbonsäure-tert-butylester
27/ 3-(2-{[(4,5-Dimethylfuran-2-yl)-(3-methyl-oxetan-3-yl)methyl]amino}-3,4-dioxocyclobut-1-enylamino)-2-hydroxy-N,N-dimethylbenzamid
28/ 3-(2-{[(3-Methyloxetan-3-yl)-(3-methoxy-phenyl)methyl]amino}-3,4-dioxocyclobut-1-enyl-amino)-2-hydroxy-N,N-dimethylbenzamid
29/ 3-(2-{[(3-Methyloxetan-3-yl)-(4-methoxy-phenyl)methyl]amino}-3,4-dioxocyclobut-1-enyl-amino)-2-hydroxy-N,N-dimethylbenzamid
30/ 3-(2-{[Benzo[1,3]dioxol-5-yl-(3-methyloxetan-3-yl)methyl]amino}-3,4-dioxocyclobut-1-enylamino)-2-hydroxy-N,N-dimethylbenzamid
31/ 3-[3-(3-Hydroxypyrrolidin-1-carbonyl)phenylamino]-4-{[(5-methylfuran-2-yl)-(3-methyloxetan-3-yl)methyl]amino}cyclobut-3-en-1,2-dion
32/ 1-[3-(2-{[(5-Methylfuran-2-yl)-(3-methyl-oxetan-3-yl)methyl]amino}-3,4-dioxocyclobut-1-enylamino)benzoyl]piperidin-2-carbonsäuremethyl-ester
33/ 1-[3-(2-{[(5-Methylfuran-2-yl)-(3-methyl-oxetan-3-yl)methyl]amino}-3,4-dioxocyclobut-1-enylamino)benzoyl]pyrrolidin-2-carbonsäuremethyl-ester
34/ 2-Hydroxy-N,N-dimethyl-3-(2-{[(3-methyloxetan-3-yl)thiophen-2-ylmethyl]amino}-3,4-dioxocyclobut-1-enylamino)benzamid
35/ 2-Hydroxy-N,N-dimethyl-3-(2-{[(3-methyloxetan-3-yl)-(5-methylthiophen-2-yl)methyl]amino}-3,4-dioxocyclobut-1-enylamino)benzamid
36/ 3-(2-{[Furan-2-yl-(3-methyloxetan-3-yl)-methyl]amino}-3,4-dioxocyclobut-1-enylamino)-2-hydroxy-N,N-dimethylbenzamid
37a/ 2-Hydroxy-N,N-dimethyl-3-(2-{[(4-methylfuran-2-yl)-(3-methyloxetan-3-yl)methyl]amino}-3,4-dioxocyclobut-1-enylamino)benzamid
37b/ 2-Hydroxy-N,N-dimethyl-3-(2-{[(3-methylfuran-2-yl)-(3-methyloxetan-3-yl)methyl]amino}-3,4-dioxocyclobut-1-enylamino)benzamid
38/ 2-Hydroxy-N,N-dimethyl-3-(2-{[(4-isopropylfuran-2-yl)-(3-methyloxetan-3-yl)methyl]amino}-3,4-dioxocyclobut-1-enylamino)benzamid
39/ 3-(2-Hydroxy-6-methoxypyridin-3-ylamino)-4-{[(5-methylfuran-2-yl)-(3-methyloxetan-3-yl)-methyl]amino}cyclobut-3-en-1,2-dion
40/ 3-(6-Chlor-2-hydroxypyridin-3-ylamino)-4-{[(5-methylfuran-2-yl)-(3-methyloxetan-3-yl)methyl]amino}cyclobut-3-en-1,2-dion
41/ 3-{[(5-Methylfuran-2-yl)-(3-methyloxetan-3-yl)methyl]amino}-4-(2-oxo-2,3-dihydro-1H-benzoimidazol-4-ylamino)cyclobut-3-en-1,2-dion
42/ 2-Hydroxy-3-(2-{[(5-methylfuran-2-yl)-(3-methyloxetan-3-yl)methyl]amino}-3,4-dioxocyclobut-1-enylamino)benzonitril
43/ (R)-1-[3-(2-{[(5-Methylfuran-2-yl)-(3-methyloxetan-3-yl)methyl]amino}-3,4-dioxocyclobut-1-enylamino)benzolsulfonyl]pyrrolidin-2-carbonsäuremethylester
44/ (S)-1-[4-Chlor-3-(2-{[(5-methylfuran-2-yl)-(3-methyloxetan-3-yl)methyl]amino}-3,4-dioxocyclobut-1-enylamino)benzolsulfonyl]pyrrolidin-2-carbonsäure-methylester
45/ (S)-1-[2,6-Difluor-3-(2-{[(5-methylfuran-2-yl)-(3-methyloxetan-3-yl)methyl]amino}-3,4-dioxocyclobut-1-enylamino)benzoyl]pyrrolidin-2-carbonsäuremethylester
46/ (S)-1-[2-Chlor-3-(2-{[(5-methylfuran-2-yl)-(3-methyloxetan-3-yl)methyl]amino}-3,4-dioxocyclobut-1-enylamino)benzoyl]pyrrolidin-2-carbonsäuremethylester
47/ (R)-1-[2-Hydroxy-3-(2-{[(R)-(5-methylfuran-2-yl)-(3-methyloxetan-3-yl)-methyl]amino}-3,4-dioxocyclobut-1-enylamino)benzoyl]pyrrolidin-2-carbon-säuremethylester
48/ 2-Hydroxy-N-methyl-3-(2-{[(R)-(5-methylfuran-2-yl)-(3-methyloxetan-3-yl)methyl]amino}-3,4-dioxocyclobut-1-enylamino)-N-(2,2,2-trifluorethyl)benzamid
49/ 2-Hydroxy-N,N-dimethyl-3-(2-{[((R)-5-methylfuran-2-yl)-(3-methyloxetan-3-yl)methyl]amino}-3,4-dioxocyclobut-1-enylamino)benzamid
51/ {[2-Hydroxy-3-(2-{[(R)-(5-methylfuran-2-yl)-(3-methyloxetan-3-yl)methyl]amino}-3,4-dioxocyclobut-1-enylamino)benzoyl]methylamino}essigsäure-methylester
52/ 6-Chlor-2-hydroxy-N,N-dimethyl-3-(2-{[(R)-(5-methylfuran-2-yl)-(3-methyloxetan-3-yl)methyl]-amino}-3,4-dioxocyclobut-1-enylamino)benzamid
53/ 3-[4-Chlor-2-hydroxy-3-(4-methylpiperazin-1-sulfonyl)phenylamino]-4-{[(R)-(5-methylfuran-2-yl)-(3-methyloxetan-3-yl)methyl]amino}cyclobut-3-en-1,2-dion.

## Claims

1. Disubstituted 3,4-diamino-3-cyclobutene-1,2-dione compound corresponding to general formula (I) below, or one of the pharmaceutically acceptable salts or solvates thereof: 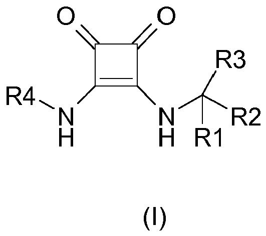 in which,
R1 represents a hydrogen atom or a methyl,
R2 represents a ring comprising four atoms, chosen from the structures (1) and (2) below: 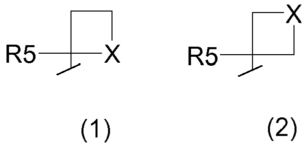 in which R5 and X have the meaning given hereinafter,
R3 represents an aromatic or heteroaromatic ring selected from the group consisting of the rings corresponding to formulae (a) to (o) below: 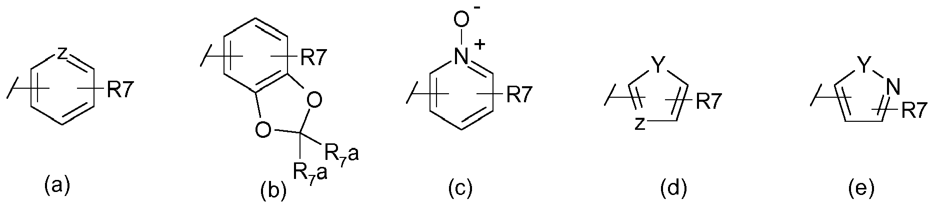 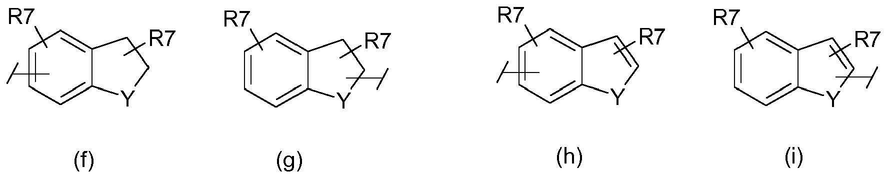 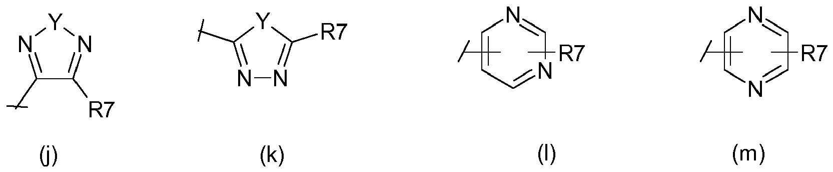 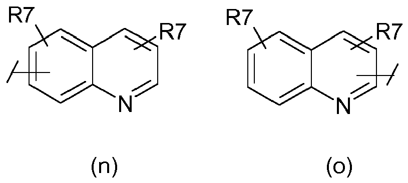 in which R7, R7a, Y and Z have the meaning given hereinafter, it being specified that the rings (a) to (o) can optionally bear several R7 groups, which may be identical or different, the total number of such R7 groups being at most equal to the number of substitutable atoms of the ring;
R4 represents an aromatic or heteroaromatic ring selected from the group consisting of the rings corresponding to formulae (p) to (z) and (aa) to (ak) below: 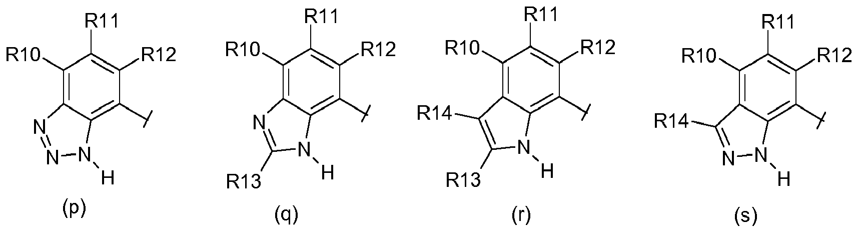 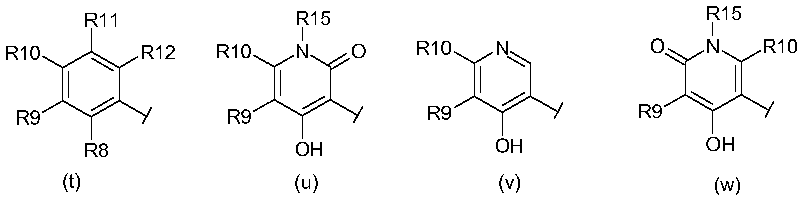 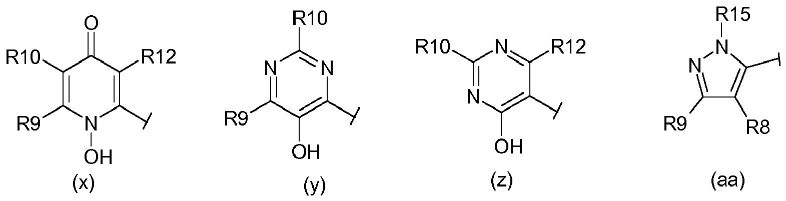 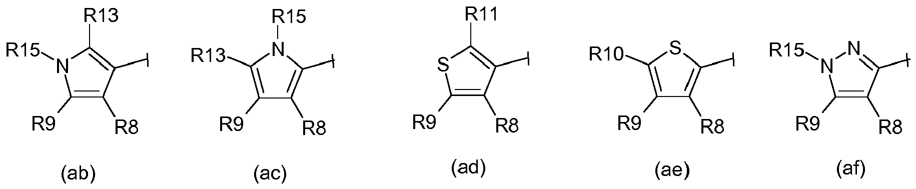 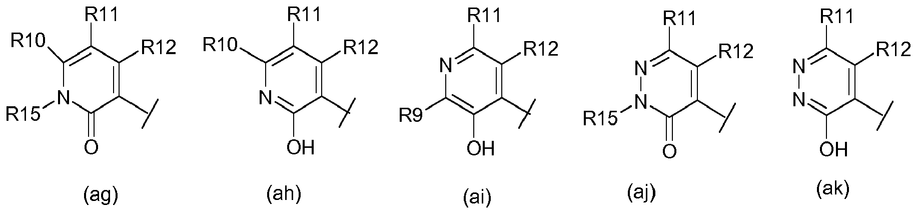 in which R7, R8, R9, R10, R11, R12, R13, R14 and R15 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a radical R16, a halogen, -CF₃, -COR16,-OR16, -NR16R17, -NO₂, -CN, -SO₂R16, -SO₂NR16R17,-NR16COR17, -CONR16R17, -NR16CO₂R17 or -CO₂R16,
R7a represents a hydrogen atom or an alkyl radical having from 1 to 5 carbon atoms,
R8 represents a hydrogen atom, a halogen atom, or an-OH, -SH, -CONHOR16, -CONR160H, -NR16R17, -SO₃H, -OCOR16, -NHSO₂R16, -SO₂NR16R17, -NHCOR16, -CONR16R17,-NR16CO₂R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen, a halogen atom and an alkyl, alkoxy, -CF₃,-OCF₃, -OH, -NO₂, -CN, -SO₂R16, -SO₂NR16R17, -NR16COR17,-NR16CO₂R17, -CONR16R17, -COR16 or -CO₂R16 radical,
or alternatively, when two of the R9, R10, R11 and R12 radicals are in the ortho position on an aromatic or heteroaromatic ring selected from the group consisting of the rings corresponding to formulae (p) to (z) and (aa) to (ak) above, then they can together form, with the bond which links them together, an aryl, heteroaryl, cycloalkyl or heterocycloalkyl ring,
R13 and R14 are identical or different and are independently chosen from the group consisting of a hydrogen atom, a halogen atom, an alkyl, -CF₃, -OCF₃,-OH, -SH, -CN, -SO₂R16, -SO₂NR16R17, -NHSO₂NR16R17,-NR16R17, -NR16CONR16R17, -NR16COR17, -NR16CO₂R17,-CONR16R17, -COR16 and -CO₂R16,
R15 represents a hydrogen atom or an -OH, -SO₂R16,-COR16, -CO₂R16, aryl, heteroaryl, arylalkyl, heteroarylalkyl, alkyl, cycloalkyl or cycloalkylalkyl radical,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X represents an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

2. Compound according to Claim 1, **characterized in that**, in the abovementioned formula (I):
R1 represents a hydrogen atom,
R2 represents a four-membered ring corresponding to structure (2) below: 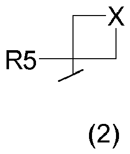 in which R5 and X have the meaning given hereinafter,
R3 represents an aromatic or heteroaromatic ring selected from the group consisting of the rings corresponding to formulae (a), (b) and (d) below: 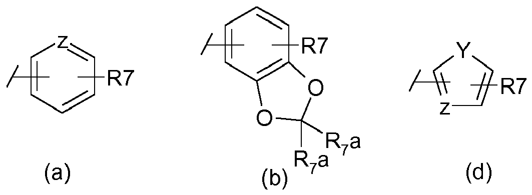 in which R7, R7a, Y and Z have the meaning given hereinafter, it being specified that the rings (a), (b) and (d) can optionally bear several R7 groups, which may be identical or different, the total number of such R7 groups being at most equal to the number of substitutable atoms of the ring;
R4 represents an aromatic or heteroaromatic ring selected from the group consisting of the rings corresponding to formulae (p), (q), (t), (z), (ad), (ag) and (ah) below: 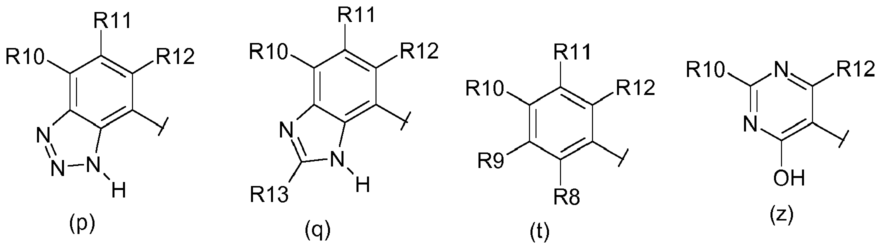 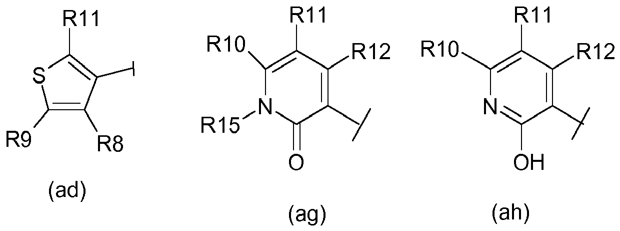 in which R8, R9, R10, R11, R12, R13 and R15 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a halogen, or an R16, -CF₃, -COR16, -OR16, -NR16R17, -NO₂, -CN, -SO₂R16, -SO₂NR16R17, -NR16COR17,-CONR16R17, -NR16CO₂R17 or -CO₂R16 radical,
R7a represents a hydrogen atom or an alkyl radical having from 1 to 5 carbon atoms,
R8 represents a hydrogen atom, or an -OH, -SH,-CONHOR16, -CONR160H, -NR16R17, -SO₃H, -OCOR16,-NHSO₂R16, -SO₂NR16R17, -NHCOR16, -CONR16R17,-NR16CO₂R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen atom, a halogen atom and an alkyl, alkoxy,-CF₃, -OCF₃, -OH, -NO₂, -CN, -SO₂R16, -SO₂NR16R17,-NR16COR17, -NR16CO₂R17, -CONR16R17, -COR16 or -CO₂R16 radical,
or alternatively, when two of the R9, R10, R11 and R12 radicals are in the ortho position on an aromatic or heteroaromatic ring selected from the group consisting of the rings corresponding to formulae (p), (q), (t), (z), (ad), (ag) and (ah) above, then they can together form, with the bond which links them together, an aryl, heteroaryl, cycloalkyl or heterocycloalkyl ring,
R13 is chosen from the group consisting of a hydrogen atom, a halogen atom, and an alkyl, -CF₃, -OCF₃, -OH,-SH, -CN, -SO₂R16, -SO₂NR16R17, -NHSO₂NR16R17, -NR16R17, -NR16CONR16R17, -NR16COR17, -NR16CO₂R17, -CONR16R17,-COR16 or -CO₂R16 radical,
R15 represents a hydrogen atom or an -OH, -SO₂R16,-COR16, -CO₂R16, aryl, heteroaryl, arylalkyl, heteroarylalkyl, alkyl, cycloalkyl or cycloalkylalkyl radical,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X represents an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

3. Compound according to Claim 1 or 2, **characterized in that**, in the abovementioned formula (I):
R1 represents a hydrogen atom,
R2 represents a ring comprising four atoms, corresponding to structure (2) below: 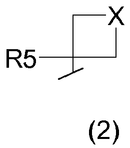 in which R5 and X have the meaning given hereinafter,
R3 represents a heteroaromatic ring corresponding to formula (d) below: 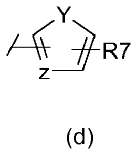 in which R7, Y and Z have the meaning given hereinafter, it being specified that the ring (d) can optionally bear several R7 groups, which may be identical or different, the total number of such R7 groups being at most equal to the number of substitutable atoms of the ring;
R4 represents an aromatic or heteroaromatic ring selected from the group consisting of the rings corresponding to formulae (p), (q), (t), (z), (ad), (ag) and (ah) below: 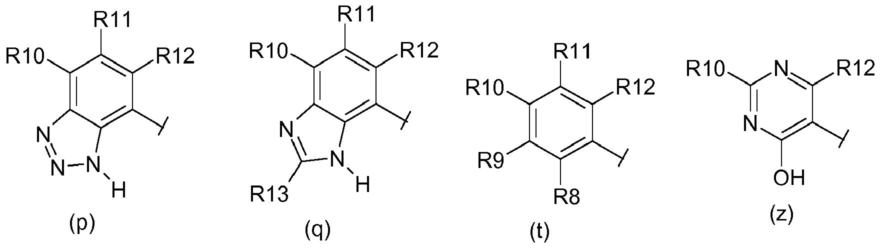 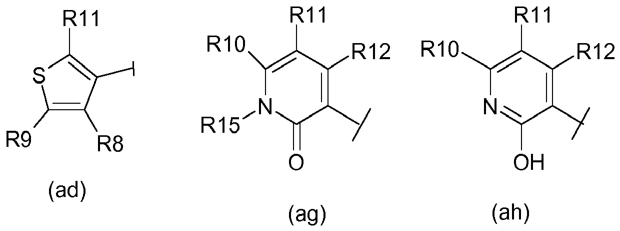 in which R8, R9, R10, R11, R12, R13 and R15 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a halogen atom, or an R16, -CF₃, -COR16,-OR16, -NR16R17, -NO₂, -CN, -SO₂R16, -SO₂NR16R17,-NR16COR17, -CONR16R17, -NR16CO₂R17 or -CO₂R16 radical,
R8 represents a hydrogen atom, or an -OH, -SH,-CONHOR16, -CONR160H, -NR16R17, -SO₃H, -OCOR16,-NHSO₂R16, -SO₂NR16R17, -NHCOR16, -CONR16R17,-NR16CO₂R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen atom, a halogen atom and an alkyl, alkoxy,-CF₃, -OCF₃, -OH, -NO₂, -CN, -SO₂R16, -SO₂NR16R17,-NR16COR17, -NR16CO₂R17, -CONR16R17, -COR16 or -CO₂R16 radical,
or alternatively, when two of the R9, R10, R11 and R12 radicals are in the ortho position on an aromatic or heteroaromatic ring selected from the group consisting of the rings corresponding to formulae (p), (q), (t), (z), (ad), (ag) and (ah) above, they can together form, with the bond which links them together, an aryl, heteroaryl, cycloalkyl or heterocycloalkyl ring,
R13 is chosen from the group consisting of a hydrogen atom, a halogen atom, and an alkyl, -CF₃, -OCF₃, -OH,-SH, -CN, -SO₂R16, -SO₂NR16R17, -NHSO₂NR16R17, -NR16R17, -NR16CONR16R17, -NR16COR17, -NR16CO₂R17, -CONR16R17,-COR16 or -CO₂R16 radical,
R15 represents a hydrogen atom, or an -OH, -SO₂R16,-COR16, -CO₂R16, aryl, heteroaryl, arylalkyl, heteroarylalkyl, alkyl, cycloalkyl or cycloalkylalkyl radical,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X represents an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

4. Compound according to one of Claims 1 to 3, **characterized in that**, in the abovementioned formula (I) :
R1 represents a hydrogen atom,
R2 represents a ring comprising four atoms, corresponding to structure (2) below: 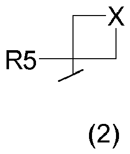 in which R5 and X have the meaning given hereinafter,
R3 represents a heteroaromatic ring corresponding to formula (d) below: 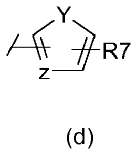 in which R7, Y and Z have the meaning given hereinafter, it being specified that the ring (d) can optionally bear several R7 groups, which may be identical or different, the total number of such R7 groups being at most equal to the number of substitutable atoms of the ring;
R4 represents an aromatic ring corresponding to formula (t) below: 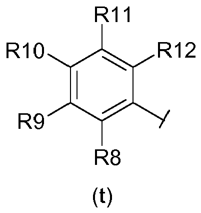 in which R8, R9, R10, R11 and R12 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a radical R16, a halogen, -CF₃, -COR16,-OR16, -NR16R17, -NO₂, -CN, -SO₂R16, -SO₂NR16R17,-NR16COR17, -CONR16R17, -NR16CO₂R17 or -CO₂R16,
R8 represents a hydrogen atom, or an -OH, -SH,-CONHOR16, -CONR160H, -NR16R17, -SO₃H, -OCOR16,-NHSO₂R16, -SO₂NR16R17, -NHCOR16, -CONR16R17,-NR16CO₂R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen atom, a halogen atom and an alkyl, alkoxy,-CF₃, -OCF₃, -OH, -NO₂, -CN, -SO₂R16, -SO₂NR16R17,-NR16COR17, -NR16CO₂R17, -CONR16R17, -COR16 or -CO₂R16 radical,
or alternatively, when two of the R9, R10, R11 and R12 radicals are in the ortho position on the aromatic ring (t), they can together form, with the bond which links them together, an aryl, heteroaryl, cycloalkyl or heterocycloalkyl ring,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X represents an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

5. Pharmaceutical composition comprising an effective amount of a compound, or of a pharmaceutically acceptable salt of said compound, according to Claim 1, in combination with a pharmaceutically acceptable solvent or support.

6. Compound according to Claim 1 or pharmaceutical composition according to Claim 5, for use as a medicament.

7. Compound according to Claim 1 or pharmaceutical composition according to Claim 5, for use in the treatment of α-chemokine-mediated diseases.

8. Compound or pharmaceutical composition according to Claim 7, for use in the treatment of diseases of the group comprising neutrophilic dermatosis, in particular psoriasis, atopic dermatitis, acne, rosacea, asthma, chronic obstructive pulmonary diseases, respiratory diseases in adults, arthritis, inflammatory bowel diseases, Crohn's disease, transplant rejection, cystic fibrosis and skin cancers.

9. Compound or pharmaceutical composition according to Claim 7, for use in the treatment of skin diseases such as neutrophilic dermatosis, in particular psoriasis, atopic dermatitis, acne and rosacea.

10. Compound according to Claim 1, chosen from the list consisting of:
1/ 2-hydroxy-N,N-dimethyl-3-(2-{[(5-methylfuran-2-yl)-(3-methyloxetan-3-yl)methyl]amino}-3,4-dioxocyclobut-1-enylamino)benzamide
2/ 3-(2-{[(3-fluoromethyloxetan-3-yl)-(5-methyl-furan-2-yl)methyl]amino}-3,4-dioxocyclobut-1-enylamino)-2-hydroxy-N,N-dimethylbenzamide
3/ 3-(2-{[(3-ethyloxetan-3-yl)-(5-methylfuran-2-yl)methyl]amino}-3,4-dioxocyclobut-1-enylamino)-2-hydroxy-N,N-dimethylbenzamide
4/ 2-hydroxy-3-(2-{[(5-methylfuran-2-yl)-(3-methyloxetan-3-yl)methyl]amino}-3,4-dioxocyclobut-1-enylamino)benzoic acid
5/ 3-[2-hydroxy-3-((R)-3-hydroxypyrrolidine-1-carbonyl)phenylamino]-4-{[(5-methylfuran-2-yl)-(3-methyloxetan-3-yl)methyl]amino}cyclobut-3-ene-1,2-dione
6/ 3-[2-hydroxy-3-((S)-3-hydroxypyrrolidine-1-carbonyl)phenylamino]-4-{[(5-methylfuran-2-yl)-(3-methyloxetan-3-yl)methyl]amino}cyclobut-3-ene-1,2-dione
7/ (R)-1-[2-hydroxy-3-(2-{[(5-methylfuran-2-yl)-(3-methyloxetan-3-yl)methyl]amino}-3,4-dioxocyclobut-1-enylamino)benzoyl]pyrrolidine-2-carboxylic acid tert-butyl ester
8/ methyl (R)-1-[2-hydroxy-3-(2-{[(R)-(5-methylfuran-2-yl)-(3-methyloxetan-3-yl)methyl]amino}-3,4-dioxocyclobut-1-enylamino)benzoyl]pyrrolidine-2-carboxylate
9/ methyl (S)-1-[2-hydroxy-3-(2-{[(R)-(5-methylfuran-2-yl)-(3-methyloxetan-3-yl)methyl]amino}-3,4-dioxocyclobut-1-enylamino)benzoyl]pyrrolidine-2-carboxylate
10/ (R)-1-[2-hydroxy-3-(2-{[(5-methylfuran-2-yl)-(3-methyloxetan-3-yl)methyl]amino}-3,4-dioxocyclobutl-enylamino)benzoyl]pyrrolidine-2-carboxylic acid
11/ 3-[2-hydroxy-3-(1-hydroxyethyl)phenylamino]-4-{[(5-methylfuran-2-yl)-(3-methyloxetan-3-yl)methyl]amino}cyclobut-3-ene-1,2-dione 12/ 3-(2-hydroxy-3-isobutyrylphenylamino)-4-{[(5-methylfuran-2-yl)-(3-methyloxetan-3-yl)methyl]amino}cyclobut-3-ene-1,2-dione 13/ 3-(4-hydroxy-2-methyl-3-oxo-2,3-dihydro-1H-isoindol-5-ylamino)-4-{[(5-methylfuran-2-yl)-(3-methyloxetan-3-yl)methyl]amino}cyclobut-3-ene-1,2-dione 14/ 3-[2-hydroxy-3-(pyrrolidine-1-carbonyl)phenylamino]-4-{[(5-methylfuran-2-yl)-(3-methyloxetan-3-yl)methyl]amino}cyclobut-3-ene-1,2-dione 15/ 3-[2-hydroxy-3-(morpholine-4-carbonyl)phenylamino]-4-{[(5-methylfuran-2-yl)-(3-methyloxetan-3-yl)methyl]amino}cyclobut-3-ene-1,2-dione 16/ 3-(4-hydroxypyrimidin-5-ylamino)-4-{[(5-methylfuran-2-yl)-(3-methyl-oxetan-3-yl)methyl]amino}cyclobut-3-ene-1,2-dione 17/ 3-{[(5-methylfuran-2-yl)-(3-methyloxetan-3-yl)methyl]amino}-4-(1-methyl-2-oxo-1,2-dihydroquinolin-3-ylamino)cyclobut-3-ene-1,2-dione
18/ 3-{[(5-methylfuran-2-yl)-(3-methyloxetan-3-yl)methyl]amino}-4-(6-methyl-2-oxo-2H-pyran-3-ylamino)cyclobut-3-ene-1,2-dione
19/ 3-{[(5-methylfuran-2-yl)-(3-methyloxetan-3-yl)methyl]amino}-4-(1-methyl-2-oxo-1,2-dihydropyridin-3-ylamino)cyclobut-3-ene-1,2-dione
20/ 3-(2-hydroxypyridin-3-ylamino)-4-{[(5-methylfuran-2-yl)-(3-methyloxetan-3-yl)methyl]amino}cyclobut-3-ene-1,2-dione
21/ 2-hydroxy-N,N-dimethyl-3-(2-{[(5-methylfuran-2-yl)-(3-methyloxetan-3-yl)methyl]amino}-3,4-dioxocyclobut-1-enylamino)benzenesulfonamide
22/ 2-hydroxy-N,N-dimethyl-3-(2-{[(R)-(5-methylfuran-2-yl)-(3-methyloxetan-3-yl)methyl]amino}-3,4-dioxocyclobut-1-enylamino)benzenesulfonamide
23/ 3-(3H-benzotriazol-4-ylamino)-4-{[(5-methylfuran-2-yl)-(3-methyloxetan-3-yl)methyl]amino}cyclobut-3-ene-1,2-dione
24/ 3-{[(5-methyl-furan-2-yl)-(3-methyloxetan-3-yl)methyl]amino}-4-(2-oxo-2,3-dihydrobenzooxazol-7-ylamino)cyclobut-3-ene-1,2-dione
25/ dimethylamide 3-hydroxy-4-(2-{[(R)-(5-methylfuran-2-yl)-(3-methyloxetan-3-yl)methyl]amino}-3,4-dioxcyclobut-1-enylamino)thiophene-2-carboxylate
26/ tert-butyl 3-[[2-(3-dimethylcarbamoyl-2-hydroxyphenylamino)-3,4-dioxo-cyclobut-1-enylamino]-(5-methylfuran-2-yl)methyl]-3-methylazetidine-1-carboxylate
27/ 3-(2-{[(4,5-dimethylfuran-2-yl)-(3-methyloxetan-3-yl)methyl]amino}-3,4-dioxocyclobut-1-enylamino)-2-hydroxy-N,N-dimethylbenzamide
28/ 3-(2-{[(3-methyloxetan-3-yl)-(3-methoxyphenyl)methyl]amino}-3,4-dioxocyclobut-1-enylamino)-2-hydroxy-N,N-dimethylbenzamide
29/ 3-(2-{[(3-methyloxetan-3-yl)-(4-methoxyphenyl)methyl]amino}-3,4-dioxocyclobut-1-enylamino)-2-hydroxy-N,N-dimethylbenzamide
30/ 3-(2-{[benzo[1,3]dioxol-5-yl-(3-methyloxetan-3-yl)methyl]amino}-3,4-dioxocyclobut-1-enylamino)-2-hydroxy-N,N-dimethylbenzamide
31/ 3-[3-(3-hydroxypyrrolidine-1-carbonyl)phenylamino]-4-{[(5-methylfuran-2-yl)-(3-methyloxetan-3-yl)methyl]amino}cyclobut-3-ene-1,2-dione
32/ methyl 1-[3-(2-{[(5-methylfuran-2-yl)-(3-methyloxetan-3-yl)methyl]amino}-3,4-dioxocyclobut-1-enylamino)benzoyl]piperidine-2-carboxylate
33/ methyl 1-[3-(2-{[(5-methylfuran-2-yl)-(3-methyloxetan-3-yl)methyl]amino}-3,4-dioxocyclobut-1-enylamino)benzoyl]pyrrolidine-2-carboxylate
34/ 2-hydroxy-N,N-dimethyl-3-(2-{[(3-methyloxetan-3-yl)thiophen-2-ylmethyl]amino}-3,4-dioxocyclobut-1-enylamino)benzamide
35/ 2-hydroxy-N,N-dimethyl-3-(2-{[(3-methyloxetan-3-yl)-(5-methylthiophen-2-yl)methyl]amino}-3,4-dioxocyclobut-1-enylamino)benzamide
36/ 3-(2-{[furan-2-yl-(3-methyloxetan-3-yl)methyl]amino}-3,4-dioxocyclobut-1-enylamino)-2-hydroxy-N,N-dimethylbenzamide
37a/ 2-hydroxy-N,N-dimethyl-3-(2-{[(4-methylfuran-2-yl)-(3-methyloxetan-3-yl)methyl]amino}-3,4-dioxocyclobut-1-enylamino)benzamide
37b/ 2-hydroxy-N,N-dimethyl-3-(2-{[(3-methylfuran-2-yl)-(3-methyloxetan-3-yl)methyl]amino}-3,4-dioxocyclobut-1-enylamino)benzamide
38/ 2-hydroxy-N,N-dimethyl-3-(2-{[(4-isopropylfuran-2-yl)-(3-methyloxetan-3-yl)methyl]amino}-3,4-dioxocyclobut-1-enylamino)benzamide
39/ 3-(2-hydroxy-6-methoxypyridin-3-ylamino)-4-{[(5-methylfuran-2-yl)-(3-methyloxetan-3-yl)methyl]amino}cyclobut-3-ene-1,2-dione 40/ 3-(6-chloro-2-hydroxypyridin-3-ylamino)-4-{[(5-methylfuran-2-yl)-(3-methyloxetan-3-yl)methyl]amino}cyclobut-3-ene-1,2-dione 41/ 3-{[(5-methylfuran-2-yl)-(3-methyloxetan-3-yl)methyl]amino}-4-(2-oxo-2,3-dihydro-1H-benzoimidazol-4-ylamino)cyclobut-3-ene-1,2-dione
42/ 2-hydroxy-3-(2-{[(5-methylfuran-2-yl)-(3-methyloxetan-3-yl)methyl]amino}-3,4-dioxocyclobut-1-enylamino)benzonitrile
43/ methyl (R)-1-[3-(2-{[(5-methylfuran-2-yl)-(3-methyloxetan-3-yl)methyl]amino}-3,4-dioxocyclobut-1-enylamino)benzenesulfonyl]pyrrolidine-2-carboxylate
44/ methyl (S)-1-[4-chloro-3-(2-{[(5-methylfuran-2-yl)-(3-methyloxetan-3-yl)methyl]amino}-3,4-dioxocyclobut-1-enylamino)benzenesulfonyl]pyrrolidine-2-carboxylate
45/ methyl (S)-1-[2,6-difluoro-3-(2-{[(5-methylfuran-2-yl)-(3-methyloxetan-3-yl)methyl]amino}-3,4-dioxocyclobut-1-enylamino)benzoyl]pyrrolidine-2-carboxylate
46/ methyl (S)-1-[2-chloro-3-(2-{[(5-methylfuran-2-yl)-(3-methyloxetan-3-yl)methyl]amino}-3,4-dioxocyclobut-1-enylamino)benzoyl]pyrrolidine-2-carboxylate
47/ methyl (R)-1-[2-hydroxy-3-(2-{[(R)-(5-methylfuran-2-yl)-(3-methyloxetan-3-yl)-methyl]amino}-3,4-dioxocyclobut-1-enylamino)benzoyl]pyrrolidine-2-carboxylate
48/ 2-hydroxy-N-methyl-3-(2-{[(R)-(5-methylfuran-2-yl)-(3-methyloxetan-3-yl)methyl]amino}-3,4-dioxocyclobut-1-enylamino)-N-(2,2,2-trifluoroethyl)benzamide:
49/ 2-hydroxy-N,N-dimethyl-3-(2-{[((R)-5-methylfuran-2-yl)-(3-methyloxetan-3-yl)methyl]amino}-3,4-dioxocyclobut-1-enylamino)benzamide
51/ methyl {[2-hydroxy-3-(2-{[(R)-(5-methylfuran-2-yl)-(3-methyloxetan-3-yl)methyl]amino}-3,4-dioxocyclobut-1-enylamino)benzoyl]methylamino}acetate
52/ 6-chloro-2-hydroxy-N,N-dimethyl-3-(2-{[(R)-(5-methylfuran-2-yl)-(3-methyloxetan-3-yl)methyl]amino}-3,4-dioxocyclobut-1-enylamino)benzamide
53/ 3-[4-chloro-2-hydroxy-3-(4-methylpiperazine-1-sulfonyl)phenylamino]-4-{[(R)-(5-methylfuran-2-yl)-(3-methyloxetan-3-yl)methyl]amino}cyclobut-3-ene-1,2-dione
